# EUROPEAN PATENT APPLICATION

(11) **EP 4 574 131 A1**
(43) Date of publication of application: **25.06.2025**
(21) Application number: 23854754.1
(22) Date of filing: 13.07.2023
(51) Int. Cl.: A61K 8/891, A61Q 1/00, A61Q 3/02, A61Q 5/00, A61Q 5/02, A61Q 5/12, A61Q 15/00, A61Q 17/04, C08K 5/01, C08K 5/05, C08L 83/04, C08L 83/05, C08L 83/07

(54) **COSMETIC PREPARATION CONTAINING CROSSLINKED ORGANIC SILICON RESIN AND METHOD FOR PRODUCING SAME**

(30) Priority: 17.08.2022 JP 2022130099
(71) Applicant: SHIN-ETSU CHEMICAL CO., LTD., Tokyo 1000005 (JP)
(72) Inventor: ABE Takuya, Annaka-shi, Gunma 379-0224 (JP); KONISHI Masayuki, Tokyo 100-0005 (JP)
(74) Representative: Mewburn Ellis LLP
(86) International application number: PCT/JP2023/025862
(87) International publication number: WO 2024/038717

(57) **Abstract**

The present invention provides a crosslinked organic silicon resin which is an addition reaction product of the component (A) and the component (B) described below, wherein the amount of a hydrogen gas generated from this crosslinked organic silicon resin per mass thereof is up to 1.5 mL/g in a standard state. This crosslinked organic silicon resin has excellent long-term stability and forms a coating film that exhibits excellent oil resistance and high bendability, while being free from stickiness and brittleness.

Component (A): an alkenyl group-containing organic silicon resin that has one or more alkenyl groups in each molecule

Component (B): an organohydrogen polysiloxane that has two or more hydrosilyl groups in each molecule.
The present invention also provides a cosmetic preparation which comprises this crosslinked organic silicon resin and has good feeling of use and good spreadability, while having excellent secondary adhesion prevention properties.

## Description

### TECHNICAL FIELD

The present invention relates to a cosmetic preparation including a crosslinked organosilicon resin and a method for producing the cosmetic preparation.

### BACKGROUND ART

The long-lasting performance of a cosmetic preparation can be improved by adding an organosilicon resin to the cosmetic preparation. However, the addition of an organosilicon resin to a cosmetic preparation has two issues. The first issue is the brittleness of a film formed using such a cosmetic preparation. Films formed by applying a cosmetic preparation including an organosilicon resin to hair, face, hand, or the like are likely to crack or detach even with a small motion and are thus poor in terms of followability. In particular, since an MQ resin composed of an M unit (R₃SiO_{1/2}) and a Q unit (SiO_{4/2}) is considerably tough, the film is brittle although having a suitable mechanical strength. Thus, it is difficult to form a self-supporting film. On the other hand, for example, MTQ, MDQ, MDTQ and DT resins, which include T and D units, are capable of enhancing the softness of the film compared with an MQ resin and forming tough self-supporting films having suitable followability. However, the film still has a certain degree of toughness derived from resins; there is room for improvement of the abrasion resistance of the film. Patent Document 1 discloses that applying an emulsion including a plasticized MQ resin to a hair cosmetic preparation enables the formation of films free of cracks. However, using a silicone gum as a plasticizer reduces the hardness of the film and increases the adhesion of the film. This results in an uncomfortable sensation, such as stickiness, which degrades feel on use.

The second issue is oil resistance. When the cosmetic preparation is applied to the skin, resistance to sebum, that is, oil resistance, is required. Sebum is composed primarily of hydrocarbon oils, ester oils, triglycerides and the like. The organosilicon resins used as a film-forming agent have a high compatibility with the above oils. Therefore, subsequent to the application of the cosmetic preparation, the film may become swollen with time, which reduces the physical strength of the cosmetic film and degrades long-lasting performance. In Patent Document 2, a composition that includes an organosilicon resin and a silicone-modified acrylic polymer and has high oil resistance is proposed. Since a silicone-modified acrylic polymer is capable of forming a soft film, certain degrees of softness and followability can be imparted. However, a sufficient degree of oil resistance cannot be achieved, and it is not possible to enhance long-lasting performance in an effective manner.

Crosslinked organosilicon resins formed by crosslinking an organosilicon resin with silicone have been recently reported. In Patent Document 3, it is reported that a crosslinked organosilicon resin is prepared by heating a vinyl group-containing organosilicon resin and a hydrosilyl group-containing silicone and, as a result, a cured product having high flexibility is formed. However, it is difficult to use it as a film-forming agent because it is poorly soluble in volatile oils. Although crosslinked organosilicon resins similar to the above have been reported in Patent Documents 4 and 5, they are gelatinous and do not have a film-forming ability. In Patent Document 6, a crosslinked organosilicon resin is prepared by crosslinking a hydrosilyl group-containing organosilicon resin with a vinyl group-containing silicone using heat curing. The cured product has stickiness and poor solubility in volatile oils. In Patent Document 7, a crosslinked organosilicon resin soluble in volatile oils is prepared by reacting a hydrosilyl group-containing organosilicon resin with a vinyl group-containing silicone in which the number of reactive sites is limited. It is difficult to use this crosslinked organosilicon resin because hydrosilyl groups have low reactivity and the remaining hydrosilyl groups cause a reaction over time to increase viscosity and generate a hydrogen gas. In Patent Document 8, an organopolysiloxane gel is prepared by crosslinking an organosilicon resin having an unsaturated group with an organohydrogenpolysiloxane including 30 or more D units. This organopolysiloxane gel forms a sticky film because the chain length of the crosslinking agent is large and, consequently, the solvent is held in the network, which increases the likelihood of formation of gelatinous films.

### PRIOR ART DOCUMENTS

### PATENT DOCUMENTS

| | |
|---|---|
| Patent Document 1: | JP-A 2015-515981 |
| Patent Document 2: | JP-A 2018-095617 |
| Patent Document 3: | JP-A 2009-052038 |
| Patent Document 4: | JP-A 2010-540721 |
| Patent Document 5: | JP-A 2015-519426 |
| Patent Document 6: | JP-A 2015-505878 |
| Patent Document 7: | JP-A 2021-007486 |
| Patent Document 8: | JP-A 2020-521014 |

### SUMMARY OF INVENTION

### TECHNICAL PROBLEM

The present invention was made in light of the above circumstances. An object of the present invention is to provide a crosslinked organosilicon resin having high stability over time and a capability of forming a film that is highly resistant to oils, free of stickiness or brittleness, and highly flexible, a cosmetic preparation that includes the crosslinked organosilicon resin, has suitable feel on use and suitable elongation, and is excellent in terms of prevention of secondary adhesion, and a method for producing the cosmetic preparation.

### SOLUTION TO PROBLEM

The inventors of the present invention conducted extensive studies in order to achieve the object and consequently found that, compared with a hydrosilyl group-containing organosilicon resin, the alkenyl groups included in an alkenyl group-containing organosilicon resin have high reactivity and the amount of unreacted functional groups can be reduced accordingly and that controlling the ratio of the amount of the hydrosilyl groups to the amount of the alkenyl groups reduces the amount of residual hydrosilyl groups, and an increase in viscosity over time and the generation of a hydrogen gas can be limited consequently.

Moreover, the content of alkenyl groups in the alkenyl group-containing organosilicon resin and the chain length of the organohydrogenpolysiloxane were specified. It was found that the resulting crosslinked organosilicon resin forms a soft film that is free of stickiness and highly flexible.

In general, a hard film has low flexibility, while a highly flexible film is soft. Thus, it has been considered that the strength and flexibility of a film are mutually incompatible. However, the crosslinked organosilicon resin according to the present invention has high flexibility and is therefore excellent in terms of followability, although being capable of forming a tough film. Furthermore, resistance to oils, such as sebum, is markedly enhanced compared with unmodified one. In general, the higher the molecular weight of an organosilicon resin, the higher the oil resistance of the organosilicon resin. Since there is a limitation on the increase in the molecular weight of an organosilicon resin, oil resistance also has its critical point. Crosslinking an organosilicon resin with a crosslinking agent results in a virtual increase in the molecular weight of the organosilicon resin and consequently increases the critical point. Thus, the crosslinked organosilicon resin has high oil resistance which cannot be achieved by the organosilicon resins known in the related art. Moreover, when it is added to an cosmetic preparation as a film-forming agent, the cosmetic preparation does not become sticky when used and has suitable feel on use. In addition, since having suitable adhesion to the skin, the cosmetic preparation has suitable elongation and excellent rubfastness, such as prevention of secondary adhesion.

Specifically, the present invention provides the cosmetic preparation and the method for producing the cosmetic preparation below.
1. A cosmetic preparation including a crosslinked organosilicon resin that is a product of an addition reaction between a component (A) and a component (B), an amount of a hydrogen gas generated by the crosslinked organosilicon resin per weight under a normal condition being up to 1.5 mL/g:
   (A) an alkenyl group-containing organosilicon resin represented by formula (1), the alkenyl group-containing organosilicon resin having one or more alkenyl groups per molecule,
      [Chem. 1]

      (R¹R²₂SiO_{1/2})ₐ₁(R²₃SiO_{1/2})ₐ₂(R³₃SiO_{1/2})ₐ₃(R²₂SiO_{2/2})_{b}(R²SiO_{3/2})_{c}(SiO_{4/2})_{d} (1)

      wherein R¹'s each independently are an alkenyl group having 2 to 8 carbon atoms, R²'s each independently are a group selected from an alkyl group having 1 to 30 carbon atoms, an aryl group having 6 to 30 carbon atoms and an aralkyl group having 7 to 30 carbon atoms, R³'s each independently are a group selected from an organopolysiloxane-containing group and R², one or more R³'s included in each of the R³₃SiO_{1/2} units are organopolysiloxane-containing groups, and a1, a2, a3, b, c and d are numbers that satisfy 0 < a1 ≤ 5, 0 < a2 ≤ 400, 0 ≤ a3 ≤ 400, 0 ≤ b ≤ 320, 0 ≤ c ≤ 320, 0 < d ≤ 1,000 and 0.5 ≤ (a1 + a2 + a3)/d ≤ 1.5; and
   (B) an organohydrogenpolysiloxane represented by formula (2), the organohydrogenpolysiloxane having two or more hydrosilyl groups per molecule: in an amount such that an amount of the hydrosilyl groups is 0.5 to 1.2 moles per mole of the alkenyl groups included in the component (A),
      [Chem. 2]

      (R⁴₃SiO_{1/2})ₑ(R⁴₂SiO_{2/2})_{f}(R²SiO_{3/2})_{g}(SiO_{4/2})ₕ (2)

      wherein R²'s are the same things as above, R⁴'s each independently are a hydrogen atom or a group represented by R², two or more of R⁴'s are hydrogen atoms, and e, f, g and h are zero or a positive number, where 2 ≤ e + f + g + h < 32.
2. The cosmetic preparation according to 1, wherein the organopolysiloxane-containing group represented by R³ is one or more groups represented by formulae (3) to (6),
   [Chem. 3]

   -CₘH₂ₘ-(CH₂)₂-(SiOR²₂)ᵢ-SiR²₃ (3)

   -CₘH₂ₘ-(CH₂)₂-SiR²ⱼ₁-(OSiR²₃)₃₋ⱼ₁ (4)

   -CₘH₂ₘ-(CH₂)₂-SiR²ⱼ₁-(OSiR²ⱼ₂(OSiR²₃)₃₋ⱼ₂)₃₋ⱼ₁ (5)

   -CₘH₂ₘ-(CH₂)₂-SiR²ⱼ₁-(OSiR²j₂(OSiR²j₃(OSiR²₃)₃₋ⱼ₃)₃₋ⱼ₂)₃₋ⱼ₁ (6)

   wherein R² is the same thing as above, m is an integer of 0 ≤ m ≤ 5, i is an integer of 0 ≤ i ≤ 500 and j1 to j3 each are an integer of 0 to 2.
3. The cosmetic preparation according to 1 or 2, wherein, in formula (1), b = 0 and c = 0.
4. The cosmetic preparation according to any one of 1 to 3, wherein, in formula (2), g = 0 and h = 0.
5. The cosmetic preparation according to any one of 1 to 4, wherein the crosslinked organosilicon resin has a weight-average molecular weight of 5,000 to 1,000,000.
6. The cosmetic preparation according to any one of 1 to 5, wherein, in formula (2), 0 ≤ f < 30, two of the R⁴'s are hydrogen atoms, and the crosslinked organosilicon resin is solid at 25°C.
7. The cosmetic preparation according to any one of 1 to 6, wherein the amount of the hydrogen gas generated by the crosslinked organosilicon resin per weight under the normal condition is 0.01 to 1.2 mL/g.
8. The cosmetic preparation according to any one of 1 to 7, wherein the crosslinked organosilicon resin is soluble in a volatile oil at 25°C, the volatile oil having a boiling point of up to 250°C at 1,013 hPa.
9. The cosmetic preparation according to 8, wherein the volatile oil is one or more selected from an silicone oil, isododecane and ethanol.
10. The cosmetic preparation according to any one of 1 to 9, wherein a content of the crosslinked organosilicon resin is 0.1% to 40% by weight.
11. The cosmetic preparation according to any one of 1 to 10, the cosmetic preparation being a sunscreen cosmetic preparation or a makeup cosmetic preparation.
12. The cosmetic preparation according to any one of 1 to 11, the cosmetic preparation being a non-aqueous composition.
13. A method for producing the cosmetic preparation according to any one of 1 to 12, the method including a step of reacting:
   (A) an alkenyl group-containing organosilicon resin represented by formula (1), the alkenyl group-containing organosilicon resin having one or more alkenyl groups per molecule,
      [Chem. 4]

      (R¹R²₂SiO_{1/2})ₐ₁(R²₃SiO_{1/2})ₐ₂(R³₃SiO_{1/2})ₐ₃(R²₂SiO_{2/2})_{b}(R²SiO_{3/2})_{c}(SiO_{4/2})_{d} (1)

      wherein R¹'s each independently are an alkenyl group having 2 to 8 carbon atoms, R²'s each independently are a group selected from an alkyl group having 1 to 30 carbon atoms, an aryl group having 6 to 30 carbon atoms, and an aralkyl group having 7 to 30 carbon atoms, R³'s each independently are a group selected from an organopolysiloxane-containing group and R², one or more R³'s included in each of the R³₃SiO_{1/2} units are organopolysiloxane-containing groups, and a1, a2, a3, b, c, and d are numbers that satisfy 0 < a1 ≤ 5, 0 < a2 ≤ 400, 0 ≤ a3 ≤ 400, 0 ≤ b ≤ 320, 0 ≤ c ≤ 320, 0 < d ≤ 1,000, and 0.5 ≤ (a1 + a2 + a3)/d ≤ 1.5; with
   (B) an organohydrogenpolysiloxane represented by formula (2), the organohydrogenpolysiloxane having two or more hydrosilyl groups per molecule,
      [Chem. 5]

      (R⁴₃SiO_{1/2})ₑ(R⁴₂SiO_{2/2})_{f}(R²SiO_{3/2})_{g}(SiO_{4/2})ₕ (2)

      wherein R²'s are the same things as above, R⁴'s each independently are a hydrogen atom or a group represented by R², two or more of R⁴'s are hydrogen atoms, and e, f, g and h are zero or a positive number, where 2 ≤ e + f + g + h < 32,
      such that an amount of the hydrosilyl groups is 0.5 to 1.2 moles per mole of the alkenyl groups included in the component (A), to produce a crosslinked organosilicon resin.

### ADVANTAGEOUS EFFECTS OF INVENTION

The crosslinked organosilicon resin according to the present invention has high stability over time and a capability of forming a film that is highly resistant to oils, free of stickiness or brittleness, and highly flexible. A cosmetic preparation including the crosslinked organosilicon resin has suitable feel on use and suitable elongation and is excellent in terms of prevention of secondary adhesion.

### DESCRIPTION OF EMBODIMENTS

Details of the present invention are described below.

The cosmetic preparation according to the present invention is a cosmetic preparation including a crosslinked organosilicon resin that is a product of an addition reaction between the components (A) and (B) below, the amount of hydrogen gas generated by the crosslinked organosilicon resin per weight under a normal condition being up to 1.5 mL/g. In the present invention, the name of a component may be described in cosmetic labeling names or International Nomenclature of Cosmetic Ingredient (INCI) names. In the case where the cosmetic labeling name and INCI name of a cosmetic are the same, the English name may be omitted.

### [Component (A)]

The component (A) according to the present invention is an alkenyl group-containing organosilicon resin that is represented by average compositional formula (1) below and has one or more alkenyl groups per molecule. The components (A) may be used alone or in a combination of two or more.
[Chem. 6]

(R¹R²₂SiO_{1/2})ₐ₁(R²₃SiO_{1/2})ₐ₂(R³₃SiO_{1/2})ₐ₃(R²₂SiO_{2/2})_{b}(R²SiO_{3/2})_{c}(SiO_{4/2})_{d} (1)

[In the above formula, R¹'s each independently are an alkenyl group having 2 to 8 carbon atoms, R²'s each independently are a group selected from an alkyl group having 1 to 30 carbon atoms, an aryl group having 6 to 30 carbon atoms and an aralkyl group having 7 to 30 carbon atoms, R³'s each independently are a group selected from an organopolysiloxane-containing group and R², one or more R³'s included in each of the R³₃SiO_{1/2} units are organopolysiloxane-containing groups, and a1, a2, a3, b, c and d are numbers that satisfy 0 < a1 ≤ 5, 0 < a2 ≤ 400, 0 ≤ a3 ≤ 400, 0 ≤ b ≤ 320, 0 ≤ c ≤ 320, 0 < d ≤ 1,000, and 0.5 ≤ (a1 + a2 + a3)/d ≤ 1.5.]

### [Component (B)]

The component (B) according to the present invention is an organohydrogenpolysiloxane that is represented by average compositional formula (2) and has two or more hydrosilyl groups per molecule. The components (B) may be used alone or in a combination of two or more. The amount of addition reaction is such that the amount of the hydrosilyl groups is 0.5 to 1.2 moles per mole of the alkenyl groups included in the component (A). The above amount of the hydrosilyl groups is preferably 0.5 to 1.2 moles, is more preferably 0.8 to 1.2 moles, and is further preferably 0.9 to 1.1.
[Chem. 7]

(R⁴₃SiO_{1/2})ₑ(R⁴₂SiO_{2/2})_{f}(R²SiO_{3/2})_{g}(SiO_{4/2})ₕ (2)

[In the above formula, R²'s are the same things as above, R⁴'s each independently are a hydrogen atom or a group represented by R², two or more of R⁴'s are hydrogen atoms, and e, f, g and h are zero or a positive number, where 2 ≤ e + f + g + h < 32.]

In the above formula, R¹'s each independently are an alkenyl group having 2 to 8 carbon atoms. Specific examples thereof include a vinyl group, an allyl group, an isopropenyl group, a butenyl group, a pentenyl group, a hexenyl group, a cyclohexenyl group and an octenyl group. In particular, a vinyl group and an allyl group are preferable.

In the above formula, R²'s each independently are a group selected from an alkyl group having 1 to 30 carbon atoms, an aryl group having 6 to 30 carbon atoms and an aralkyl group having 7 to 30 carbon atoms. Among these, an alkyl group having 1 to 10 carbon atoms, an aryl group, an aralkyl group and a fluorine-substituted alkyl group are preferable. Specific examples thereof include a methyl group, an ethyl group, a propyl group, a butyl group, a pentyl group, a cyclopentyl group, a cyclohexyl group, a phenyl group, a tolyl group and a trifluoropropyl group. An alkyl group having 1 to 5 carbon atoms, a phenyl group and a trifluoropropyl group are particularly preferable. Optionally, a part of R² may include one or more groups selected from a hydroxyl group and an alkoxy group having 1 to 8 carbon atoms.

As for a1, a2, a3, b, c and d in the alkenyl group-containing organosilicon resin represented by formula (1) above, a1 satisfies 0 < a1 ≤ 5, preferably satisfies 0 < a1 ≤ 4.5, more preferably satisfies 1 ≤ a1 ≤ 4, and further preferably satisfies 1 ≤ a1 ≤ 3. If a1 is more than 5, possibility of gelation is increased and film formability also becomes degraded. a2 satisfies 0 ≤ a2 ≤ 400, preferably satisfies 0 ≤ a2 ≤ 100, and more preferably satisfies 0 ≤ a2 ≤ 50. a3 satisfies 0 ≤ a3 ≤ 400, preferably satisfies 0 ≤ a3 ≤ 100, and more preferably satisfies 0 ≤ a3 ≤ 50. If a3 is more than 400, the melting point of the resin is lowered and film formability becomes degraded accordingly. b and c satisfy 0 ≤ b ≤ 320 and 0 ≤ c ≤ 320. It is preferable that b = 0 and c = 0. a1, a2, a3 and d are values that satisfy 0 < d ≤ 1,000 and 0.5 ≤ (a1 + a2 + a3)/d ≤ 1.5 and are preferably values that satisfy 0.7 ≤ (a1 + a2 + a3)/d ≤ 1.2. If (a1 + a2 + a3)/d is less than the above lower limit, the degree of crosslinking is increased, molecular weight is increased accordingly, and it becomes gelatinous. If (a1 + a2 + a3)/d is more than the above upper limit, molecular weight is reduced and film formability becomes degraded.

The alkenyl group-containing organosilicon resin represented by formula (1) above is constituted by an essential structure consisting of a Q unit (SiO_{4/2}) and an M unit (R²₃SiO_{1/2} and R¹R²₂SiO_{1/2}) and an optional structure consisting of a D unit (R²₂SiO_{2/2}) and a T unit (R²SiO_{3/2}). The above resin may be either solid or liquid at 25°C and is preferably solid in consideration of film formability. Examples thereof include an MQ resin, an MTQ resin, an MDQ resin and an MDTQ resin. The weight-average molecular weight of the above resin is preferably 1,000 to 30,000 and is more preferably 3,000 to 15,000 in consideration of performance and workability, such as filtration. Note that the above weight-average molecular weight can be determined as a polystyrene-equivalent weight-average molecular weight by gel permeation chromatography (GPC) analysis.

In the organohydrogenpolysiloxane represented by formula (2) above, which has two or more hydrosilyl groups per molecule, R⁴'s each independently are a hydrogen atom or a monovalent hydrocarbon group that has 1 to 30 carbon atoms and does not have an aliphatic unsaturated bond, and two or more of R⁴'s are hydrogen atoms.

In formula (2) above, e, f, g and h are zero or a positive number and are selected so as to satisfy 2 ≤ e + f + g + h < 32. It is preferable that g = 0 and h = 0. It is more preferable that e = 2, 0 ≤ f < 30, g = 0 and h = 0. It is further preferable that e = 2, 0 ≤ f ≤ 20, g = 0 and h = 0. If the number of silicon atoms included in the component (B) is 32 or more, the crosslinked organosilicon resin is likely to become gelatinous as a result of holding the solvent and consequently form a sticky film after the vaporization of the solvent. When the number of silicon atoms included in the component (B) is less than 32, the crosslinked organosilicon resin is likely to become liquidus because being soluble in the solvent. This increases the likelihood of formation of a non-sticky film after the vaporization of the solvent.

R³'s each independently are an organopolysiloxane-containing group or R² above. Examples of the organopolysiloxane-containing group include the groups represented by general formulae (3) to (6) below. In each of the R³₃SiO_{1/2} units, one or more R³'s are organopolysiloxane-containing groups. Optionally, a part of R³'s may be a hydroxyl group.
[Chem. 8]

-CₘH₂ₘ-(CH₂)₂-(SiOR²₂)ᵢ-SiR²₃ (3)

-CₘH₂ₘ-(CH₂)₂-SiR²ⱼ₁-(OSiR²₃)₃₋ⱼ₁ (4)

-CₘH₂ₘ-(CH₂)₂-SiR²j₁-(OSiR²ⱼ₂(OSiR²₃)₃₋ⱼ₂)₃₋ⱼ₁ (5)

-CₘH₂ₘ-(CH₂)₂-SiR²ⱼ₁-(OSiR²j₂(OSiR²ⱼ₃(OSiR²₃)₃₋ⱼ₃)₃₋ⱼ₂)₃₋ⱼ₁ (6)

(in the above formula, R² is the same thing as above, n and i are integers that satisfy 0 ≤ n ≤ 5 and 0 ≤ i ≤ 500, and j1 to j3 are an integer of 0 to 2.)

m is an integer of 0 ≤ m ≤ 5 and is preferably 0 ≤ m ≤ 2. i is an integer of 0 ≤ i ≤ 500, is preferably 1 ≤ i ≤ 100, and is more preferably 1 ≤ i ≤ 50. If i is more than 500, the melting point of the resin is lowered and film formability becomes degraded accordingly. j1 to j3 each are an integer of 0 to 2.

In formula (1) above, it is preferable that b = 0 and c = 0. When b and c are zero, the alkenyl group-containing organosilicon resin does not include a soft skeleton, such as the D or T unit, and is composed only of the M and Q units. Using an alkenyl group-containing organosilicon resin that does not include the D or T unit as a raw material enables the crosslinked organosilicon resin, which is the product of the addition reaction, to form a tough film.

In formula (2) above, it is preferable that g = 0 and h = 0. When g and h are zero, the organohydrogenpolysiloxane does not include a branched component, such as the T or Q unit, and has a linear molecular structure composed only of the M and D units. Using a linear organohydrogenpolysiloxane as a raw material enables the crosslinked organosilicon resin, which is the product of the addition reaction, to form a soft film.

Two or more groups represented by formula (2) may be included. The larger the chain length of the group represented by formula (2), the greater the effect to impart softness to the organosilicon resin. Thus, for example, addition of two groups represented by formula (2) having different chain lengths enables the physical properties of the film to be controlled.

### [Physical Properties of Crosslinked Organosilicon Resin]

The weight-average molecular weight of the crosslinked organosilicon resin according to the present invention is preferably 5,000 to 1,000,000, is more preferably 8,000 to 500,000, and is further preferably 10,000 to 500,000. It is more preferable that the above weight-average molecular weight fall within the above range in consideration of performance and workability, such as filtration. Note that the above weight-average molecular weight can be determined as a polystyrene-equivalent weight-average molecular weight by gel permeation chromatography (GPC) analysis (the same applies hereinafter).

The crosslinked organosilicon resin may be solid, gelatinous, or liquid at 25°C. A film can be readily formed by, for example, dissolving the crosslinked organosilicon resin in a liquid oil and vaporizing it. Although this film is tough and brittle when having not been crosslinked, the brittleness of the film is improved by crosslinking and a soft film free of stickiness can be prepared. The crosslinked organosilicon resin is preferably solid or gelatinous and is more preferably solid in consideration of film formability. The film-forming ability can be determined by dropping 1.5 g of a solution prepared by diluting the crosslinked organosilicon resin with isododecane or decamethylcyclopentasiloxane to a concentration of 30% by weight onto PTFE (fluororesin), subsequently performing drying at 105°C for 3 hours, and determining whether a self-supporting film is formed. In the case where the film is not formed, oils exude through cracks or the like formed in the film and oil resistance becomes significantly degraded. This results in poor followability to the skin and unnatural finish.

The crosslinked organosilicon resin according to the present invention can be further suitably used as a film-forming agent. Although the organosilicon resin forms a brittle and tough film when having not been crosslinked, subsequent to the crosslinking, the crosslinked organosilicon resin is improved in terms of brittleness and capable of forming a soft film free of stickiness. This is because, although the organosilicon resin forms a tough film when having not been crosslinked, performing crosslinking with soft chains imparts softness to the film. In general, a hard film has low flexibility, while a highly flexible film is soft. Thus, it has been considered that the strength and flexibility of a film are mutually incompatible. However, the crosslinked organosilicon resin according to the present invention has high flexibility and is therefore excellent in terms of followability, although being capable of forming a tough film.

Moreover, a film formed of the crosslinked organosilicon resin has markedly high resistance to oils, such as sebum, compared with a film formed of the organosilicon resin that has not been crosslinked. In general, the higher the molecular weight of an organosilicon resin, the higher the oil resistance of the organosilicon resin. Since there is a limitation on the increase in the molecular weight of an organosilicon resin, oil resistance also has its critical point. Crosslinking an organosilicon resin with a crosslinking agent results in a virtual increase in the molecular weight of the organosilicon resin and consequently increases the critical point. Thus, the crosslinked organosilicon resin has high oil resistance which cannot be achieved by the organosilicon resins known in the related art.

When f in formula (2) above is an integer that satisfies 0 < f < 30, the crosslinked organosilicon resin is solid at 25°C, that is, a crosslinked organosilicon resin having particularly high film formability can be produced.

When f in formula (2) above satisfies 0 ≤ f < 30 and two of R⁴'s are hydrogen atoms, the crosslinked organosilicon resin is solid at 25°C, that is, a crosslinked organosilicon resin having particularly high film formability can be produced. If f is 30 or more or three or more of R⁴'s are hydrogen atoms, the possibility of the resin gelating upon the removal of the diluting solvent may be increased disadvantageously. In such a case, a gel-derived feel may be sensed although film formability is achieved.

When a1 satisfies 0 < a1 ≤ 3 in formula (1) above, f satisfies 0 ≤ f < 20 in formula (2) above, and two of R⁴'s are hydrogen atoms, the crosslinked organosilicon resin is solid at 25°C, that is, a crosslinked organosilicon resin having particularly high film formability can be produced. Moreover, the film formed using the resin has particularly high flex resistance and oil resistance.

The amount of the hydrogen gas generated from the crosslinked organosilicon resin per weight under normal conditions is up to 1.5 mL. If the above amount is more than 1.5 mL/g, the amount of hydrogen gas generated over time and the likelihood of viscosity increasing over time as a result of reaction between the residual hydroxyl or alkoxy groups and hydrosilyl groups are increased. This degrades stability over time. The amount of hydrogen gas generated is preferably 0.01 to 1.2 mL/g and is more preferably 0.02 to 1.0 mL/g.

The amount of hydrogen gas generated per weight can be calculated on the basis of the volume of the hydrogen gas generated by the reaction of the hydrosilyl groups with a base. Examples of the calculation method include, but are not limited to, the following method.

### <Method for Determining Amount of Hydrogen Gas>

To a mixed solution of 50 g of the crosslinked organosilicon resin which is diluted to 50% by weight with decamethylcyclopentasiloxane and 10 g of 1-butanol, 10 g of a 20-weight% aqueous sodium hydroxide solution is added dropwise. The volume of the hydrogen gas generated is divided by the net weight of the crosslinked organosilicon resin to obtain the amount of the hydrogen gas generated per weight.

### [Production Method]

The crosslinked organosilicon resin can be synthesized by any method known in the related art. For example, the crosslinking can be performed by reacting the surface silanol groups of an organosilicon resin with an organopolysiloxane having two terminal hydroxyl groups. However, it is difficult to precisely control the amount of organopolysiloxane used for crosslinking because of the difficulty in perfectly controlling the amount of silanol groups present on the surface of the organosilicon resin. The synthesis can also be performed by the addition reaction of an organosilicon resin having a hydrosilyl group with an organopolysiloxane having two terminal alkenyl groups. However, since the hydrosilyl groups of the organosilicon resin have poor reactivity, the residual hydrosilyl groups cause a reaction over time to increase viscosity and generate a hydrogen gas disadvantageously. Therefore, one of the preferable methods for producing a crosslinked organosilicon resin crosslinked with silicone is to perform the synthesis by the addition reaction of an organosilicon resin having an alkenyl group with an organopolysiloxane having two terminal hydrosilyl groups.

The method for producing the crosslinked organosilicon resin in which a hydrosilylation reaction is conducted is described in further details below.

In the step of the hydrosilylation reaction between the alkenyl group-containing organosilicon resin represented by average compositional formula (1) above and the organohydrogenpolysiloxane represented by formula (2) above, the molar ratio of terminal hydrosilyl group/unsaturated group can be selected from 0.5 to 2.0. The above molar ratio is preferably 0.5 to 1.2, is more preferably 0.8 to 1.2, and is further preferably 0.9 to 1.1. If the above ratio is excessively high, the amount of residual hydrosilyl groups is excessively large and stability over time may become degraded consequently.

The above hydrosilylation reaction is preferably conducted in the presence of a platinum or rhodium catalyst. Preferable examples thereof include chloroplatinic acid, alcohol-modified chloroplatinic acid and chloroplatinic acid-vinyl siloxane complex. The amount of the catalyst used is adjusted such that the platinum or rhodium content is preferably up to 50 ppm and is more preferably up to 20 ppm, because an excessive amount of the catalyst causes staining of the sample.

The above addition reaction may be conducted in the presence of an organic solvent as needed. Examples of the organic solvent include cyclic organopolysiloxanes, such as octamethylcyclotetrasiloxane, decamethylcyclopentasiloxane and dodecamethylcyclohexasiloxane; aromatic hydrocarbons, such as toluene and xylene; ketone-based organic solvents, such as acetone, methyl ethyl ketone, diethyl ketone and methyl isobutyl ketone; aliphatic hydrocarbons, such as hexane, heptane, octane and cyclohexane; and aliphatic alcohols, such as methanol, ethanol, 1-propanol, 2-propanol, 1-butanol, 2-methyl-1-propanol, 2-butanol, 2-methyl-2-propanol, 1-pentanol, 2-methylbutanol, 2-pentanol, 1-hexanol, 2-methylpentanol, 1-heptanol, 1-octanol, 1-nonanol, 1-decanol, phenol, benzyl alcohol, ethylene glycol and 1,2-propylene glycol. In particular, ethanol, 1-propanol and 2-propanol are preferable in consideration of reactivity.

The amount of the solvent used is preferably 1% to 80% by weight and is more preferably 5% to 50% by weight of the total amount of the reaction liquid (system). When the amount of the solvent used falls within the above range, the inside of the reaction system can be kept homogeneous and the reaction can be conducted with efficiency.

The conditions under which the addition reaction is conducted are not limited; it is preferable that heating be performed for about 1 to 10 hours under reflux at 50°C to 150°C or, more preferably, 80°C to 120°C.

Optionally, a step of removing the rhodium or platinum catalyst with active carbon subsequent to the addition reaction may be conducted. The amount of the active carbon used is preferably 0.001% to 5.0% by weight and is more preferably 0.01% to 1.0% by weight of the total amount of the system. When the amount of the active carbon used falls within the above range, the staining of the sample can be further limited.

Optionally, a step of removing the residual hydrosilyl groups subsequent to the addition reaction may be conducted. In particular, in the case where the crosslinked organosilicon resin is used in the production of cosmetic preparations or the like, the hydrosilyl groups may be deactivated over time by a dehydrogenation reaction. This involves the generation of a hydrogen gas, which is disadvantageous in terms of safety. Therefore, in such a case, it is preferable to conduct the step of removing the hydrosilyl groups.

One of the methods for removing the hydrosilyl groups is to hydrolyze the unreacted hydrosilyl groups by the addition of a basic catalyst and subsequently perform neutralization by the addition of an acidic catalyst to the basic catalyst at the same molar equivalent. Examples of the basic catalyst include a strongly basic catalyst and a weakly basic catalyst. Examples of the strongly basic catalyst include alkali metal hydroxides, such as lithium hydroxide, sodium hydroxide, potassium hydroxide, calcium hydroxide and barium hydroxide. Examples of the weakly basic catalyst include alkali metal carbonates, such as sodium carbonate and calcium carbonate; and alkali metal hydrogen carbonates, such as sodium hydrogen carbonate and potassium hydrogen carbonate. In particular, a strongly basic catalyst is preferably used in order to facilitate the dehydrogenation reaction. Specifically, sodium hydroxide is preferable. Examples of the acidic catalyst include inorganic acids, such as hydrochloric acid, sulfuric acid, sulfurous acid, fuming sulfuric acid and phosphoric acid; sulfonic acids, such as p-toluenesulfonic acid, methanesulfonic acid and trifluoromethanesulfonic acid; and carboxylic acids, such as oxalic acid, formic acid, an acetic acid, propionic acid, benzoic acid, citric acid and trifluoroacetic acid.

In general, it is preferable to use the above acid or base in combination of water and perform heating at a temperature equal to or less than the boiling point of water, rather than using the acid or base alone. By the above step, the hydrosilyl groups (SiH groups) are converted to hydroxysilyl groups (SiOH groups). Note that treating the crosslinked organosilicon resin with a base catalyst causes a reaction of the silanol and alkoxy groups included in the organosilicon resin, which results in changes in physical properties. Therefore, it is not preferable to remove the hydrosilyl groups by this method.

### [Cosmetic Preparation]

When the crosslinked organosilicon resin according to the present invention (hereinafter, may be referred to as "component (a)") is added to a cosmetic preparation, the crosslinked organosilicon resin serves as a film-forming agent and the cosmetic preparation is free of stickiness upon application and has an excellent feel on use, suitable resistance to water and oils, and suitable adhesion to the skin and is therefore excellent in terms of elongation, finish, water resistance, oil resistance, long-lasting performance (durability) and rubfastness, such as prevention of secondary adhesion.

The crosslinked organosilicon resin according to the present invention may be formed into an O/W emulsion, which serves as an intermediate composition, in consideration of ease of addition of the crosslinked organosilicon resin to an aqueous or emulsion composition. That is, an O/W emulsion prepared by dispersing a solution of the crosslinked organosilicon resin according to the present invention in an oil, which serves as a disperse phase, in a water phase, which serves as a continuous phase, is the intermediate composition. The method for preparing the O/W emulsion is not limited; the O/W emulsion can be prepared by any method known in the related art. For example, the emulsification may be performed using only one or two or more activators having an HLB of 10 or more. An activator having an HLB of less than 10, a higher alcohol and the like may be used as a stabilizer. A carbomer or the like may be added to the water phase in order to increase viscosity.

The content of the crosslinked organosilicon resin (a) according to the present invention is preferably 0.1% to 40% by weight and is further preferably 0.1% to 10% by weight of the total amount of the cosmetic preparation. If the above content is less than 0.1% by weight, sufficiently high oil resistance cannot be achieved. If the above content is more than 40% by weight, feel on use may become degraded.

The crosslinked organosilicon resin according to the present invention may be dissolved in a solvent and used in the form of a solution. In the case where a volatile oil having a boiling point of up to 250°C at 1,013 hPa is used as a solvent, a film can be formed as a result of vaporization of the solvent and the effect of the film-forming agent can be further produced. The volatile oil having a boiling point of up to 250°C at 1,013 hPa is preferably one or more selected from a silicone oil, isododecane and ethanol. The above oils can be used alone or in combination of two or more.

### (b) Volatile Oil

It is preferable to use a volatile oil that allows a film to be formed at an early stage subsequent to the application of the cosmetic preparation and to produce the advantageous effects. In this regard, it is preferable to use a volatile oil having a boiling point of up to 250°C. Among these, in particular, silicone oils, such as cyclotetrasiloxane (INCI), cyclopentasiloxane (INCI), cyclohexasiloxane (INCI), dimethicone (INCI), disiloxane (INCI), trisiloxane (INCI), methyl trimethicone (INCI), ethyl trisiloxane (INCI) and ethyl methicone (INCI), such as decamethylcyclopentasiloxane; and hydrocarbon oils, such as isododecane, (INCI), undecane (INCI), dodecane (INCI) and hydrogenated polyisobutene (Labeling name (INCI: Hydrogenated Polyisobutene)), are preferable. Examples of the volatile oil further include lower alcohols, such as ethanol (Labeling name (INCI: Alcohol)) and isopropanol (Labeling name (INCI: Isopropyl Alcohol)); and acetate esters, such as ethyl acetate (Labeling name (INCI: Ethyl Acetate)) and butyl acetate (Labeling name (INCI: Butyl Acetate)). The above volatile oils can be selected appropriately in accordance with the type of the cosmetic base included in the cosmetic preparation and used in combination with one another. For example, in the case where the cosmetic base is silicone-based, it is appropriate to select a silicone oil in order to enhance the compatibility of the entire composition of the cosmetic preparation. It is preferable to use a silicone oil in order to achieve a suitable feel. Examples of commercial products thereof include TMF-1.5, KF-995, KF-96L-1cs, KF-96L-1.5cs, KF-96L-2cs and KF-4422 produced by Shin-Etsu Chemical Co., Ltd. The amount of the volatile oil (B) added is preferably 0.1% to 70% by weight and is further preferably 0.1% to 20% by weight of the total amount of the cosmetic preparation or the amount of the solution product. If the amount of the volatile oil (B) added is less than 0.1% by weight, solubility may become degraded. If the amount of the volatile oil (B) added is more than 70% by weight, a feeling of dryness may be sensed.

### [Other Components]

The cosmetic preparation according to the present invention may include, as other components, various components included in common cosmetic preparations. Examples of the other components that may be included in the cosmetic preparation include (1) an oil other than the component (b), (2) an aqueous component, (3) a surfactant, (4) a powder, (5) a composition including a crosslinked organopolysiloxane and an oil that is liquid at room temperature, (6) a film-forming agent other than the component (A), (7) an ultraviolet absorbing/scattering agent and (8) another additive. The above components may be used alone or in combination of two or more appropriately. The above components are selected and used appropriately in accordance with the type and the like of the cosmetic preparation. The content of the other components may be set to the content known in the related art in accordance with the type and the like of the cosmetic preparation.

### (1) Oil Other Than Component (B)

The cosmetic preparation according to the present invention may include an oil other than the component (B). The oil is nonvolatile and may be any of solid, semi-solid and liquid at room temperature (25°C). Examples of the oil include a silicone oil, a silicone wax, a natural animal or vegetable oil or fat, a semisynthetic oil or fat, a hydrocarbon oil, a higher alcohol, a fatty acid, an ester oil, a fluorocarbon oil and an ultraviolet absorber.

### • Silicone Oil

Examples of the silicone oil include alkyl-modified silicones, such as hexyl dimethicone (INCI); long-chain alkyl-modified silicones, such as caprylyl methicone (INCI); low- to high-viscosity, linear or branched organopolysiloxanes, such as phenyl trimethicone (INCI), diphenyl dimethicone (INCI), diphenylsiloxy phenyl trimethicone (INCI), tetraphenyl dimethyl disiloxane (INCI) and methylhydrogen polysiloxane; amino-modified organopolysiloxanes, such as amodimethicone (INCI) and aminopropyl dimethicone (INCI); pyrrolidone-modified organopolysiloxanes, such as PCA dimethicone (INCI); silicone rubbers, such as pyrrolidonecarboxylic acid-modified organopolysiloxane, dimethicone (INCI), gum-like dimethylpolysiloxane having a high degree of polymerization, gum-like amino-modified organopolysiloxane and gum-like dimethylsiloxane-methyl phenyl siloxane copolymer; and a low-viscosity organopolysiloxane solution of a silicone gum or rubber and a solution of an amino acid-modified silicone, fluorine-modified silicone, a silicone resin, or a silicone resin. Examples of commercial silicone oils include KF-96A-6cs, KF-54, KF-54HV and KF-56A produced by Shin-Etsu Chemical Co., Ltd.

### • Solid, Oil-Based Component

In the present invention, in the case where the cosmetic preparation is to be solidified, it is preferable to use an oil-based component that is solid at 25°C. Examples of the oil-based component that is solid at 25°C include a wax, a hydrocarbon, an ester, a higher alcohol, and a higher fatty acid that preferably have a melting point of 40°C or more and more preferably have a melting point of 60°C to 110°C. The above oil-based component is not limited and may be any material that is commonly included in a cosmetic preparation. Specific examples thereof include vegetable waxes, such as a carnauba wax (INCI: Copernicia Cerifera (Carnauba) Wax), a sugarcane wax, a candelilla wax (INCI: Euphorbia Cerifera (Candelilla) Wax), a refined candelilla wax, a rice wax, a Japan wax, a jojoba wax, a kapok wax, a rice bran wax, a myrica cerifera fruit wax, shea butter, cacao butter, a Japanese wax (INCI: Rhus Succedanea Fruit Wax), a montan wax (INCI: Montan Wax) and hydrogenated castor oil isostearate; animal waxes, such as a beeswax, a beef fat, a beef bone fat, lard (INCI: Lard), a horse fat (INCI: Horse Fat), a mutton fat, lanolin (INCI: Lanolin), an insect wax, a shellac wax and spermaceti; semisynthetic waxes, such as a lanolin ester, a lanolin fatty acid ester and a beeswax acid ester; hardened oils, such as a hardened castor oil and a hardened coconut oil; hydrocarbon waxes, such as solid paraffin, polyethylene, ceresin, ozokerite and a microcrystalline wax; wax esters, such as synthetic beeswax; amino acid stearyl alcohols, such as dioctyldodecyl lauroyl glutamate; fatty acids, such as stearic acid and behenic acid; silicone waxes, such as an acrylic silicone resin of an acrylic-silicone graft or block copolymer (e.g., acrylic-silicone graft copolymers KP-561P and 562P produced by Shin-Etsu Chemical Co., Ltd.), and derivatives of the above materials. It is preferable to use one or two or more selected from the above materials.

### • Natural Animal and Vegetable Oils and Fats and Semisynthetic Oils and Fats

Examples of the natural animal and vegetable oils and semisynthetic oils include germ oils, such as an avocado oil (Labeling name (INCI: Persea Gratissima (Avocado) Oil)), a linseed oil (Labeling name (INCI: Linum Usitatissimum (Linseed) Seed Oil)), an almond oil (Labeling name (INCI: Prunus Amygdalus Dulcis (Sweet Almond) Oil)), a perilla oil (Labeling name), an olive oil (Labeling name (INCI: Olea Europaea (Olive) Fruit Oil)), a torreya californica oil (Labeling name (INCI: Torreya Californica (California Nutmeg) Oil)), a citronella oil (Labeling name (INCI: Cymbopogon Nardus (Citronella) Oil)), a torreya nucifera seed oil (Labeling name (INCI: Torreya Nucifera Seed Oil)), an apricot kernel oil (Labeling name (INCI: Kyounin Yu)), a wheat germ oil (Labeling name (INCI: Triticum Vulgare (Wheat) Germ Oil)), a sesame oil (Labeling name (INCI: Sesamum Indicum (Sesame) Seed Oil)), a rice germ oil (Labeling name (INCI: Oryza Sativa (Rice) Germ Oil)), a rice bran oil (Labeling name (INCI: Oryza Sativa (Rice) Bran Oil)), a sasanqua oil (Labeling name (INCI: Camellia Kissi Seed Oil)), a safflower oil (Labeling name (INCI: Carthamus Tinctorius (Safflower) Seed Oil)), a soybean oil (Labeling name (INCI: Glycine Soja(Soybean)Oil)), a tea oil (Labeling name (INCI: Camellia Sinensis Seed Oil)), a camellia oil (Labeling name (INCI: Camellia Japonica Seed Oil)), an evening primrose oil (Labeling name (INCI: Oenothera Biennis (Evening Primrose) Oil)), a rapeseed oil (Labeling name), and a corn germ oil (Labeling name (INCI: Zea Mays (Corn) Germ Oil)); natural vegetable oils, such as a persic oil (Labeling name), a palm oil (Labeling name (INCI: Elaeis Guineensis (Palm) Oil)), a palm kernel oil (Labeling name (INCI: Elaeis Guineensis (Palm) Kernel Oil)), a castor oil (Labeling name (INCI: Ricinus Communis (Castor) Seed Oil)), a sunflower oil (Labeling name (INCI: Helianthus Annuus (Sunflower) Seed Oil)), a grape seed oil (Labeling name (INCI: Vitis Vinifera (Grape) Seed Oil)), a jojoba seed oil (Labeling name (INCI: Simmondsia Chinensis (Jojoba) Seed Oil)), a macadamia seed oil (Labeling name (INCI: Macadamia Ternifolia Seed Oil)), a meadowfoam oil (Labeling name (INCI: Limnanthes Alba (Meadowfoam) Seed Oil)), a cotton seed oil (Labeling name (INCI: Gossypium Herbaceum (Cotton) Seed Oil)), coconut oil (Labeling name (INCI: Cocos Nucifera (Coconut) Oil)) and a peanut oil (Labeling name (INCI: Arachis Hypogaea (Peanut) Oil)); natural animal oils, such as a shark liver oil (Labeling name (INCI: Shark Liver Oil)), a cod liver oil (Labeling name (INCI: Cod Liver Oil)), a fish liver oil (Labeling name (INCI: Fish Liver Oil)), a turtle oil (Labeling name (INCI: Turtle Oil)), a mink oil (Labeling name (INCI: Mink Oil)) and an egg yolk oil (Labeling name (INCI: Egg Oil)); and semisynthetic oils and fats, such as a hydrogenated coconut oil (Labeling name (INCI: Hydrogenated Coconut Oil)) and liquid lanolin (Labeling name (INCI: Lanolin Oil)).

### • Hydrocarbon Oil

Examples of the hydrocarbon oil include linear and branched hydrocarbon oils. Specific examples thereof include isoparaffins, such as olefin oligomer (INCI) and (C13-14) isoparaffin (INCI); and alkanes, such as isohexadecane (INCI), hydrogenated polyisobutene (Labeling name (INCI: Hydrogenated Polyisobutene)), squalane (INCI), mineral oil (INCI), coconut alkanes (INCI), (C13-15) alkane (INCI) and vaseline (Labeling name (INCI: Petrolatum).

### • Higher Fatty Acid

Examples of the higher fatty acid include hydroxystearic acid (Labeling name (INCI: Hydroxystearic Acid)).

### • Higher Alcohols

Examples of the higher alcohols include linear saturated alcohols having 6 or more carbon atoms, such as lauryl alcohol (INCI), hexyl decanol (INCI), oleyl alcohol (INCI), isostearyl alcohol (INCI), octyldodecanol (INCI), decyltetradecanol (INCI), myristyl alcohol (INCI), cetyl alcohol (INCI), stearyl alcohol (INCI) and behenyl alcohol (INCI); and batyl alcohol (INCI). Examples of the higher alcohols further include sterols, such as cholesterol (INCI), sitosterol (Labeling name (INCI: Beta-Sitosterol)), phytosterols (INCI) and lanosterol (INCI).

### • Ester Oils

Examples of the ester oil include diisobutyl adipate (Labeling name (INCI: Diisobutyl Adipate)), dihexyldecyl adipate (Labeling name), diheptylundecyl adipate (Labeling name (INCI: Diheptylundecyl Adipate)), dibutyl adipate (Labeling name (INCI: Dibutyl Adipate)), BG di(caprylate/caprate) (Labeling name (INCI: Butylene Glycol Dicaprylate/Dicaprate)), isostearyl isostearate (Labeling name (INCI: Isostearyl Isostearate)), isocetyl isostearate (Labeling name (INCI: Isocetyl Isostearate)), trimethylolpropane triisostearate (Labeling name (INCI: Trimethylolpropane Triisostearate)), glycol diethylhexanoate (Labeling name (INCI: Glycol Diethylhexanoate)), cetyl ethylhexanoate (Labeling name (INCI: Cetyl Ethylhexanoate)), trimethylolpropane triethylhexanoate (Labeling name (INCI: Trimethylolpropane Triethylhexanoate)), pentaerythrityl tetraethylhexanoate (Labeling name (INCI: Pentaerythrityl Tetraethylhexanoate)), cetyl octanoate (Labeling name (INCI: Cetyl Ethylhexanoate)), octyldodecyl esters, such as octyldodecyl stearoyloxy stearate (Labeling name (INCI: Octyldodecyl Stearoyl Stearate)), oleyl oleate (Labeling name (INCI: Oleyl Oleate)), octyldodecyl oleate (Labeling name (INCI: Octyldodecyl Oleate)), decyl oleate (Labeling name (INCI: Decyl Oleate)), neopentyl glycol dioctanoate (Labeling name (INCI: Neopentyl Glycol Diethylhexanoate)), neopentyl glycol dicaprate (Labeling name (INCI: Neopentyl Glycol Dicaprate)), diethylhexyl succinate (Labeling name (INCI: Diethylhexyl Succinate)), amyl acetate (Labeling name (INCI: Amyl Acetate)), ethyl acetate (Labeling name (INCI: Etyl Acetate)), butyl acetate (Labeling name (INCI: Butyl Aceetate)), isocetyl stearate (Labeling name (INCI: Isocetyl Stearate)), butyl stearate (Labeling name (INCI: Butyl Stearate)), diisopropyl sebacate (Labeling name (INCI: Diisopropyl Sebacate)), diethylhexyl sebacate (Labeling name (INCI: Diethylhexyl Sebacate)), isononyl isononanoate (Labeling name (INCI: Isononyl Isononanoate)), isotridecyl isononanoate (Labeling name (INCI: Isotridecyl Isononanoate)), palmitic acid esters, such as isopropyl palmitate (Labeling name (INCI: Isopropyl Palmitate)), ethylhexyl palmitate (Labeling name (INCI: Ethylhexyl Isopalmitate)) and hexyldecyl palmitate (Labeling name (INCI: Isocetyl Palmitate, Hexyldecyl Palmitate)), myristic acid esters, such as isopropyl myristate (Labeling name (INCI: Isopropyl Myristate)), octyldodecyl myristate (Labeling name (INCI: Octyldodecyl Myristate)) and myristyl myristate (Labeling name (INCI: Myristyl Myristate)), ethylhexyl laurate (Labeling name (INCI: Ethylhexyl Laurate)), hexyl laurate (Labeling name (INCI: Hexyl Laurate)), dioctyldodecyl lauroyl glutamate (Labeling name (INCI: Dioctyldodecyl Lauroyl Glutamate)), isopropyl lauroyl sarcosinate (Labeling name (INCI: Isopropyl Lauroyl Sarcosinate)) and coco-alkyl (caprylate/caprate) (Labeling name (INCI: Coco-Caprylate·Caprate)).

Among the above ester oils, examples of glyceride oils include triethylhexanoin (INCI), glyceryl tri(caprylate/caprate) (Labeling name (INCI: Caprylic/Capric Triglyceride)) and triglyceryl (caprylate/caprate/succinate) (Labeling name (INCI: Caprylic/Capric/Succinic Triglyceride)).

### • Fluorocarbon Oils

Examples of the fluorocarbon oils include perfluorodecalin (INCI), perfluorononyl dimethicone (INCI) and perfluoromethylcyclopentane (INCI).

### • Ultraviolet Absorbers

Examples of the ultraviolet absorber include homosalate (INCI), octocrylene (INCI), t-butylmethoxydibenzoylmethane (Labeling name (INCI: Butyl Methoxydibenzoylmethane)), ethylhexyl salicylate (INCI: Ethylhexyl Salicylate), diethylamino hydroxybenzoyl hexyl benzoate (Labeling name (INCI: Diethylamino Hydroxybenzoyl Hexyl Benzoate)), oxybenzone-6 (Labeling name (INCI: Benzophenone-6)), oxybenzone-9 (Labeling name (INCI: Benzophenone-9)), oxybenzone-1 (Labeling name (INCI: Benzophenone-1)), polysilicone-15 (INCI), octyl dimethoxybenzylidene dioxoimidazolidine propionate (Labeling name (INCI: Ethylhexyl Dimethoxybenzylidene Dioxoimidazolidine Propionate)), oxybenzone-2 (Labeling name (INCI: Benzophenone-2)), terephthalylidene dicamphor sulfonic acid (Labeling name (INCI: Terephthalylidene Dicamphor Sulfonic Acid)), ethylhexyl triazone (INCI), methylbis(trimethylsiloxy)silyl isopentyl trimethoxycinnamate (Labeling name (INCI: Isopentyl Trimethoxycinnamate Trisiloxane)), drometrizole trisiloxane (INCI), ethylhexyl dimethyl PABA (Labeling name (INCI: Ethylhexyl Dimethyl PABA)), isopropyl p-methoxycinnamate (Labeling name (INCI: Isopropyl Methoxycinnamate)), ethylhexyl methoxycinnamate (Labeling name (INCI: Ethylhexyl Methoxycinnamate)), bis-ethylhexyloxyphenol methoxyphenyl triazine (Labeling name (INCI: Bis-Ethylhexyloxyphenol Methoxyphenyl Triazine)), oxybenzone-3 (Labeling name (INCI: Benzophenone-3)), oxybenzone-4 (Labeling name (INCI: Benzophenone-4)), oxybenzone-5 (Labeling name (INCI: Benzophenone-5)), phenylbenzimidazole sulfonic acid (Labeling name (INCI: Phenylbenzimidazole Sulfonic Acid)), methylene bis-benzotriazolyl tetramethylbutylphenol (Labeling name (INCI: Methylene Bis-Benzotriazolyl Tetramethylbutylphenol)), glyceryl dimethoxycinnamate ethylhexanoate (Labeling name (INCI: Glyceryl Ethylhexanoate Dimethoxycinnamate)), glyceryl PABA (Labeling name (INCI: Glyceryl PABA)), diisopropyl methyl cinnamate (Labeling name (INCI: Diisopropyl Methyl Cinnamate)), cinoxate (INCI) and ethylhexyl dimethoxybenzylidene dioxoimidazolidine propionate (Labeling name (INCI: Ethylhexyl Dimethoxybenzylidene Dioxoimidazolidine Propionate)). It is possible to use a UVA absorber (e.g., hexyl diethylamino hydroxybenzoyl benzoate) in combination with a UVB absorber (e.g., ethylhexyl methoxycinnamate). It is also possible to use any of the above absorbers in combination with an appropriate one.

The content of the oil (2) other than the component (b) is preferably 0.1% to 30% by weight, is more preferably 0.1% to 20% by weight, and is further preferably 0.1% to 10% by weight of the total amount of the cosmetic preparation. If the above content is less than 0.1% by weight, solubility may become degraded. If the above content is more than 30% by weight, stickiness may be sensed and oil resistance may become degraded.

### (2) Aqueous Component

The aqueous component is not limited and may be any aqueous component that is commonly included in cosmetic preparations. Specific examples thereof include water and sugar alcohols, such as sorbitol (INCI), maltose (INCI) and xylitol (INCI). Specific examples thereof further include polyhydric alcohols, such as BG (Labeling name (INCI: Butylene Glycol)), PG (Labeling name (INCI: Propylene Glycol)), DPG (Labeling name (INCI: Dipropylene Glycol)), pentylene glycol (INCI), 1,10-decanediol (INCI), octanediol (INCI), 1,2-hexanediol (INCI), erythritol (INCI), glycerin (INCI), diglycerin (INCI) and polyethylene glycol; and humectants, such as glucose (INCI), glyceryl glucoside (INCI), betaine (INCI), Na chondroitin sulfate (Labeling name (INCI: Sodium Chondroitin Sulfate)), PCA-Na (Labeling name (INCI: Sodium PCA)), methyl gluceth-10 (INCI), methyl gluceth-20 (INCI), hyaluronic acid, egg yolk lecithin, soy lecithin, phosphatidylcholine, phosphatidylethanolamine, phosphatidylserine, phosphatidylglycerol, phosphatidylinositol and sphingophospholipid.

### (3) Surfactants

Examples of the surfactant include nonionic, anionic, cationic and zwitterionic activators. The surfactant is not limited and may be any surfactant commonly included in cosmetic preparations. Among the above surfactants, partially crosslinked polyether-modified silicone, partially crosslinked polyglycerol-modified silicone, linear or branched polyoxyethylene-modified organopolysiloxane, linear or branched polyoxyethylene polyoxypropylene-modified organopolysiloxane, linear or branched polyoxyethylene/alkyl comodified organopolysiloxane, linear or branched polyoxyethylene polyoxypropylene/alkyl comodified organopolysiloxane, linear or branched polyglycerol-modified organopolysiloxane, and linear or branched polyglycerol/alkyl comodified organopolysiloxane are preferable.

Examples of the partially crosslinked polyether-modified silicone include (dimethicone/(PEG-10/15)) crosspolymer (INCI), (PEG-15/lauryl dimethicone) crosspolymer (INCI), (PEG-10/lauryl dimethicone) crosspolymer (INCI) and (PEG-15/lauryl polydimethylsiloxyethyl dimethicone) crosspolymer (INCI). Examples of the partially crosslinked polyglycerol-modified silicone include (dimethicone/polyglycerol-3) crosspolymer (INCI), (lauryl dimethicone/polyglycerol-3) crosspolymer (INCI) and (polyglycerol-3/lauryl polydimethylsiloxyethyl dimethicone) crosspolymer (INCI). In the case where partially crosslinked polyether-modified silicone or partially crosslinked polyglycerol-modified silicone is used, in the composition including the crosslinked organopolysiloxane and an oil that is liquid at room temperature, it is preferable that the crosslinked organopolysiloxane swell by incorporating an amount of the liquid oil which is equal to or more than the weight of the crosslinked organopolysiloxane.

Examples of the liquid oil that can be used include the component (B) and the liquid silicone oils, hydrocarbon oils, ester oils, natural animal and vegetable oils, semisynthetic oils and fluorocarbon oils described above in (1) Oils Other Than Component (B). Examples thereof include cyclopentasiloxane (INCI), dimethicone (INCI), mineral oil (INCI), isododecane (INCI), isohexadecane (INCI), triethylhexanoin (INCI), isotridecyl isononanoate (Labeling name (INCI: Isotridecyl Isononanoate)) and squalane (INCI).

Examples of commercial crosslinked organopolysiloxanes that swell as a result of incorporating a liquid oil include KSG-210, KSG-240, KSG-270, KSG-310, KSG-320, KSG-330, KSG-340, KSG-320Z, KSG-350Z, KSG-710, KSG-810, KSG-820, KSG-830, KSG-840, KSG-820Z and KSG-850Z produced by Shin-Etsu Chemical Co., Ltd.

Examples of surfactants that are not crosslinked organopolysiloxanes include PEG-11 methyl ether dimethicone (INCI), PEG/PPG-20/22 butyl ether dimethicone (INCI), PEG-3 dimethicone (INCI), PEG-10 dimethicone (INCI), PEG-9 polydimethylsiloxyethyl dimethicone (INCI), lauryl PEG-9 polydimethylsiloxyethyl dimethicone (INCI), cetyl PEG/PPG-10/1 dimethicone (INCI), polyglyceryl-3 disiloxane dimethicone (INCI), polyglyceryl-3 polydimethylsiloxyethyl dimethicone (INCI), lauryl polyglyceryl-3 polydimethylsiloxyethyl dimethicone (INCI) and bis-butyldimethicone polyglyceryl-3 (INCI). Examples of commercial products thereof include KF-6011, KF-6011P, KF-6012, KF-6015, KF-6017, KF-6043, KF-6028, KF-6038, KF-6048, KF-6100, KF-6104, KF-6105, KF-6106 and KF-6115 produced by Shin-Etsu Chemical Co., Ltd.

The amount of the surfactant added is preferably 0.1% to 20% by weight of the total amount of the cosmetic preparation. When the above amount is 0.1% by weight or more, the dispersion and emulsification effects can be achieved sufficiently. When the above amount is up to 20% by weight, a sticky feel is not sensed when the cosmetic preparation is used. The HLB of the surfactant is not limited and is preferably 2 to 14.5 in order to maintain the water resistance of the cosmetic preparation.

### (4) Powders

Examples of the powder include a color pigment, an inorganic powder, a metal powder, an organic powder, and an inorganic/organic composite powder, which are described specifically below.

### • Color Pigments

The color pigment is not limited and may be any color pigment commonly used for coloring cosmetic preparations. Examples thereof include red iron oxide (Labeling name (INCI: Iron Oxides)), yellow iron oxide (Labeling name (INCI: Iron Oxides)), white titanium oxide (Labeling name (INCI: Titanium Dioxide)), black iron oxide (Labeling name (INCI: Iron Oxides)), ultramarine blue (Labeling name (INCI: Ultramarines)), Prussian blue (Labeling name (INCI: Ferric Ferrocyanide, Ferric Ammonium Ferrocyanide)), manganese violet (Labeling name (INCI: Manganese Violet)), cobalt titanate (Labeling name (INCI: Cobalt Titanium Oxide)), chromium hydroxide (Labeling name (INCI: Chromium Hydroxide Green)), chromium oxide (Labeling name (INCI: Chromium Oxide Greens)), (Al/cobalt) oxide (Labeling name (INCI: Cobalt Aluminum Oxide)), cobalt titanate (Labeling name (INCI: Cobalt Titanium Oxide)), (titanium/titanium oxide) fired product (Labeling name (INCI: Titanium/Titanium Dioxide)), (Li/cobalt) titanate (Labeling name (INCI: Lithium Cobalt Titanate)), cobalt titanate (Labeling name (INCI: Cobalt Titanium Oxide)), (iron oxide/titanium oxide) sintered product (Labeling name), a composite doped with a hetero metal, such as iron oxide-doped titanium oxide (Labeling name (INCI: Iron Oxides, Titanium Dioxide)), inorganic brown pigments, such as titanium nitride (Labeling name (INCI: Titanium Nitride)), iron hydroxide (Labeling name (INCI: Iron Hydroxide)) and γ-iron oxide, inorganic yellow pigments, such as Chinese yellow and color pigments, such as lake of a tar-derived colorant and a lake of a natural colorant. Any of the above pigments may be used.

The shape of the pigment particles is not limited and may be any of spherical, approximately spherical, rod-like, spindle-like, petal-like, strip-like, indefinite and the like. The geometrical form of the pigment particles is not limited and may be any form that allows the pigment to color the cosmetic preparation.

### • Inorganic Powder

Examples of the inorganic powder include microparticles of the following substances: zirconium oxide (Labeling name (INCI: Zirconium Dioxide)), zinc oxide (Labeling name (INCI: Zinc Oxide)), cerium oxide (Labeling name (INCI: Cerium Oxide)), Mg oxide (Labeling name (INCI: Magnesium Oxide)), Ba sulfate (Labeling name (INCI: Barium Sulfate)), calcium sulfate (Labeling name (INCI: Calcium Sulfate)), Mg sulfate (Labeling name (INCI: Magnesium Sulfate)), Ca carbonate (Labeling name (INCI: Calcium Carbonate)), Mg carbonate (Labeling name (INCI: Magnesium Carbonate)), talc (INCI), mica (INCI), kaolin (INCI), synthetic fluorphlogopite (Labeling name (INCI: Synthetic Fluorphlogopite), synthetic ferrous golden mica (Labeling name), black mica (Labeling name (INCI: Biotite), K silicate (Labeling name (INCI: Potassium Silicate)), silica (INCI), Al silicate (Labeling name (INCI: Aluminum Silicate)), Mg silicate (Labeling name (INCI: Magnesium Silicate)), (Al/Mg) silicate (Labeling name (INCI: Magnesium Aluminum Silicate)), Ca silicate (Labeling name (INCI: Calcium Silicate)), (Al/Ca/Na) silicate (Labeling name (INCI: Aluminum Calcium Sodium Silicate)), (Li/Mg/Na) silicate (Labeling name (INCI: Lithium Magnesium Sodium Silicate)) (Na/Mg) silicate (Labeling name (INCI: Sodium Magnesium Silicate)), (Ca/Al) borosilicate (Labeling name (INCI: Calcium Aluminum Borosilicate)), (Ca/Na) borosilicate (Labeling name (INCI: Calcium Sodium Borosilicate)), hydroxyapatite (INCI), bentonite (INCI), montmorillonite (INCI), hectorite (INCI), zeolite (INCI), alumina (INCI), Al hydroxide (Labeling name (INCI: Aluminum Hydroxide)), boron nitride (Labeling name (INCI: Boron Nitride)) and glass (Labeling name (INCI: Glass)).

Examples of inorganic coloring pearl pigments include pearl agents, such as mica (INCI) coated with titanium oxide (Labeling name (INCI: Titanium Dioxide)) and synthetic fluorphlogopite (Labeling name (INCI: Synthetic Fluorphlogopite) coated with titanium oxide (Labeling name (INCI: Titanium Dioxide)); and pearl pigments, such as bismuth oxychloride (Labeling name (INCI: Bismuth Oxychloride)), bismuth oxychloride (Labeling name (INCI: Bismuth Oxychloride)) coated with titanium oxide (Labeling name (INCI: Titanium Dioxide)), talc (INCI) coated with titanium oxide (Labeling name (INCI: Titanium Dioxide)), fish scale flake (Labeling name) and colored mica coated with titanium oxide (Labeling name (INCI: Titanium Dioxide)). They may be, but not necessarily, subjected to a known surface treatment commonly used for cosmetics.

### • Metal Powders

Examples of the metal powder particles include metal microparticles of Al (Labeling name (INCI: Aluminum, Aluminum Powder)), copper (Labeling name (INCI: Copper Powder)), silver (Labeling name (INCI: Silver Powder)) and gold (Labeling name (INCI: Gold)).

### • Organic Powders

Examples of the organic powder include powders of silicone, polyamide, polyacrylic acid/acrylate ester, polyester, polyethylene (INCI), polypropylene (INCI), polystyrene (INCI), styrene/acrylic acid copolymer, divinylbenzene/styrene copolymer, polyurethane, a vinyl resin, a urea resin, a melamine resin, benzoguanamine, polymethyl benzoguanamine, tetrafluoroethylene, polymethyl methacrylate, cellulose (INCI), silk (INCI), nylon (Labeling name), a phenolic resin, an epoxy resin and polycarbonate.

In particular, examples of silicone powders include silicone resin particles; polymethyl silsesquioxane (INCI), a silicone rubber powder and a silicone resin-coated silicone rubber powder; and a (vinyl dimethicone/methicone silsesquioxane) crosspolymer (Labeling name (INCI: vinyl dimethicone/methicone silsesquioxane crosspolymer)), a (diphenyl dimethicone/vinyl diphenyl dimethicone/silsesquioxane) crosspolymer (Labeling name (INCI: diphenyl dimethicone/vinyl diphenyl dimethicone/silsesquioxane crosspolymer)), a polysilicone-1 crosspolymer (INCI) and polysilicone-22 (INCI).

Examples of commercial silicone powders include KMP-590, KMP-591, KMP-592, KMP-597, KMP-598, KSP-100, KSP-101, KSP-102, KSP-105, KSP-300, KSP-411, KSP-441, KM-9729 and KM-440 produced by Shin-Etsu Chemical Co., Ltd.

For example, metal soaps may also be used. Specific examples thereof include powders of zinc stearate (Labeling name (INCI: Zinc Stearate)), Al stearate (Labeling name (INCI: Aluminum Stearate)), Ca stearate (Labeling name (INCI: Calcium Stearate)), Mg stearate (Labeling name (INCI: Magnesium Stearate)), zinc myristate (Labeling name (INCI: Zinc Myristate)), Mg myristate (Labeling name (INCI: Magnesium Myristate)), (zinc/Na) cetyl phosphate (Labeling name (INCI: Sodium Zinc Cetyl Phosphate)) and K cetyl phosphate (Labeling name (INCI: Potassium Cetyl Phosphate)).

Furthermore, for example, organic colorants may also be used. Specific examples thereof include tar colorants, such as Red 3, Red 104(1) (Labeling name (INCI: Red 28, Red 28 Lake)), Red 106, Red 201 (Labeling name (INCI: Red 6)), Red 202 (Labeling name (INCI: Red 7)), Red 204, Red 205, Red 220 (Labeling name (INCI: Red 34)), Red 226 (Labeling name (INCI: Red 30)), Red 227 (Labeling name (INCI: Red 33, RED 33 Lake)), Red 228 (Labeling name (INCI: Red 36)), Red 230(1) (Labeling name (INCI: Red 22, Red 22 Lake)), Red 230(2) (Labeling name), Red 401 (Labeling name), Red 505 (Labeling name), Yellow 4 (Labeling name (INCI: Yellow 5)), Yellow 5 (Labeling name (INCI: Yellow 6, Yellow 6 Lake)), Yellow 202(1) (Labeling name (INCI: Yellow 8)), Yellow 203 (Labeling name (INCI: Yellow 10, Yellow 10 Lake)), Yellow 204 (Labeling name (INCI: Yellow 11)), Yellow 401, Blue 1 (Labeling name (INCI: Blue 1, Blue 1 Lake)), Blue 2, Blue 201, Blue 205 (Labeling name (INCI: Blue 4))Blue 404 (Labeling name), Green 3 (Labeling name (INCI: Green 3, Green 3 Lake)), Green 201 (Labeling name (INCI: Green 5)), Green 202 (Labeling name (INCI: Green 6)), Green 204 (Labeling name (INCI: Green 8)), Green 205 (Labeling name), Orange 201 (Labeling name (INCI: Orange 5)), Orange 203 (Labeling name (INCI: Pigment Orange 5)), Orange 204 (Labeling name), Orange 205 (Labeling name (INCI: Orange 4, Orange 4 Lake)), Orange 206 (Labeling name (INCI: Orange 10)) and Orange 207 (Labeling name (INCI: Orange 11)); and natural colorants, such as cochineal (INCI), laccaic acid (Labeling name (INCI: Laccaic Acid)), carthamus red (Labeling name (INCI: Carthamus Tinctorius (Safflower) Flower Extract)), lithospermum officinale root extract (Labeling name (INCI: Lithospermum Officinale Root Extract)), gardenia yellow (Labeling name) and gardenia blue (Labeling name (INCI: Hydrolyzed Gardenia Florida Extract)).

### • Inorganic/Organic Composite Powders

Examples of the inorganic/organic composite powder include a composite powder produced by coating the surfaces of inorganic powder particles with an organic powder by a method publicly known and commonly used in the related art.

The surfaces of the above powder particles may be treated optionally. The surface-treating agent is preferably a treatment agent capable of imparting hydrophobicity in consideration of the water resistance of the cosmetic preparation. Examples of the treatment agent capable of imparting hydrophobicity include, but are not limited to, a silicone treatment agent, a wax, a paraffin, an organic fluorine compound, such as perfluoroalkyl and phosphate, a surfactant, an amino acid, such as N-acylglutamic acid and metal soaps, such as aluminum stearate and magnesium myristate.

A silicone treatment agent is more preferable. Examples thereof include silanes or silylating agents, such as triethoxycaprylylsilane (INCI), dimethicone (INCI), methicone (INCI), hydrogen dimethicone (INCI), triethoxysilylethyl polydimethylsiloxyethyl dimethicone (INCI), triethoxysilylethyl polydimethylsiloxyethyl hexyl dimethicone (INCI) and an (acrylates/tridecyl acrylate/triethoxysilylpropyl methacrylate/dimethicone methacrylate) copolymer (Labeling name (INCI: Acrylates/Tridecyl Acrylate/Triethoxysilylpropyl Methacrylate/Dimethicone Methacrylate Copolymer).

Specific examples of the above silicone treatment agents include AES-3083, KF-99P, KF-9901, KF-9908, KF-9909, KP-574 and KP-541 produced by Shin-Etsu Chemical Co., Ltd. The above surface hydrophobizing agents may be used alone or in combination of two or more. Specific examples of surface-treated color pigments include KTP-09 series produced by Shin-Etsu Chemical Co., Ltd and, in particular, KTP-09W, KTP-09R, KTP-09Y and KTP-09B.

Among the above powders, an oil-absorbing powder is particularly preferable in consideration of reduction in stickiness and resistance to secondary adhesion. Examples of the oil-absorbing powder include a silicone rubber powder, a silicone resin-coated silicone rubber powder, a porous powder and fumed silica. Note that the expression "oil-absorbing" means that the capacity of absorbing dimethicone (6cs) is 1.0 mL/g or more, is preferably 1.2 mL/g or more, and is further preferably 1.5 mL/g or more. The content of the oil-absorbing powder added is preferably 0.1% to 10% by weight, is more preferably 1% to 8% by weight, and is further preferably 3% to 8% by weight. If the above content is more than 10% by weight, the spreadability of the cosmetic preparation may become degraded.

### (5) Composition Including Crosslinked Organopolysiloxane and Oil That Is Liquid at Room Temperature

In the composition including a crosslinked organopolysiloxane and an oil that is liquid at room temperature, it is preferable that the crosslinked organopolysiloxane swells by absorbing an amount of the liquid oil which is equal to or larger than the amount of the crosslinked organopolysiloxane. Examples of the liquid oil that can be used include the component (B) and the liquid silicone oils, hydrocarbon oils, ester oils, natural animal and vegetable oils, semisynthetic oils and fluorocarbon oils described above in (1) Oils, which are optional components. Examples thereof include cyclopentasiloxane (INCI), dimethicone (INCI), mineral oil (INCI), isododecane (INCI), isohexadecane (INCI), triethylhexanoin (INCI), isotridecyl isononanoate (Labeling name (INCI: Isotridecyl Isononanoate)) and squalane (INCI).

The component (5) is a compound that does not have a polyether or polyglycerol structure in the molecular structure, unlike the component (3) above. Specific examples thereof include a (dimethicone/vinyl dimethicone) crosspolymer (INCI), a (dimethicone/phenyl vinyl dimethicone) crosspolymer (Labeling name (INCI: Dimethicone/Phenyl Vinyl dimethicone crosspolymer)), a (vinyl dimethicone/lauryl dimethicone) crosspolymer (INCI) and a (lauryl polydimethylsiloxyethyl dimethicone/bis-vinyldimethicone) crosspolymer (INCI).

Examples of commercial products of the composition including a crosslinked organopolysiloxane and an oil that is liquid at room temperature include KSG-15, KSG-1510, KSG-16, KSG-1610, KSG-19, KSG-016F, KSG-18A, KSG-41A, KSG-42A, KSG-43, KSG-44, KSG-042Z, KSG-045Z and KSG-048Z produced by Shin-Etsu Chemical Co., Ltd.

### (6) Film-Forming Agent Other Than Component (A)

A film-forming agent other than the component (A) is used primarily to further maintain the durability of the advantageous effects of the cosmetic preparation. The film-forming agent is preferably, but not limited to, a silicone-based composition in order to impart water repellency. Specifically, trimethylsiloxysilicate, an acrylic-silicone film agent, silicone-modified norbornene, silicone-modified pullulan, silicone-modified polyvinyl alcohol and the like can be used.

Examples of the film-forming agent that is a silicone-based composition include trimethylsiloxysilicate (Labeling name (INCI: Trimethylsiloxysilicate), an (acrylates/dimethicone) copolymer (INCI), a (norbornene/tris(trimethylsiloxy)silylnorbornene) copolymer (INCI) and pullulan tri(trimethylsiloxy)silylpropyl carbamate (Labeling name (INCI: Trimethylsiloxysilylcarbamoyl Pullulan).

The film-forming agent may be added to the cosmetic preparation after having been dissolved in an oil that is liquid at room temperature. Examples of the liquid oil that can be used include the component (B) and the liquid silicone oils, hydrocarbon oils, ester oils, natural animal and vegetable oils, semisynthetic oils and fluorocarbon oils described above in (1) Oils, which are optional components. Examples thereof include cyclopentasiloxane (INCI), dimethicone (INCI), mineral oil (INCI), isododecane (INCI), isohexadecane (INCI), triethylhexanoin (INCI), isotridecyl isononanoate (Labeling name (INCI: Isotridecyl Isononanoate)), squalane (INCI) and butyl acetate (Labeling name (INCI: Butyl Acetate)).

Specific examples of commercial silicone film-forming agents include KF-7312J, KP-545, KP-549, KP-543, NBN-30-ID, TSPL-30-ID and TSPL-30-D5 produced by Shin-Etsu Chemical Co., Ltd.

### (7) Ultraviolet Absorbing/Scattering Agents

Examples of the ultraviolet absorbing/scattering agent include particles capable of absorbing and scattering ultraviolet radiation, such as microparticulate titanium oxide, microparticulate iron-containing titanium oxide, microparticulate zinc oxide, microparticulate cerium oxide and a composite thereof. A dispersion prepared by dispersing the particles capable of absorbing and scattering ultraviolet radiation in an oil may also be used.

Examples of the liquid oil that can be used include the component (B) and the liquid silicone oils, hydrocarbon oils, ester oils, natural animal and vegetable oils, semisynthetic oils and fluorocarbon oils described above in (1) Oils Other Than Component (B). Examples thereof include cyclopentasiloxane (INCI), dimethicone (INCI), mineral oil (INCI), isododecane (INCI), isohexadecane (INCI), triethylhexanoin (INCI), isotridecyl isononanoate (Labeling name (INCI: Isotridecyl Isononanoate)) and squalane (INCI).

Specific examples of the dispersion prepared by dispersing the particles capable of absorbing and scattering ultraviolet radiation in an oil include SPD series (product name) produced by Shin-Etsu Chemical Co., Ltd. and, in particular, SPD-T5, SPD-T5L, SPD-Z5, SPD-Z5L, SPD-T6, SPD-Z6 and SPD-T7.

### (8) Other Additives

Examples of the other additive include an oil-soluble gelling agent, a preservative, a bactericide, an antiperspirant, a fragrance, a salt, an antioxidant, a pH-controlling agent, a chelating agent, an algefacient, an anti-inflammatory agent, skin beautifying components (e.g., a whitening agent, a cell activator, a skin roughness improver, a circulation promoting ingredient, a skin astringent and an antiseborrheic agent), a vitamin, an amino acid, a nucleic acid, a hormone and an inclusion compound.

### • Oil-Soluble Gelling Agents

Examples of the oil-soluble gelling agent include metal soaps, such as aluminum stearate, magnesium stearate and zinc myristate; amino acid derivatives, such as lauroyl glutamic acid (Labeling name (INCI: Lauroyl Glutamic Acid)) and α,γ-di-n-butylamine; dextrin fatty acid esters, such as dextrin palmitate (Labeling name (INCI: Dextrin Palmitate)), dextrin isostearate (Labeling name (INCI: Dextrin Isostearate)), dextrin myristate (Labeling name (INCI: Dextrin Myristate)), stearoyl inulin (Labeling name (INCI: Stearoyl Inulin)) and dextrin (palmitate/ethylhexanoate) (Labeling name (INCI: Dextrin Palmitate/Ethylhexanoate)); sucrose fatty acid esters, such as sucrose palmitate and sucrose stearate; fructo-oligosaccharide fatty acid esters, such as fructo-oligosaccharide stearate and fructo-oligosaccharide 2-ethylhexanoate; benzylidene derivatives of sorbitol, such as monobenzylidene sorbitol and dibenzylidene sorbitol; organically modified clay minerals, such as disteardimonium hectorite (INCI), stearalkonium hectorite (INCI) and hectorite; and stearalkonium bentonite (INCI).

### • Preservatives and Bactericides

Examples of the preservatives and bactericides include alkyl esters of p-hydroxybenzoic acid, benzoic acid, sodium benzoate, sorbic acid, potassium sorbate, phenoxyethanol, imidazolidinyl urea, salicylic acid, isopropyl methyl phenol, carbolic acid, p-chloro-m-cresol, hexachlorophene, benzalkonium chloride, chlorhexidine chloride, trichlorocarbanilide, iodopropynyl butylcarbamate, polylysine, photosensitizers, silver and plant extracts.

### • Fragrances

Examples of the fragrance include natural fragrances and synthetic fragrances. Examples of the natural fragrances include botanical fragrances extracted from petals, leaves, wood, pericarp, or the like; and animal fragrances, such as musk and civet. Examples of the synthetic fragrances include hydrocarbons, such as monoterpene; alcohols, such as an aliphatic alcohol and an aromatic alcohol; aldehydes, such as a terpene aldehyde and an aromatic aldehyde; ketones, such as a alicyclic ketone; esters, such as a terpene ester; and lactones, phenols, oxides, nitrogen-containing compounds and acetals.

### • Salts

Examples of the salt include an inorganic acid salt, an organic acid salt, an amine salt, and an amino acid salt. Examples of the inorganic acid salt include a sodium salt, a potassium salt, a magnesium salt, a calcium salt, an aluminum salt, a zirconium salt and a zinc salt of an inorganic acid, such as hydrochloric acid, sulfuric acid, carbonic acid, or nitric acid. Examples of the organic acid salt include salts of organic acids, such as acetic acid, dehydroacetic acid, citric acid, malic acid, succinic acid, ascorbic acid and stearic acid. Examples of the amine salt and amino acid salt include salts of amines, such as triethanolamine; and salts of amino acids, such as glutamic acid. Examples of other salts that can be used include salts of hyaluronic acid, chondroitin sulfate and the like and acid-alkali neutralization salts used in pharmaceutical formulation.

### • Antioxidants

Examples of the antioxidant include, but are not limited to, carotinoid, ascorbic acid and salts thereof, ascorbyl stearate, tocophenol, tocophenol acetate, tocopherol, p-t-butylphenol, butyl hydroxyanisole, dibutyl hydroxytoluene, phytic acid, ferulic acid, thiotaurine, hypotaurine, a sulfite salt, erythorbic acid and salts thereof, chlorogenic acid, epicatechin, epigallocatechin, epigallocatechin gallate, apigenin, kaempferol, myricetin and quercetin.

### • pH-Controlling Agents

Examples of the pH-controlling agent include lactic acid, citric acid, glycolic acid, succinic acid, tartaric acid, dl-malic acid, potassium carbonate, sodium hydrogen carbonate and ammonium hydrogen carbonate.

### • Chelating Agents

Examples of the chelating agent include alanine, sodium edetate, poly(sodium phosphate), sodium metaphosphate and phosphoric acid.

### • Algefacients

Examples of the algefacient include L-menthol, camphor and menthyl lactate.

### • Anti-Inflammatory Agents

Examples of the anti-inflammatory agent include allantoin, glycyrrhizin acid and salts thereof, glycyrrhetinic acid, stearyl glycyrrhetinate, tranexamic acid and azulene.

### • Skin Beautifying Components

Examples of the skin beautifying components include whitening agents, such as a placental extract, arbutin, glutathione and a saxifraga sarmentosa extract; cell activators, such as royal jelly, a photosensitizer, a cholesterol derivative and a deproteinized calf blood extract; a skin roughness improver; circulation promoting ingredients, such as nonanoic acid vanillylamide, nicotinic acid benzyl ester, nicotinic acid β-butoxyethyl ester, capsaicin, zingerone, cantharides tincture, ichthammol, caffeine, tannic acid, α-borneol, tocopherol nicotinate, inositol hexanicotinate, cyclandelate, cinnarizine, tolazoline, acetylcholine, verapamil, cepharanthin and γ-oryzanol; a skin astringent; and antiseborrheic agents, such as sulfur and thianthol.

### • Vitamins

Examples of the vitamins include vitamin A, such as a vitamin A oil, retinol, retinol acetate, or retinol palmitate; vitamin B's (vitamin B2's, such as riboflavin, riboflavin butyrate, or flavin adenine nucleotide, vitamin B6, such as pyridoxine hydrochloride, pyridoxine dioctanoate, or pyridoxine tripalmitate, vitamin B12 and derivatives thereof and vitamin B15 and derivatives thereof); vitamin C, such as L-ascorbic acid, L-ascorbic acid dipalmitic acid ester, L-ascorbic acid-2-sodium sulfate, or dipotassium L-ascorbic acid phosphoric acid diester; vitamin D, such as ergocalciferol or cholecalciferol; vitamin E, such as α-tocopherol, β-tocopherol, γ-tocopherol, dl-α-tocopherol acetate, dl-α-tocopherol nicotinate, or dl-α-tocopherol succinate; nicotinic acids, such as nicotinic acid, benzyl nicotinate and nicotinamide; vitamin H; vitamin P; pantothenic acids, such as calcium pantothenate, D-pantothenyl alcohol, pantothenyl ethyl ether and pantothenyl ethyl ether acetate; and biotin.

### • Amino Acids

Examples of the amino acid include glycine, valine, leucine, isoleucine, serine, threonine, phenylalanine, arginine, lysine, aspartic acid, glutamic acid, cystine, cysteine, methionine and tryptophan.

### • Nucleic Acids

Examples of the nucleic acid include deoxyribonucleic acid.

### • Hormones

Examples of the hormone include estradiol and ethenyl estradiol.

### • Inclusion Compounds

Examples of the inclusion compound include cyclodextrin.

The above-described cosmetic preparation may be in the form of an emulsion or a non-aqueous system. When it is required to impart a fresh feel on use, an emulsified form is selected. The emulsified form may be any of the following: an oil-in-water (O/W) emulsion, water-in-oil (W/O) emulsion, oil-in-water-in-oil (O/W/O) emulsion or water-in-oil-in-water (W/O/W) emulsion. When it is required to obtain an oily feel or water resistance, a non-aqueous composition or a powder composition can be selected. In either of these cases, a good cosmetic preparation can be obtained. In the present invention, the term "non-aqueous composition" refers to a composition which does not contain water intentionally. Among these, a non-aqueous composition, which may have particularly high oil resistance, is preferable.

The crosslinked organosilicon resin according to the present invention can be added to a cosmetic preparation by any method. For example, the crosslinked organosilicon resin may be dissolved in the component (B) and used in the form of a solution. The crosslinked organosilicon resin may be dried up with a spray drier or the like such that solid component can be brought into a state in which it can be readily redissolved. In another case, the crosslinked organosilicon resin may be formed into an O/W emulsion and added to a cosmetic preparation.

The cosmetic preparation according to the present invention may be used in a variety of products, such as beauty essences, milky lotions, creams, hair care products, foundations, makeup bases, sunscreens, concealers, cheek color, lipsticks, gloss, balms, mascaras, eye shadows, eye liners, body makeup, deodorants and nail cosmetics. Among these, makeup cosmetic preparations, such as foundations, makeup bases, lipsticks, mascaras and eye liners, and cosmetic preparations having a sunscreening effect are particularly preferable. The form of the cosmetic preparation according to the present invention may be selected from among various forms, including liquids, creams, solids, pastes, gels, mousses, sprays, clays, powders and sticks.

### EXAMPLES

The present invention is described in further details in accordance with Examples and Comparative Examples below. Note that the present invention is not limited by Examples below. The symbol "%" used for describing composition refers to "% by weight" unless otherwise specified. The content of a component is the content of the product used. The alkenyl group-containing organosilicon resins used as raw materials below are the one synthesized in accordance with the production method known in the related art. In the present invention, crosslinked organosilicon resins and cosmetic preparations are described as Examples. In Examples and Comparative Examples below, the reaction ratio of alkenyl groups is calculated by ¹H-NMR spectrum analysis on the basis of the amount of alkenyl groups that remain subsequent to the reaction.

### [Example 1]

### Method for Producing 30% Solution of

### Crosslinked Organosilicon Resin/Decamethylcyclopentasiloxane

Into a reaction container, 1,000 g of a 50% decamethylcyclopentasiloxane solution of a powdery alkenyl group-containing organosilicon resin (weight-average molecular weight: 7,430, vinyl value: 0.229 mmol/g) represented by average compositional formula (E1) below, 700 g of decamethylcyclopentasiloxane, 126.9 g of organopolysiloxane having two terminal hydrosilyl groups (amount of hydrogen gas generated: 20.3 mL/g, hydrosilyl group/vinyl group: 1.0) which is represented by formula (E2) below, and 0.6 g of a 2-propanol solution containing 0.5% chloroplatinic acid were charged. The resulting mixture was heated at 120°C for 8 hours in order to cause a reaction. Subsequently, heating was performed under reduced pressure in order to remove the solvent by distillation. After the concentration had been adjusted to 30% by the addition of decamethylcyclopentasiloxane, filtration was performed. Hereby, a decamethylcyclopentasiloxane solution of a crosslinked organosilicon resin was prepared.

The decamethylcyclopentasiloxane solution of the crosslinked organosilicon resin was then heated to 120°C to 130°C under reduced pressure in order to remove decamethylcyclopentasiloxane. The product was a solid powder (weight-average molecular weight: 221,000). The reaction ratio of alkenyl groups was 92%. The amount of the hydrogen gas generated from the residual hydrosilyl groups was 0.8 mL/g.

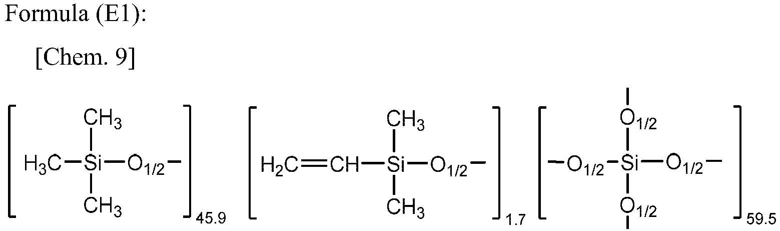

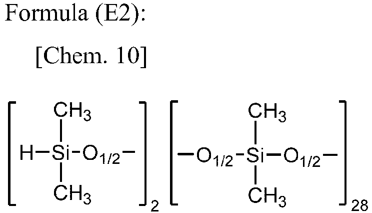

### [Example 2]

### Method for Producing 30% Solution of

### Crosslinked Organosilicon Resin/Decamethylcyclopentasiloxane

Into a reaction container, 1,000 g of a 50% decamethylcyclopentasiloxane solution of a powdery alkenyl group-containing organosilicon resin (weight-average molecular weight: 8,050, vinyl value: 0.224 mmol/g) represented by average compositional formula (E3) below, 700 g of decamethylcyclopentasiloxane, 53.8 g of organopolysiloxane having two terminal hydrosilyl groups (amount of hydrogen gas generated: 51.3 mL/g, hydrosilyl group/vinyl group: 1.1) which is represented by formula (E4) below, and 0.6 g of a 2-propanol solution containing 0.5% chloroplatinic acid were charged. The resulting mixture was heated at 110°C for 5 hours in order to cause a reaction. Subsequently, heating was performed under reduced pressure in order to remove the solvent by distillation. After the concentration had been adjusted to 30% by the addition of decamethylcyclopentasiloxane, filtration was performed. Hereby, a decamethylcyclopentasiloxane solution of a crosslinked organosilicon resin was prepared.

The decamethylcyclopentasiloxane solution of the crosslinked organosilicon resin was then heated to 120°C to 130°C under reduced pressure in order to remove decamethylcyclopentasiloxane. The product was a solid powder (weight-average molecular weight: 154,000). The reaction ratio of alkenyl groups was 93%. The amount of the hydrogen gas generated from the residual hydrosilyl groups was 1.0 mL/g.

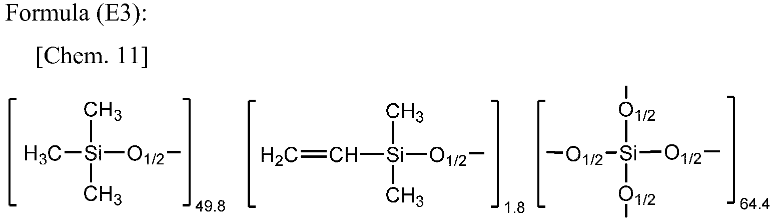

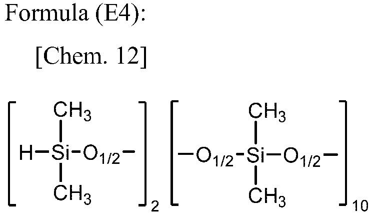

### [Example 3]

### Method for Producing 30% Solution of

### Crosslinked Organosilicon Resin/Decamethylcyclopentasiloxane

Into a reaction container, 1,000 g of a 50% decamethylcyclopentasiloxane solution of a powdery alkenyl group-containing organosilicon resin (weight-average molecular weight: 11,450, vinyl value: 0.271 mmol/g) represented by average compositional formula (E5) below, 700 g of decamethylcyclopentasiloxane, 109.0 g of organopolysiloxane having two terminal hydrosilyl groups (amount of hydrogen gas generated: 30.6 mL/g, hydrosilyl group/vinyl group: 1.1) which is represented by formula (E6) below, and 0.6 g of a 2-propanol solution containing 0.5% chloroplatinic acid were charged. The resulting mixture was heated at 90°C for 8 hours in order to cause a reaction. Subsequently, heating was performed under reduced pressure in order to remove the solvent by distillation. After the concentration had been adjusted to 30% by the addition of decamethylcyclopentasiloxane, filtration was performed. Hereby, a decamethylcyclopentasiloxane solution of a crosslinked organosilicon resin was prepared.

The decamethylcyclopentasiloxane solution of the crosslinked organosilicon resin was then heated to 120°C to 130°C under reduced pressure in order to remove decamethylcyclopentasiloxane. The product was a solid powder (weight-average molecular weight: 213,000). The reaction ratio of alkenyl groups was 93%. The amount of the hydrogen gas generated from the residual hydrosilyl groups was 0.8 mL/g.

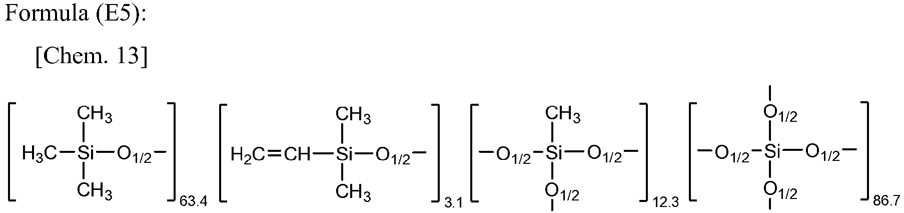

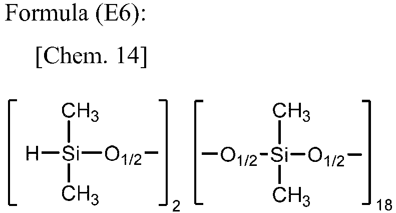

### [Example 4]

### Method for Producing 30% Solution of

### Crosslinked Organosilicon Resin/Decamethylcyclopentasiloxane

Into a reaction container, 1,000 g of a 50% decamethylcyclopentasiloxane solution of a powdery alkenyl group-containing organosilicon resin (weight-average molecular weight: 4,180, vinyl value: 0.287 mmol/g) represented by average compositional formula (E7) below, 700 g of decamethylcyclopentasiloxane, 142.4 g of organopolysiloxane having two terminal hydrosilyl groups (amount of hydrogen gas generated: 22.6 mL/g, hydrosilyl group/vinyl group: 1.0) which is represented by formula (E8) below, and 0.6 g of a 2-propanol solution containing 0.5% chloroplatinic acid were charged. The resulting mixture was heated at 110°C for 3 hours in order to cause a reaction. Subsequently, heating was performed under reduced pressure in order to remove the solvent by distillation. After the concentration had been adjusted to 30% by the addition of decamethylcyclopentasiloxane, filtration was performed. Hereby, a decamethylcyclopentasiloxane solution of a crosslinked organosilicon resin was prepared.

The decamethylcyclopentasiloxane solution of the crosslinked organosilicon resin was then heated to 120°C to 130°C under reduced pressure in order to remove decamethylcyclopentasiloxane. The product was a solid powder (weight-average molecular weight: 58,600). The reaction ratio of alkenyl groups was 94%. The amount of the hydrogen gas generated from the residual hydrosilyl groups was 0.8 mL/g.

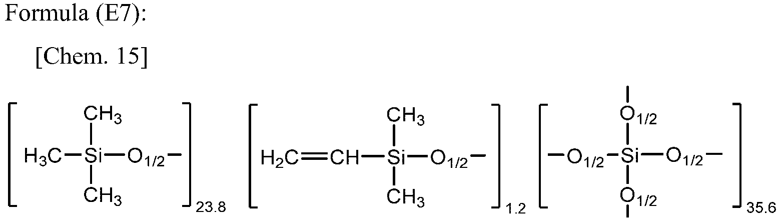

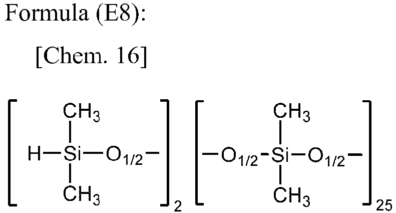

### [Example 5]

### Method for Producing 30% Solution of

### Crosslinked Organosilicon Resin/Decamethylcyclopentasiloxane

Into a reaction container, 1,000 g of a 50% decamethylcyclopentasiloxane solution of a powdery alkenyl group-containing organosilicon resin (weight-average molecular weight: 6,910, vinyl value: 0.333 mmol/g) represented by average compositional formula (E9) below, 700 g of decamethylcyclopentasiloxane, 36.3 g of organopolysiloxane having two terminal hydrosilyl groups (amount of hydrogen gas generated: 51.3 mL/g, hydrosilyl group/vinyl group: 0.5) which is represented by formula (E10) below, 92.1 g of organopolysiloxane having two terminal hydrosilyl groups (amount of hydrogen gas generated: 20.3 mL/g, hydrosilyl group/vinyl group: 0.5) which is represented by formula (E11) below, and 0.6 g of a 2-propanol solution containing 0.5% chloroplatinic acid were charged. The resulting mixture was heated at 120°C for 8 hours in order to cause a reaction. Subsequently, heating was performed under reduced pressure in order to remove the solvent by distillation. After the concentration had been adjusted to 30% by the addition of decamethylcyclopentasiloxane, filtration was performed. Hereby, a decamethylcyclopentasiloxane solution of a crosslinked organosilicon resin was prepared.

The decamethylcyclopentasiloxane solution of the crosslinked organosilicon resin was then heated to 120 to 130°C under reduced pressure in order to remove decamethylcyclopentasiloxane. The product was a solid powder (weight-average molecular weight: 184,000). The reaction ratio of alkenyl groups was 93%. The amount of the hydrogen gas generated from the residual hydrosilyl groups was 0.9 mL/g.

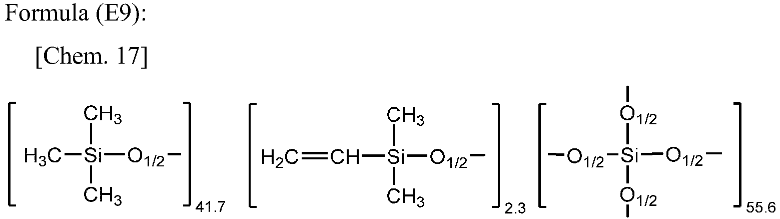

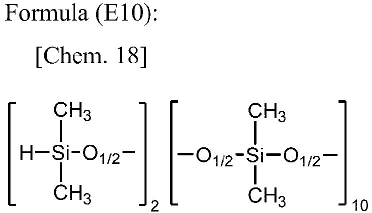

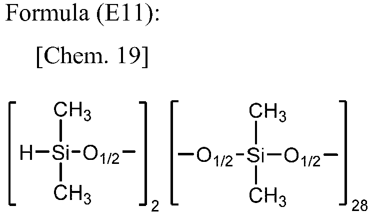

### [Example 6]

### Method for Producing 30% Solution of

### Crosslinked Organosilicon Resin/Decamethylcyclopentasiloxane

Into a reaction container, 1,000 g of a 50% decamethylcyclopentasiloxane solution of a powdery alkenyl group-containing organosilicon resin (weight-average molecular weight: 7,120, vinyl value: 0.197 mmol/g) represented by average compositional formula (E12) below, 700 g of decamethylcyclopentasiloxane, 47.2 g of organopolysiloxane having two terminal hydrosilyl groups (amount of hydrogen gas generated: 51.3 mL/g, hydrosilyl group/vinyl group: 1.1) which is represented by formula (E13) below, and 0.6 g of a 2-propanol solution containing 0.5% chloroplatinic acid were charged. The resulting mixture was heated at 100°C for 6 hours in order to cause a reaction. Subsequently, heating was performed under reduced pressure in order to remove the solvent by distillation. After the concentration had been adjusted to 30% by the addition of decamethylcyclopentasiloxane, filtration was performed. Hereby, a decamethylcyclopentasiloxane solution of a crosslinked organosilicon resin was prepared.

The decamethylcyclopentasiloxane solution of the crosslinked organosilicon resin was then heated to 120°C to 130°C under reduced pressure in order to remove decamethylcyclopentasiloxane. The product was a solid powder (weight-average molecular weight: 56,000). The reaction ratio of alkenyl groups was 94%. The amount of the hydrogen gas generated from the residual hydrosilyl groups was 0.6 mL/g.

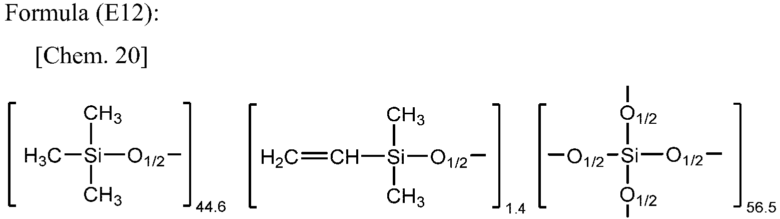

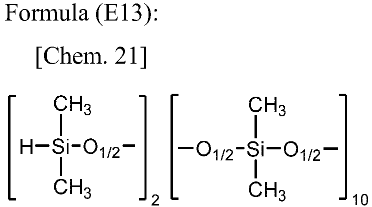

### [Example 7]

### Method for Producing 30% Solution of

### Crosslinked Organosilicon Resin/Decamethylcyclopentasiloxane

Into a reaction container, 1,000 g of a 50% decamethylcyclopentasiloxane solution of a powdery alkenyl group-containing organosilicon resin (weight-average molecular weight: 7,310, vinyl value: 0.274 mmol/g) represented by average compositional formula (E14) below, 700 g of decamethylcyclopentasiloxane, 135.6 g of organopolysiloxane having two terminal hydrosilyl groups (amount of hydrogen gas generated: 22.6 mL/g, hydrosilyl group/vinyl group: 1.0) which is represented by formula (E15) below, and 0.6 g of a 2-propanol solution containing 0.5% chloroplatinic acid were charged. The resulting mixture was heated at 80°C for 5 hours in order to cause a reaction. Subsequently, heating was performed under reduced pressure in order to remove the solvent by distillation. After the concentration had been adjusted to 30% by the addition of decamethylcyclopentasiloxane, filtration was performed. Hereby, a decamethylcyclopentasiloxane solution of a crosslinked organosilicon resin was prepared.

The decamethylcyclopentasiloxane solution of the crosslinked organosilicon resin was then heated to 120°C to 130°C under reduced pressure in order to remove decamethylcyclopentasiloxane. The product was a solid powder (weight-average molecular weight: 62,800). The reaction ratio of alkenyl groups was 92%. The amount of the hydrogen gas generated from the residual hydrosilyl groups was 0.5 mL/g.

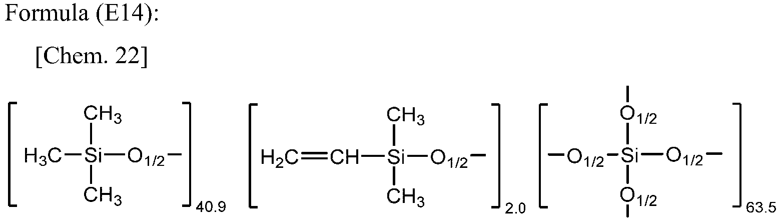

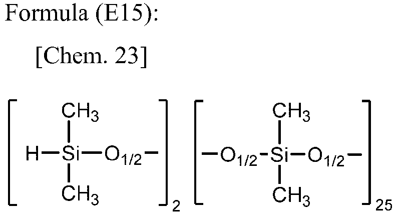

### [Example 8]

### Method for Producing 30% Solution of

### Crosslinked Organosilicon Resin/Decamethylcyclopentasiloxane

Into a reaction container, 1,000 g of a 50% decamethylcyclopentasiloxane solution of a powdery alkenyl group-containing organosilicon resin (weight-average molecular weight: 14,860, vinyl value: 0.323 mmol/g) represented by average compositional formula (E16) below, 700 g of decamethylcyclopentasiloxane, 160.0 g of organopolysiloxane having two terminal hydrosilyl groups (amount of hydrogen gas generated: 22.6 mL/g, hydrosilyl group/vinyl group: 1.0) which is represented by formula (E17) below, and 0.6 g of a 2-propanol solution containing 0.5% chloroplatinic acid were charged. The resulting mixture was heated at 110°C for 3 hours in order to cause a reaction. Subsequently, heating was performed under reduced pressure in order to remove the solvent by distillation. After the concentration had been adjusted to 30% by the addition of decamethylcyclopentasiloxane, filtration was performed. Hereby, a decamethylcyclopentasiloxane solution of a crosslinked organosilicon resin was prepared.

The decamethylcyclopentasiloxane solution of the crosslinked organosilicon resin was then heated to 120°C to 130°C under reduced pressure in order to remove decamethylcyclopentasiloxane. The product was a solid powder (weight-average molecular weight: 168,500). The reaction ratio of alkenyl groups was 92%. The amount of the hydrogen gas generated from the residual hydrosilyl groups was 0.5 mL/g.

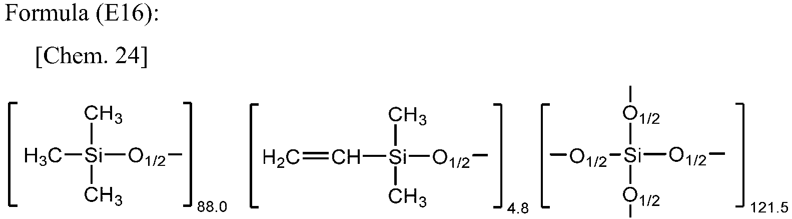

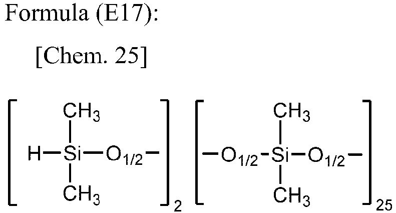

### [Example 9]

### Method for Producing 30% Solution of

### Crosslinked Organosilicon Resin/Decamethylcyclopentasiloxane

Into a reaction container, 1,000 g of a 50% decamethylcyclopentasiloxane solution of a powdery alkenyl group-containing organosilicon resin (weight-average molecular weight: 6,880, vinyl value: 0.276 mmol/g) represented by average compositional formula (E18) below, 700 g of decamethylcyclopentasiloxane, 10.2 g of tetramethyl disiloxane (amount of hydrogen gas generated: 334.3 mL/g, hydrosilyl group/vinyl group: 1.1) which is represented by formula (E19) below, and 0.6 g of a 2-propanol solution containing 0.5% chloroplatinic acid were charged. The resulting mixture was heated at 120°C for 5 hours in order to cause a reaction. Subsequently, heating was performed under reduced pressure in order to remove the solvent by distillation. After the concentration had been adjusted to 30% by the addition of decamethylcyclopentasiloxane, filtration was performed. Hereby, a decamethylcyclopentasiloxane solution of a crosslinked organosilicon resin was prepared.

The decamethylcyclopentasiloxane solution of the crosslinked organosilicon resin was then heated to 120°C to 130°C under reduced pressure **in** order to remove decamethylcyclopentasiloxane. The product was a solid powder (weight-average molecular weight: 112,000). The reaction ratio of alkenyl groups was 91%. The amount of the hydrogen gas generated from the residual hydrosilyl groups was 0.9 mL/g. 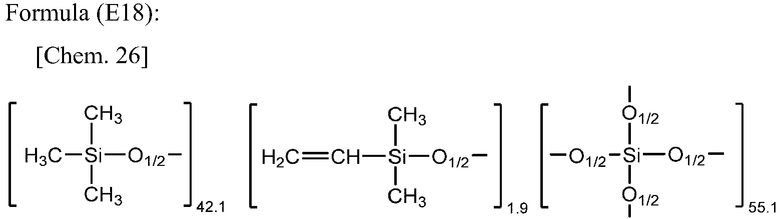 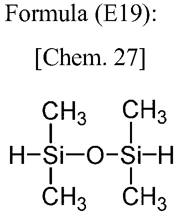 (in formula (2), e = 2, and f = g = h = 0.)

### [Example 10]

### Method for Producing 30% Solution of

### Crosslinked Organosilicon Resin/Decamethylcyclopentasiloxane

Into a reaction container, 1,000 g of a 50% decamethylcyclopentasiloxane solution of a powdery alkenyl group-containing organosilicon resin (weight-average molecular weight: 10,100, vinyl value: 0.178 mmol/g) represented by average compositional formula (E20) below, 700 g of decamethylcyclopentasiloxane, 42.8 g of organopolysiloxane having two terminal hydrosilyl groups (amount of hydrogen gas generated: 51.3 mL/g, hydrosilyl group/vinyl group: 1.1) which is represented by formula (E21) below, and 0.6 g of a 2-propanol solution containing 0.5% chloroplatinic acid were charged. The resulting mixture was heated at 90°C for 10 hours in order to cause a reaction. Subsequently, heating was performed under reduced pressure in order to remove the solvent by distillation. After the concentration had been adjusted to 30% by the addition of decamethylcyclopentasiloxane, filtration was performed. Hereby, a decamethylcyclopentasiloxane solution of a crosslinked organosilicon resin was prepared.

The decamethylcyclopentasiloxane solution of the crosslinked organosilicon resin was then heated to 120°C to 130°C under reduced pressure in order to remove decamethylcyclopentasiloxane. The product was a solid powder (weight-average molecular weight: 389,000). The reaction ratio of alkenyl groups was 92%. The amount of the hydrogen gas generated from the residual hydrosilyl groups was 0.7 mL/g. 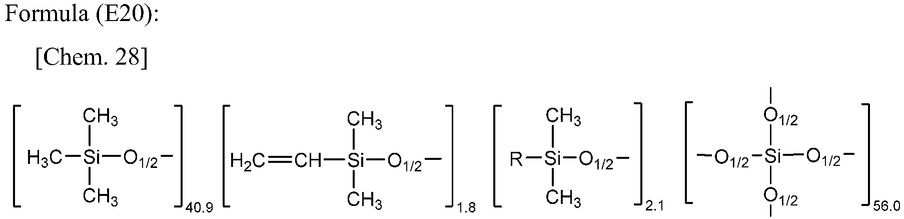 R: 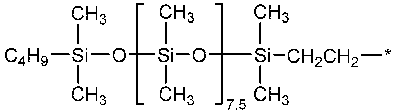 (where * represents a free radical bonded to a silicon atom.)

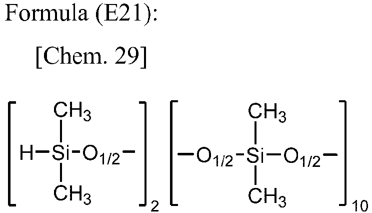

### [Example 11]

### Method for Producing 30% Solution of

### Crosslinked Organosilicon Resin/Decamethylcyclopentasiloxane

Into a reaction container, 1,000 g of a 50% decamethylcyclopentasiloxane solution of a powdery alkenyl group-containing organosilicon resin (weight-average molecular weight: 6,700, vinyl value: 0.388 mmol/g) represented by average compositional formula (E22) below, 700 g of decamethylcyclopentasiloxane, 109.2 g of organopolysiloxane having a side-chain hydrosilyl group (amount of hydrogen gas generated: 39.8 mL/g, hydrosilyl group/vinyl group: 1.0) which is represented by formula (E23) below, and 0.6 g of a 2-propanol solution containing 0.5% chloroplatinic acid were charged. The resulting mixture was heated at 120°C for 5 hours in order to cause a reaction. Subsequently, heating was performed under reduced pressure in order to remove the solvent by distillation. After the concentration had been adjusted to 30% by the addition of decamethylcyclopentasiloxane, filtration was performed. Hereby, a decamethylcyclopentasiloxane solution of a crosslinked organosilicon resin was prepared.

The decamethylcyclopentasiloxane solution of the crosslinked organosilicon resin was then heated to 120°C to 130°C under reduced pressure in order to remove decamethylcyclopentasiloxane. The product was a solid powder (weight-average molecular weight: 105,000). The reaction ratio of alkenyl groups was 93%. The amount of the hydrogen gas generated from the residual hydrosilyl groups was 0.6 mL/g.

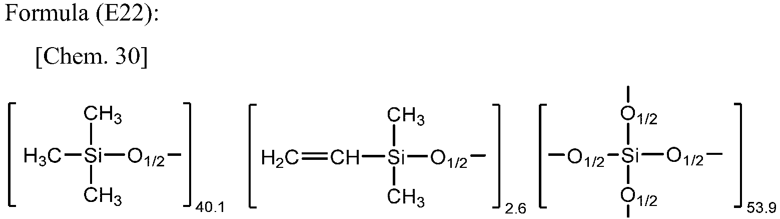

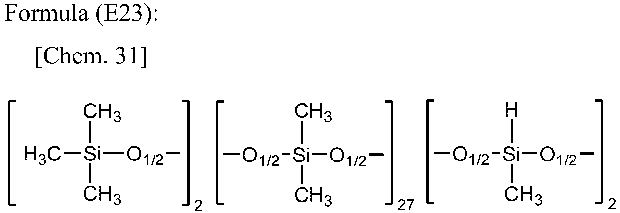

### [Example 12]

### Method for Producing 30% Solution of

### Crosslinked Organosilicon Resin/Decamethylcyclopentasiloxane

Into a reaction container, 1,000 g of a 50% decamethylcyclopentasiloxane solution of a powdery alkenyl group-containing organosilicon resin (weight-average molecular weight: 8,730, vinyl value: 0.275 mmol/g) represented by average compositional formula (E24) below, 700 g of decamethylcyclopentasiloxane, 39.7 g of organopolysiloxane having two terminal hydrosilyl groups (amount of hydrogen gas generated: 77.5 mL/g, hydrosilyl group/vinyl group: 1.0) which is represented by formula (E25) below, and 0.6 g of a 2-propanol solution containing 0.5% chloroplatinic acid were charged. The resulting mixture was heated at 120°C for 3 hours in order to cause a reaction. Subsequently, heating was performed under reduced pressure in order to remove the solvent by distillation. After the concentration had been adjusted to 30% by the addition of decamethylcyclopentasiloxane, filtration was performed. Hereby, a decamethylcyclopentasiloxane solution of a crosslinked organosilicon resin was prepared.

The decamethylcyclopentasiloxane solution of the crosslinked organosilicon resin was then heated to 120°C to 130°C under reduced pressure in order to remove decamethylcyclopentasiloxane. The product was a solid powder (weight-average molecular weight: 84,200). The reaction ratio of alkenyl groups was 95%. The amount of the hydrogen gas generated from the residual hydrosilyl groups was 1.0 mL/g.

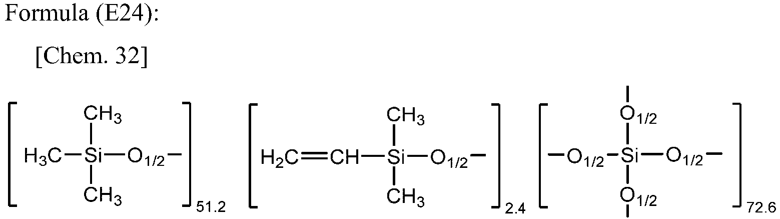

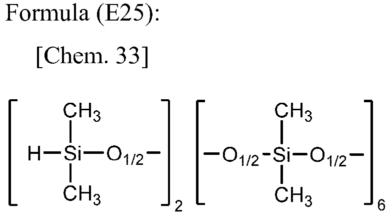

### [Example 13]

### Method for Producing 30% Solution of

### Crosslinked Organosilicon Resin/Decamethylcyclopentasiloxane

Into a reaction container, 1,000 g of a 50% decamethylcyclopentasiloxane solution of a powdery alkenyl group-containing organosilicon resin (weight-average molecular weight: 16,180, vinyl value: 0.266 mmol/g) represented by average compositional formula (E26) below, 700 g of decamethylcyclopentasiloxane, 63.9 g of organopolysiloxane having two terminal hydrosilyl groups (amount of hydrogen gas generated: 51.3 mL/g, hydrosilyl group/vinyl group: 1.1) which is represented by formula (E27) below, and 0.6 g of a 2-propanol solution containing 0.5% chloroplatinic acid were charged. The resulting mixture was heated at 100°C for 6 hours in order to cause a reaction. Subsequently, heating was performed under reduced pressure in order to remove the solvent by distillation. After the concentration had been adjusted to 30% by the addition of decamethylcyclopentasiloxane, filtration was performed. Hereby, a decamethylcyclopentasiloxane solution of a crosslinked organosilicon resin was prepared.

The decamethylcyclopentasiloxane solution of the crosslinked organosilicon resin was then heated to 120°C to 130°C under reduced pressure in order to remove decamethylcyclopentasiloxane. The product was a solid powder (weight-average molecular weight: 208,000). The reaction ratio of alkenyl groups was 92%. The amount of the hydrogen gas generated from the residual hydrosilyl groups was 0.8 mL/g.

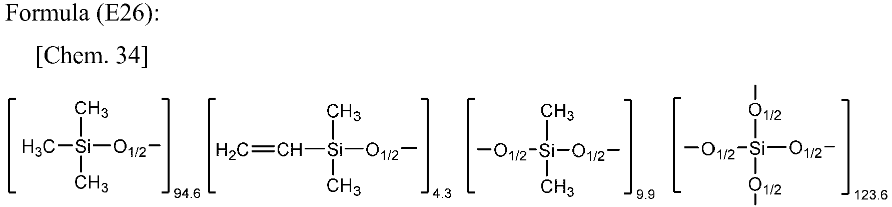

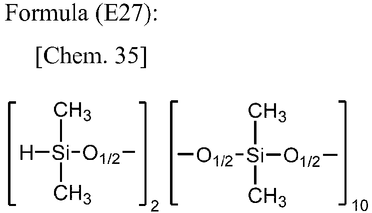

### [Example 14]

### Method for Producing 30% Solution of

### Crosslinked Organosilicon Resin/Decamethylcyclopentasiloxane

Into a reaction container, 1,000 g of a 50% decamethylcyclopentasiloxane solution of a powdery alkenyl group-containing organosilicon resin (weight-average molecular weight: 3,100, vinyl value: 0.419 mmol/g) represented by average compositional formula (E28) below, 700 g of decamethylcyclopentasiloxane, 100.7 g of organopolysiloxane having two terminal hydrosilyl groups (amount of hydrogen gas generated: 51.3 mL/g, hydrosilyl group/vinyl group: 1.1) which is represented by formula (E29) below, and 0.6 g of a 2-propanol solution containing 0.5% chloroplatinic acid were charged. The resulting mixture was heated at 110°C for 6 hours in order to cause a reaction. Subsequently, heating was performed under reduced pressure in order to remove the solvent by distillation. After the concentration had been adjusted to 30% by the addition of decamethylcyclopentasiloxane, filtration was performed. Hereby, a decamethylcyclopentasiloxane solution of a crosslinked organosilicon resin was prepared.

The decamethylcyclopentasiloxane solution of the crosslinked organosilicon resin was then heated to 120°C to 130°C under reduced pressure in order to remove decamethylcyclopentasiloxane. The product was a solid powder (weight-average molecular weight: 65,000). The reaction ratio of alkenyl groups was 92%. The amount of the hydrogen gas generated from the residual hydrosilyl groups was 0.7 mL/g.

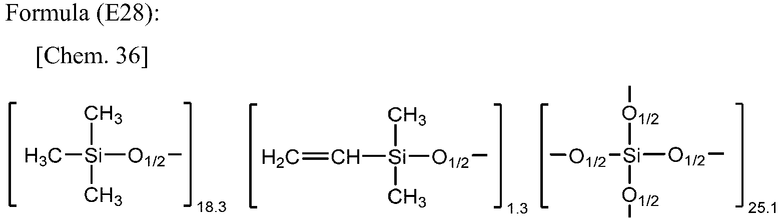

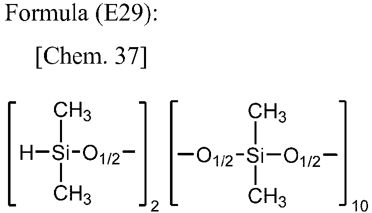

### [Example 15]

### Method for Producing 30% Solution of

### Crosslinked Organosilicon Resin/Decamethylcyclopentasiloxane

Into a reaction container, 1,000 g of a 50% decamethylcyclopentasiloxane solution of a powdery alkenyl group-containing organosilicon resin (weight-average molecular weight: 11,730, vinyl value: 0.307 mmol/g) represented by average compositional formula (E30) below, 700 g of decamethylcyclopentasiloxane, 78.5 g of organopolysiloxane having two terminal hydrosilyl groups (amount of hydrogen gas generated: 43.8 mL/g, hydrosilyl group/vinyl group: 1.0) which is represented by formula (E31) below, and 0.6 g of a 2-propanol solution containing 0.5% chloroplatinic acid were charged. The resulting mixture was heated at 120°C for 5 hours in order to cause a reaction. Subsequently, heating was performed under reduced pressure in order to remove the solvent by distillation. After the concentration had been adjusted to 30% by the addition of decamethylcyclopentasiloxane, filtration was performed. Hereby, a decamethylcyclopentasiloxane solution of a crosslinked organosilicon resin was prepared.

The decamethylcyclopentasiloxane solution of the crosslinked organosilicon resin was then heated to 120°C to 130°C under reduced pressure in order to remove decamethylcyclopentasiloxane. The product was a solid powder (weight-average molecular weight: 96,500). The reaction ratio of alkenyl groups was 97%. The amount of the hydrogen gas generated from the residual hydrosilyl groups was 0.9 mL/g.

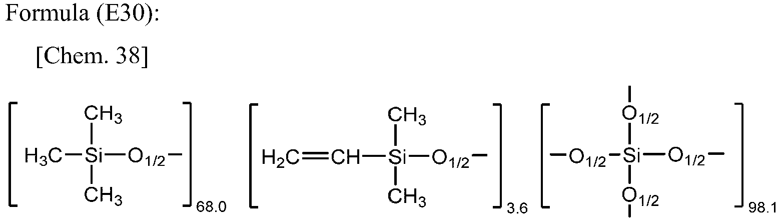

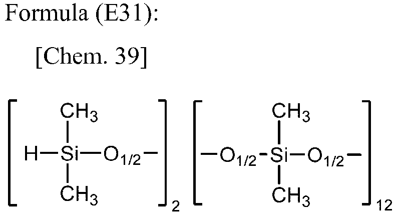

### [Comparative Example 1]

### Method for Producing 30% Solution of

### Crosslinked Organosilicon Resin/Decamethylcyclopentasiloxane

Into a reaction container, 1,000 g of a 50% decamethylcyclopentasiloxane solution of a powdery hydrosilyl group-containing organosilicon resin (weight-average molecular weight: 4,420, amount of hydrogen gas generated: 7.6 mL/g) represented by average compositional formula (E32) below, 700 g of decamethylcyclopentasiloxane, 157 g of organopolysiloxane having two terminal vinyl groups (vinyl value: 2.16 mmol/g, hydrosilyl group/vinyl group: 1.0) which is represented by formula (E33) below, and 0.6 g of a 2-propanol solution containing 0.5% chloroplatinic acid were charged. The resulting mixture was heated at 120°C for 5 hours in order to cause a reaction. Subsequently, heating was performed under reduced pressure in order to remove the solvent by distillation. After the concentration had been adjusted to 30% by the addition of decamethylcyclopentasiloxane, filtration was performed. Hereby, a decamethylcyclopentasiloxane solution of a crosslinked organosilicon resin was prepared.

The decamethylcyclopentasiloxane solution of the crosslinked organosilicon resin was then heated to 120°C to 130°C under reduced pressure in order to remove decamethylcyclopentasiloxane. The product was a solid powder (weight-average molecular weight: 128,000). The reaction ratio of hydrosilyl groups was 71%. The amount of the hydrogen gas generated from the residual hydrosilyl groups was 2.2 mL/g.

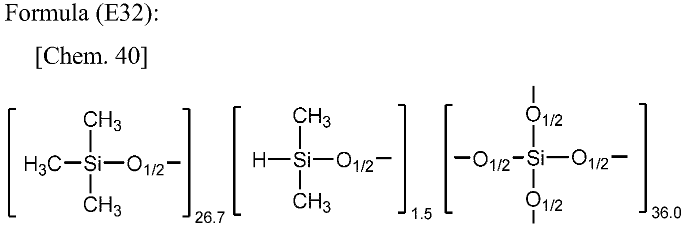

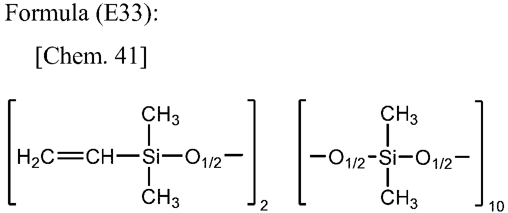

### [Comparative Example 2]

### Method for Producing 30% Solution of

### Crosslinked Organosilicon Resin/Decamethylcyclopentasiloxane

Into a reaction container, 1,000 g of a 50% decamethylcyclopentasiloxane solution of a powdery hydrosilyl group-containing organosilicon resin (weight-average molecular weight: 5,180, amount of hydrogen gas generated: 9.1 mL/g) represented by average compositional formula (E34) below, 188 g of organopolysiloxane having two terminal vinyl groups (vinyl value: 2.16 mmol/g, hydrosilyl group/vinyl group: 1.0) which is represented by formula (E35) below, 700 g of decamethylcyclopentasiloxane, and 0.6 g of a 2-propanol solution containing 0.5% chloroplatinic acid were charged. The resulting mixture was heated at 100°C for 6 hours in order to cause a reaction. Subsequently, heating was performed under reduced pressure in order to remove the solvent by distillation. To the reaction product, 250 g of ethanol was added. Subsequently, 5 g of a 5% aqueous sodium hydroxide solution was added to the reaction product in order to hydrolyze the unreacted hydrosilyl groups. Then, neutralization was performed by the addition of 0.63 g of concentrated hydrochloric acid. The reaction product was subsequently heated under reduced pressure in order to remove the solvent by distillation. Then, filtration was performed. Hereby, a decamethylcyclopentasiloxane solution of a crosslinked organosilicon resin was prepared.

The decamethylcyclopentasiloxane solution of the crosslinked organosilicon resin was then heated to 120°C to 130°C under reduced pressure in order to remove decamethylcyclopentasiloxane. The product was a solid powder (weight-average molecular weight: 96,000). The reaction ratio of hydrosilyl groups was 78%. The amount of the hydrogen gas generated from the residual hydrosilyl groups was 2.0 mL/g.

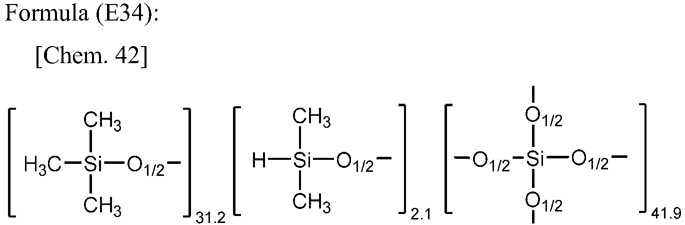

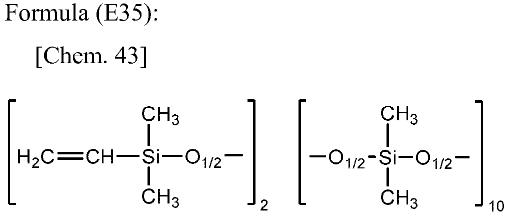

### [Comparative Example 3]

Into a reaction container, 1,000 g of a 50% decamethylcyclopentasiloxane solution of a powdery alkenyl group-containing organosilicon resin (weight-average molecular weight: 7,050, vinyl value: 0.794 mmol/g) represented by average compositional formula (E36) below, 700 g of decamethylcyclopentasiloxane, 190.7 g of organopolysiloxane having two terminal hydrosilyl groups (amount of hydrogen gas generated: 51.3 mL/g, hydrosilyl group/vinyl group: 1.1) which is represented by formula (E37) below, and 0.6 g of a 2-propanol solution containing 0.5% chloroplatinic acid were charged. The resulting mixture was heated at 110°C for 5 hours in order to cause a reaction. While the reaction was conducted, viscosity was increased and gelation occurred in the reaction container. It was not possible to form a film.

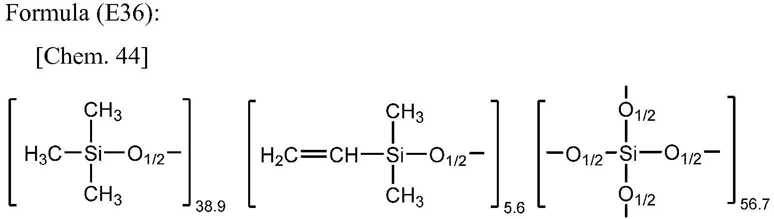

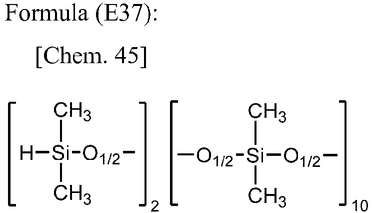

### [Comparative Example 4]

### Method for Producing 30% Solution of

### Crosslinked Organosilicon Resin/Decamethylcyclopentasiloxane

Into a reaction container, 1,000 g of a 50% decamethylcyclopentasiloxane solution of a powdery alkenyl group-containing organosilicon resin (weight-average molecular weight: 8,050, vinyl value: 0.224 mmol/g) represented by average compositional formula (E38) below, 700 g of decamethylcyclopentasiloxane, 190.3 g of organopolysiloxane having two terminal hydrosilyl groups (amount of hydrogen gas generated: 14.5 mL/g, hydrosilyl group/vinyl group: 1.1) which is represented by formula (E39) below, and 0.6 g of a 2-propanol solution containing 0.5% chloroplatinic acid were charged. The resulting mixture was heated at 110°C for 5 hours in order to cause a reaction. Subsequently, heating was performed under reduced pressure in order to remove the solvent by distillation. After the concentration had been adjusted to 30% by the addition of decamethylcyclopentasiloxane, filtration was performed. Hereby, a decamethylcyclopentasiloxane solution of a crosslinked organosilicon resin was prepared.

The decamethylcyclopentasiloxane solution of the crosslinked organosilicon resin was then heated to 120°C to 130°C under reduced pressure in order to remove decamethylcyclopentasiloxane. The product was gum-like (weight-average molecular weight: 681,000). The reaction ratio of alkenyl groups was 98%. The amount of the hydrogen gas generated from the residual hydrosilyl groups was 0.3 mL/g.

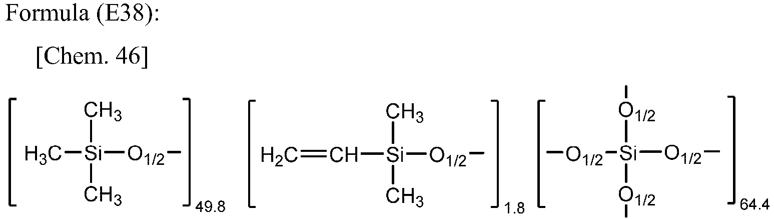

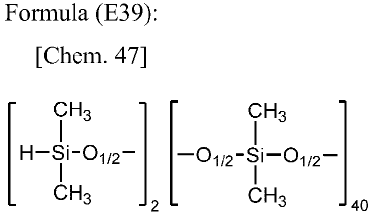

### [Comparative Example 5]

### Method for Producing 30% Solution of

### Crosslinked Organosilicon Resin/Decamethylcyclopentasiloxane

Into a reaction container, 1,000 g of a 50% decamethylcyclopentasiloxane solution of a powdery alkenyl group-containing organosilicon resin (weight-average molecular weight: 8,050, vinyl value: 0.224 mmol/g) represented by average compositional formula (E40) below, 700 g of decamethylcyclopentasiloxane, 372.9 g of organopolysiloxane having two terminal hydrosilyl groups (amount of hydrogen gas generated: 7.4 mL/g, hydrosilyl group/vinyl group: 1.1) which is represented by formula (E41) below, and 0.6 g of a 2-propanol solution containing 0.5% chloroplatinic acid were charged. The resulting mixture was heated at 110°C for 5 hours in order to cause a reaction. Subsequently, heating was performed under reduced pressure in order to remove the solvent by distillation. After the concentration had been adjusted to 30% by the addition of decamethylcyclopentasiloxane, filtration was performed. Hereby, a decamethylcyclopentasiloxane solution of a crosslinked organosilicon resin was prepared.

The decamethylcyclopentasiloxane solution of the crosslinked organosilicon resin was then heated to 120°C to 130°C under reduced pressure in order to remove decamethylcyclopentasiloxane. The product was gum-like powder (weight-average molecular weight: 724,000). The reaction ratio of alkenyl groups was 97%. The amount of the hydrogen gas generated from the residual hydrosilyl groups was 0.3 mL/g.

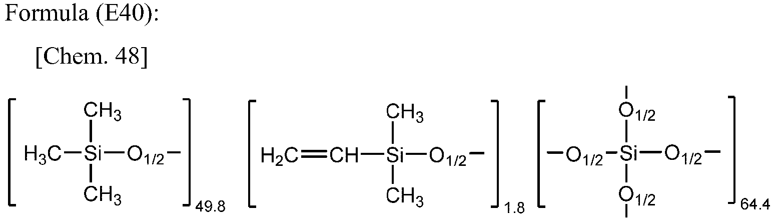

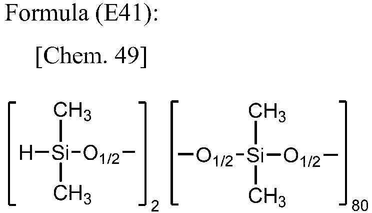

### [Comparative Example 6]

KF-7312J (trimethylsiloxysilicate: 50%, cyclopentasiloxane: 50%), produced by Shin-Etsu Chemical Co., Ltd.

### [Comparative Example 7]

KF-9021 (trimethylsiloxysilicate: 50%, cyclopentasiloxane: 50%), produced by Shin-Etsu Chemical Co., Ltd.

### <Stability Over Time>

Each of the decamethylcyclopentasiloxane solutions of the crosslinked organosilicon resins prepared in Examples and Comparative Examples was stored in a thermostat kept at 50°C, and the viscosity was measured after two weeks and after one month. An evaluation was made in accordance with the following criteria. Table 1 lists the results.
⊚ : The difference between the viscosity measured immediately after production and the viscosity measured subsequent to the storage at 50°C was less than 5%.
○: The difference between the viscosity measured immediately after production and the viscosity measured subsequent to the storage at 50°C was 5% or more and less than 10%.
×: The difference between the viscosity measured immediately after production and the viscosity measured subsequent to the storage at 50°C was 10% or more.

**[Table 1]**

| | After two weeks | After one month |
|---|---|---|
| Example 1 | ⊚ | ⊚ |
| Example 2 | ⊚ | ⊚ |
| Example 3 | ⊚ | ⊚ |
| Example 4 | ⊚ | ⊚ |
| Example 5 | ⊚ | ⊚ |
| Example 6 | ⊚ | ⊚ |
| Example 7 | ⊚ | ⊚ |
| Example 8 | ⊚ | ⊚ |
| Example 9 | ⊚ | ⊚ |
| Example 10 | ⊚ | ⊚ |
| Example 11 | ⊚ | ⊚ |
| Example 12 | ⊚ | ⊚ |
| Example 13 | ⊚ | ⊚ |
| Example 14 | ⊚ | ⊚ |
| Example 15 | ⊚ | ⊚ |
| Comparative Example 1 | ○ | × |
| Comparative Example 2 | × | × |

In all of Examples 1 to 15, the difference between the viscosity measured immediately after production and the viscosity measured after two weeks or one month was less than 5%, that is, suitable stability over time, was confirmed. It is considered that, since the alkenyl groups included in the organosilicon resin were highly reactive, the amount of residual hydrosilyl groups was small and the reaction that occurs over time was limited consequently. Comparative Example 1 is a solution of a crosslinked organosilicon resin prepared using a hydrosilyl group-containing organosilicon resin as a raw material. It is considered that viscosity was increased because reactivity was low compared with alkenyl groups, the amount of residual hydrosilyl groups was therefore large, and the reaction gradually occurred over time consequently. Comparative Example 2 is a solution produced by treating a crosslinked organosilicon resin prepared using a hydrosilyl group-containing organosilicon resin as a raw material with an alkali. It is considered that viscosity was increased because it was not possible to completely deactivate the hydrosilyl groups remaining in the organosilicon resin even by the alkali treatment and the reaction gradually occurred over time consequently. In addition, it is considered that the rate of increase in viscosity was increased because pH was shifted toward the basic region compared with Comparative Example 1.

The form, film-forming ability, and film properties (continuity, hardness, and flexibility) of each of the organosilicon resins prepared in Examples and Comparative Examples at 25°C were evaluated in accordance with the following method. Table 2 lists the results.

1) Each of the decamethylcyclopentasiloxane solutions or the other solution prepared in Examples and Comparative Examples above was heated at 120°C to 130°C under reduced pressure in order to remove decamethylcyclopentasiloxane or the other solvent and, subsequently, the form of the organosilicon resin at 25°C was observed.
2) As for film-forming ability, 1.5 g of a solution product diluted with a solvent (isododecane de or decamethylcyclopentasiloxane) to 30% was added dropwise to a PTFE (fluororesin) container, drying was then performed at 105°C for 3 hours, and whether a self-supporting film was formed was determined.
3) As for film continuity, whether the film prepared in 2) above was a continuous film (i.e., whether cracks are present or not) was determined. Samples free of cracks were evaluated as "Continuity: Yes".
4) As for film hardness, whether the film prepared in 2) above could be penetrated with the fingernail was determined. In the case where the film could be penetrated with the fingernail, an evaluation of "Soft" was given.
5) As for film stickiness, the stickiness of the film prepared in 2) above was evaluated as "No" or "Yes".
6) As for flexibility, a film was formed on an aluminum petri dish by the method described in 2) above, and the bendability of the film was evaluated. In the case where cracks were formed in the film, an evaluation of "No" was given in terms of flexibility.

**[Table 2]**

| | Form | Film-forming ability | Film continuity | Film hardness | Film stickiness | Flexibility |
|---|---|---|---|---|---|---|
| Example 1 | Solid | Yes | Yes | Soft | No | Yes |
| Example 2 | Solid | Yes | Yes | Hard | No | Yes |
| Example 3 | Solid | Yes | Yes | Hard | No | Yes |
| Example 4 | Solid | Yes | Yes | Hard | No | Yes |
| Example 5 | Solid | Yes | Yes | Soft | No | Yes |
| Example 6 | Solid | Yes | Yes | Hard | No | Yes |
| Example 7 | Solid | Yes | Yes | Hard | No | Yes |
| Example 8 | Solid | Yes | Yes | Hard | No | Yes |
| Example 9 | Solid | Yes | Yes | Hard | No | Yes |
| Example 10 | Solid | Yes | Yes | Hard | No | Yes |
| Example 11 | Solid | Yes | Yes | Hard | No | Yes |
| Example 12 | Solid | Yes | Yes | Hard | No | Yes |
| Example 13 | Solid | Yes | Yes | Soft | No | Yes |
| Example 14 | Solid | Yes | Yes | Hard | No | Yes |
| Example 15 | Solid | Yes | Yes | Hard | No | Yes |
| Comparative Example 3 | Gel-like | No | - | - | - | - |
| Comparative Example 4 | Gum-like | Yes | Yes | Soft | Yes | Yes |
| Comparative Example 5 | Gum-like | Yes | Yes | Soft | Yes | Yes |
| Comparative Example 6 | Solid | Yes | No | Hard | No | No |
| Comparative Example 7 | Solid | Yes | No | Hard | No | No |

In all of Examples 1 to 15, a homogeneous and continuous film was formed, and the film was free of stickiness and brittleness and had high flexibility when folded or bent. In Comparative Example 3, where an organosilicon resin including a large amount of alkenyl groups was reacted with an organohydrogenpolysiloxane, crosslinking density was high and a gel was formed. As a result, film formability could not be achieved. In Comparative Examples 4 and 5, films were formed of a crosslinked organosilicon resin prepared by performing crosslinking with an organohydrogenpolysiloxane having a large chain length. Both films were continuous and soft. Since the films were gum-like, they had high stickiness. In Comparative Examples 6 and 7, films were formed of a non-crosslinked organosilicon resin composed of the M and Q units. The films were hard and free of stickiness, but had cracks formed therein. The films were easily broken when folded or bent. Thus, the crosslinked organosilicon resin according to the present invention had markedly improved performance in terms of flexion and stickiness, compared with the non-crosslinked organosilicon resins known in the related art.

To each of the films prepared in Examples 1 to 15 and Comparative Examples 6 and 7, 3 µL of squalane, oleic acid, and triethylhexanoin were dropped, and the contact angles were measured. Table 3 lists the results.

**[Table 3]**

| Contact angle [°] | Squalane | Oleic acid | Triethylhexanoin |
|---|---|---|---|
| Example 1 | 41.8 | 42.1 | 42.9 |
| Example 2 | 48.5 | 49.5 | 48.0 |
| Example 3 | 42.1 | 42.8 | 39.9 |
| Example 4 | 43.8 | 42.7 | 41.9 |
| Example 5 | 39.8 | 40.1 | 40.8 |
| Example 6 | 45.6 | 49.8 | 45.7 |
| Example 7 | 40.9 | 43.8 | 41.2 |
| Example 8 | 44.3 | 45.1 | 43.0 |
| Example 9 | 45.2 | 49.0 | 46.5 |
| Example 10 | 43.7 | 46.1 | 41.8 |
| Example 11 | 48.5 | 46.5 | 46.3 |
| Example 12 | 45.4 | 45.9 | 45.8 |
| Example 13 | 42.2 | 45.6 | 44.0 |
| Example 14 | 43.8 | 45.8 | 40.4 |
| Example 15 | 45.3 | 46.5 | 40.0 |
| Comparative Example 6 | 21.2 | 10.5 | 10.3 |
| Comparative Example 7 | 25.4 | 14.3 | 12.8 |

In Examples 1 to 15, the contact angle was large with respect to all the oils, that is, squalane, oleic acid and triethylhexanoin. That is, high oil resistance was confirmed. In contrast, in Comparative Examples 6 and 7, the contact angle was small with respect to all the oils. That is, low oil resistance was confirmed. In general, the higher the molecular weight of a non-crosslinked organosilicon resin, the higher the oil resistance of the non-crosslinked organosilicon resin. Since there is a limitation on the increase in the molecular weight of a non-crosslinked organosilicon resin, oil resistance also has its critical point. Crosslinking an organosilicon resin with a crosslinking agent results in a virtual increase in the molecular weight of the organosilicon resin and consequently increases the critical point. Thus, the crosslinked organosilicon resin according to the present invention had a certain degree of oil resistance which cannot be achieved by the non-crosslinked organosilicon resins known in the related art.

The solid powder prepared in Example 3 above was mixed with each of isododecane, methyl trimethicone, dimethicone (KF-96-2cs and 6cs), triethylhexanoin and isotridecyl isononanoate at a solid concentration of 50% in order to conduct a solubility test. Table 4 lists the results.
○: Transparent, dissolved
△: Slightly turbid
×: Separated

**[Table 4]**

| Oil | Appearance |
|---|---|
| Isododecane | ○ |
| Methyl trimethicone | ○ |
| KF-96-2cs | ○ |
| KF-96-6cs | ○ |
| Triethylhexanoin | ○ |
| Isotridecyl isononanoate | ○ |

Note that the polymer was also soluble in the silicones and ester oils used for cosmetics which are other than the oils described above. The viscosities of the above solutions can be changed by adjusting the composition or molecular weight of the polymer.

### [Example 16 and Comparative Examples 8 and 9]

Emulsion-type cream foundations having the compositions described in Table 4 below were prepared.

**[Table 5]**

| No. | Composition (%) | Blank | Example | Comparative Example | |
|---|---|---|---|---|---|
| | | | 16 | 8 | 9 |
| 1 | Crosslinked polyether-modified silicone mixture (*1) | 3 | 3 | 3 | 3 |
| 2 | Crosslinked silicone mixture (*2) | 2 | 2 | 2 | 2 |
| 3 | Polyether-modified silicone (*3) | 2 | 2 | 2 | 2 |
| 4 | Diphenylsiloxy phenyl trimethicone (*4) | 5 | 5 | 5 | 5 |
| 5 | Disteardimonium hectorite | 1 | 1 | 1 | 1 |
| 6 | Cyclopentasiloxane | Balance | Balance | Balance | Balance |
| 7 | Silicone composite powder (*5) | 2 | 2 | 2 | 2 |
| 8 | Isotridecyl isononanoate | 1.7 | 1.7 | 1.7 | 1.7 |
| 9 | Acrylic silicone (*6) | 0.25 | 0.25 | 0.25 | 0.25 |
| 10 | Silicone-treated titanium oxide (*7) | 8.8 | 8.8 | 8.8 | 8.8 |
| 11 | Silicone-treated yellow iron oxide (*8) | 0.7 | 0.7 | 0.7 | 0.7 |
| 12 | Silicone-treated red iron oxide (*9) | 0.3 | 0.3 | 0.3 | 0.3 |
| 13 | Silicone-treated black iron oxide (*10) | 0.2 | 0.2 | 0.2 | 0.2 |
| 14 | Solution (30%) prepared in Example 2 | | 10 | | |
| 15 | Trimethylsiloxysilicate (*11) | | | 6 | 10 |
| 16 | BG | 5 | 5 | 5 | 5 |
| 17 | Na citrate | 0.2 | 0.2 | 0.2 | 0.2 |
| 18 | Na chloride | 0.5 | 0.5 | 0.5 | 0.5 |
| 19 | Water | 35 | 35 | 35 | 35 |
| | Total | 100 | 100 | 100 | 100 |

| | | | | | |
|---|---|---|---|---|---|
| (*1) KSG-210 produced by Shin-Etsu Chemical Co., Ltd. (*2) KSG-15 produced by Shin-Etsu Chemical Co., Ltd. (*3) KF-6017 produced by Shin-Etsu Chemical Co., Ltd. (*4) KF-56A produced by Shin-Etsu Chemical Co., Ltd. (*5) KSP-100 produced by Shin-Etsu Chemical Co., Ltd. (*6) KP-578 produced by Shin-Etsu Chemical Co., Ltd. (*7) KTP-09W produced by Shin-Etsu Chemical Co., Ltd. (*8) KTP-09Y produced by Shin-Etsu Chemical Co., Ltd. (*9) KTP-09R produced by Shin-Etsu Chemical Co., Ltd. (*10) KTP-09B produced by Shin-Etsu Chemical Co., Ltd. (*11) KF-9021 produced by Shin-Etsu Chemical Co., Ltd. (trimethylsiloxysilicate: 50%, cyclopentasiloxane: 50%) | | | | | |

### <Preparation of Cosmetic>

A: The components 1 to 7 above were mixed with one another homogeneously.
B: The components 8 to 13 above were dispersed using a three-roll mill.
C: The components 16 to 19 were mixed with one another homogeneously.
D: The mixture prepared in C was added to the mixture prepared in A, and emulsification was then performed. To the emulsion, B and the components 14 and 15 were added to prepare an emulsion-type cream foundation. The emulsion-type cream foundation was evaluated as follows.

### [Feel on Use (Sticky Feel)]

The emulsion-type cream foundation was evaluated by ten female expert panelists in terms of "sticky feel" in accordance with the following evaluation criteria, and the average of the evaluation results was calculated. On the basis of the average score, rating was done in accordance with the rating criteria below.

### [Evaluation Criteria]

5: Excellent
4: Good
3: Fair
2: Slightly poor
1 : Poor

### [Rating Criteria]

⊚: The average score was 4.0 or more
○: The average score was 3.0 or more and less than 4.0
△: The average score was 2.0 or more and less than 3.0
× : The average score was less than 2.0

### [Elongation (Spreadability)]

The emulsion-type cream foundation was subjected to a dynamical friction test using "TL201Tt" produced by Trinity-Lab. Inc. On the basis of the comparison with the blank sample, an evaluation was made in accordance with the following criteria.
⊚: Less than 105%
○: 105% or more and less than 110%
△: 110% or more and less than 115%
×: 115% or more

### [Prevention of Secondary Adhesion]

The emulsion-type cream foundation was applied to a sheet of synthetic leather in the same amount. After drying had been performed at 50°C for 3 hours, a filter paper sheet was superimposed thereon and a load of 500 g was applied thereto. The transfer to the filter paper was compared and evaluated in accordance with the following criteria.
⊚ : Transfer hardly occurred.
○: Transfer slightly occurred.
△: Transfer occurred.
× : Transfer significantly occurred.

In all evaluations, evaluation grades of "○" or higher were considered passed.

**[Table 6]**

| Evaluation Item | Example | Comparative Example | |
|---|---|---|---|
| | 16 | 8 | 9 |
| Sticky feel | ○ | △ | × |
| Elongation | ⊚ | △ | × |
| Prevention of Secondary Adhesion | ○ | × | △ |

As described in Table 6 above, the cosmetic preparation according to the present invention was excellent in terms of feel on use, elongation (spreadability) and prevention of secondary adhesion, compared with Comparative Examples 8 and 9.

Examples of the cosmetic preparation are described below. Hereinafter, evaluations were made in accordance with the same criteria as described above.

### [Example 17]

Non-Aqueous Foundation

### <Preparation of Cosmetic>

A: The components 1 to 11 were mixed with one another homogeneously.
B: The components 12 to 15 were mixed with one another homogeneously using a homomixer.
C: The mixture prepared in B was added to the mixture prepared in A, and the resulting mixture was stirred homogeneously. The component 16 was then added to the mixture to prepare a non-aqueous foundation.

| Composition | % |
|---|---|
| 1. Solution (30%) prepared in Example 15 | 12 |
| 2. Silicone composite powder (*1) | 8 |
| 3. Diphenylsiloxy phenyl trimethicone (*2) | 3 |
| 4. Silicone-alkyl branched polyether-modified silicone (*3) | 2 |
| 5. Acrylic silicone (*4) | 1.5 |
| 6. Disteardimonium hectorite | 1.5 |
| 7. Silica dimethyl silylate | 1.6 |
| 8. Homosalate | 3 |
| 9. Cetyl ethylhexanoate | 3 |
| 10. Isododecane | 10 |
| 11. Dimethicone (2cs) | Balance |
| 12. Dimethicone (6cs) | 10 |
| 13. Silicone-alkyl branched polyglycerol-modified silicone (*5) | 0.75 |
| 14. Metal soap-treated microparticulate titanium oxide | 2.25 |
| 15. Silicone-treated pigment (*6) | 10 |
| 16. Ethanol | 6 |
| Total | 100.00 |

| | |
|---|---|
| (*1) KSP-100 produced by Shin-Etsu Chemical Co., Ltd. (*2) KF-56A produced by Shin-Etsu Chemical Co., Ltd. (*3) KF-6038 produced by Shin-Etsu Chemical Co., Ltd. (*4) KP-550 produced by Shin-Etsu Chemical Co., Ltd. (*5) KF-6115 produced by Shin-Etsu Chemical Co., Ltd. (*6) KTP-09W, Y, R, B produced by Shin-Etsu Chemical Co., Ltd. | |

It was confirmed that the non-aqueous foundation had a suitable feel on use and suitable elongation and was excellent in terms of prevention of secondary adhesion.

### [Example 18]

W/O Foundation

### <Preparation of Cosmetic>

A: The components 2 to 9 were mixed with one another homogeneously.
B: The components 10 to 13 were mixed with one another homogeneously using a high-pressure mixer.
C: The components 14 to 17 were mixed with one another homogeneously.
D: The mixture prepared in C was added to the mixture prepared in A, and emulsification was then performed. The mixture prepared in B and the component 1 were added to the resulting emulsion to prepare a W/O foundation.

| Composition | % |
|---|---|
| 1. Solution (30%) prepared in Example 7 | 3 |
| 2. Crosslinked polyglycerol-modified silicone mixture (*1) | 4 |
| 3. Diphenylsiloxy phenyl trimethicone (*2) | 3 |
| 4. Silicone-alkyl branched polyglycerol-modified silicone (*3) | 3 |
| 5. Phenyl-modified partially crosslinked dimethylpolysiloxane composition (*4) | 2 |
| 6. Ethyl methicone (*5) | Balance |
| 7. Ethylhexyl salicylate | 5 |
| 8. Bis-ethylhexyloxyphenol methoxyphenyl triazine | 1 |
| 9. Disteardimonium hectorite | 1.2 |
| 10. Isononyl isononanoate | 5.7 |
| 11. Silicone composite powder (*6) | 1 |
| 12. Acrylic silicone (*7) | 0.2 |
| 13. Silicone-treated pigment (*8) | 10 |
| 14. BG | 8 |
| 15. Na citrate | 0.2 |
| 16. Na chloride | 0.5 |
| 17. Water | 44.9 |
| Total | 100.0 |

| | |
|---|---|
| (*1) KSG-830 produced by Shin-Etsu Chemical Co., Ltd. (*2) KF-56A produced by Shin-Etsu Chemical Co., Ltd. (*3) KF-6105 produced by Shin-Etsu Chemical Co., Ltd. (*4) KSG-18A produced by Shin-Etsu Chemical Co., Ltd. (*5) KF-4422 produced by Shin-Etsu Chemical Co., Ltd. (*6) KSP-105 produced by Shin-Etsu Chemical Co., Ltd. (*7) KP-578 produced by Shin-Etsu Chemical Co., Ltd. (*8) KTP-09W, Y, R, B produced by Shin-Etsu Chemical Co., Ltd. | |

It was confirmed that the W/O foundation had a suitable feel on use and suitable elongation and was excellent in terms of prevention of secondary adhesion.

### [Example 19]

O/W Makeup Base

### <Preparation of Cosmetic>

A: The components 1 to 4 were mixed with one another homogeneously.
B: The components 5 to 14 were mixed with one another homogeneously.
C: The mixture prepared in A was added to the mixture prepared in B, and emulsification was then performed. Hereby, an O/W makeup base was prepared.

| Composition | % |
|---|---|
| 1. Solution (30%) prepared in Example 7 | 3 |
| 2. Partially crosslinked silicone mixture (*1) | 5 |
| 3. Diphenylsiloxy phenyl trimethicone (*2) | 5 |
| 4. Microparticulate titanium oxide dispersion (*3) | 10 |
| 5. BG | 10 |
| 6. Betaine | 1 |
| 7. Polyether-modified silicone (*3) | 1.5 |
| 8. Sodium acrylate-sodium acryloyldimethyltaurate copolymer composition (*4) | 1 |
| 9. (Acrylates/alkyl acrylate (C10-30)) crosspolymer (2% aqueous solution) | 20 |
| 10. Arginine (10% aqueous solution) | QS |
| 11. Bisabolol | 0.1 |
| 12. Ethylhexylglycerin | 0.1 |
| 13. EDTA-2Na (10% aqueous solution) | 0.1 |
| 14. Water | Balance |
| Total | 100.0 |

| | |
|---|---|
| (*1) KSG-19 produced by Shin-Etsu Chemical Co., Ltd. (*2) KF-56A produced by Shin-Etsu Chemical Co., Ltd. (*3) SPD-T7 produced by Shin-Etsu Chemical Co., Ltd. (*4) KF-6043 produced by Shin-Etsu Chemical Co., Ltd. (*5) SIMULGEL EG produced by SEPPIC | |

It was confirmed that the O/W makeup base had a suitable feel on use and suitable elongation and was excellent in terms of resistance to transfer of foundation deposited thereon.

### [Example 20]

Oil-Based Mascara

### <Preparation of Cosmetic>

A: The components 1 to 9 were heated to 95°C and subsequently mixed with one another homogeneously.
B: The components 10 to 14 were mixed with one another homogeneously using a disper.
C: The mixture prepared in B was added to the mixture prepared in A, and the resulting mixture was stirred at 90°C homogeneously. Then, cooling was slowly performed. Hereby, an oil-based mascara was prepared.

| Composition | % |
|---|---|
| 1. Solution (30% solution) prepared in Example 15 | 12 |
| 2. Isododecane solution of trimethylsiloxysilicate (*1) | 10 |
| 3. Dextrin palmitate (*2) | 2 |
| 4. Paraffin wax | 6 |
| 5. Microcrystalline wax | 7 |
| 6. Isododecane | 20 |
| 7. Silicone-treated black iron oxide (*3) | 5 |
| 8. Silicone-treated talc (*3) | 5 |
| 9. Polymethyl silsesquioxane (*4) | 5 |
| 10. Organically modified clay mineral | 6 |
| 11. Silicone branched polyether-modified silicone (*5) | 1.5 |
| 12. Propylene carbonate | 1.6 |
| 13. Methyl p-hydroxybenzoate | 0.1 |
| 14. Isododecane | Balance |
| Total | 100.0 |

| | |
|---|---|
| (*1) X-21-5595 produced by Shin-Etsu Chemical Co., Ltd. (*2) Leoparl KL2 produced by Chiba Flour Milling Co., Ltd. (*3) KF-9909 treatment produced by Shin-Etsu Chemical Co., Ltd. (*4) KMP-590 produced by Shin-Etsu Chemical Co., Ltd. (*5) KF-6028 produced by Shin-Etsu Chemical Co., Ltd. | |

It was confirmed that the oil-based mascara had a suitable feel on use, suitable long-lasting performance, and suitable elongation and finish and was excellent in terms of rubfastness. It is possible to adjust the film performance properties and feel on use, such as finish, by using a hard and brittle film, such as trimethylsiloxysilicate, in combination.

### [Example 21]

W/O Mascara

### <Preparation of Cosmetic>

A: The components 1 to 7 were heated to 95°C and then mixed with one another homogeneously.
B: The components 11 to 14 were mixed with one another homogeneously using a disper, the resulting mixture was added to A. Subsequently, heating was performed to 90°C.
C: The components 8 to 10 were added to the mixture prepared in B. The resulting mixture was heated to 85°C and then stirred homogeneously.
D: The components 15 to 17 were heated to 85°C and then mixed with one another homogeneously.
E: The mixture prepared in D was added to the mixture prepared in C, and emulsification was then performed. Subsequently, cooling was slowly performed. Hereby, a W/O mascara was prepared.

| Composition | % |
|---|---|
| 1. Solution (30% solution) prepared in Example 11 | 8 |
| 2. Solution of acrylic-silicone-based graft copolymer (*1) | 9 |
| 3. Dextrin (palmitate/ethylhexanoate) (*2) | 3 |
| 4. Silicone wax (*3) | 5 |
| 5. Ceresin | 2.5 |
| 6. Beeswax | 4 |
| 7. Diphenylsiloxy phenyl trimethicone (*4) | 3 |
| 8. Silicone-treated black iron oxide (*5) | 5 |
| 9. Silicone-treated talc (*5) | 4.5 |
| 10. Amorphous silicic anhydride (*6) | 2.7 |
| 11. Isododecane | Balance |
| 12. Organically modified clay mineral | 4 |
| 13. Branched polyether-modified silicone (*7) | 2.2 |
| 14. Propylene carbonate | 1.3 |
| 15. Phenoxyethanol | 0.2 |
| 16. 1,3-Butylene glycol | 2 |
| 17. Purified water | 12.8 |
| Total | 100.0 |

| | |
|---|---|
| (*1) KP-550 produced by Shin-Etsu Chemical Co., Ltd. (*2) Leoparl TT2 produced by Chiba Flour Milling Co., Ltd. (*3) KP-561P produced by Shin-Etsu Chemical Co., Ltd. (*4) KF-56A produced by Shin-Etsu Chemical Co., Ltd. (*5) KF-9901 treatment produced by Shin-Etsu Chemical Co., Ltd. (*6) AEROSIL972 produced by Nippon Aerosil Co., Ltd. (*7) KF-6017 produced by Shin-Etsu Chemical Co., Ltd. | |

It was confirmed that the W/O mascara had a suitable feel on use, suitable long-lasting performance, and suitable elongation and finish and was excellent in terms of rubfastness. It is possible to adjust the film performance properties and feel on use, such as finish, by using a soft film, such as a silicone-modified acrylic polymer, in combination.

### [Example 22]

Lipstick

### <Preparation of Cosmetic>

A: The components 9 to 16 were dispersed using a three-roll mill.
B: The components 1 to 8 were heated to 95°C and then mixed with one another homogeneously.
C: The mixture prepared in A, the mixture prepared in B, and the components 17 and 18 were mixed with one another homogeneously, and the resulting mixture was heated to 85°C.
D: The mixture prepared in C was charged into a stick container. Hereby, a lipstick was prepared.

| Composition | % |
|---|---|
| 1. Synthetic wax | 7 |
| 2. Paraffin wax | 3 |
| 3. Silicone wax (*1) | 10.5 |
| 4. Triethylhexanoin | 15.5 |
| 5. Neopentyl glycol diethylhexanoate | 14 |
| 6. Neopentyl glycol dicaprate | 7 |
| 7. Hydrogenated polyisobutene | 20 |
| 8. Diphenyl dimethicone (*2) | 7.5 |
| 9. Talc | 0.7 |
| 10. Red No. 201 | QS |
| 11. Red No. 202 | QS |
| 12. Yellow No. 4 AL | QS |
| 13. Silicone-treated titanium oxide (*3) | 3 |
| 14. Silicone-treated black iron oxide (*3) | QS |
| 15. Silicone-treated red iron oxide (*3) | QS |
| 16. Diglyceryl triisostearate | 4 |
| 17. Silicone-treated mica (*3) | 5.5 |
| 18. Solution (30% solution) prepared in Example 2 | 1 |
| Total | 100.0 |

| | |
|---|---|
| (*1) KP-561P produced by Shin-Etsu Chemical Co., Ltd. (*2) KF-54HV produced by Shin-Etsu Chemical Co., Ltd. (*3) KF-574 treatment produced by Shin-Etsu Chemical Co., Ltd. | |

It was confirmed that the lipstick had a suitable feel on use, suitable long-lasting performance, and suitable elongation and finish and was excellent in terms of rubfastness.

### [Example 23]

W/O Sunscreen Milk

### <Preparation of Cosmetic>

A: The components 1 to 12 were mixed with one another homogeneously.
B: The components 15 to 21 were mixed with one another homogeneously.
C: The mixture prepared in B was added to the mixture prepared in A, and emulsification was then performed. The components 13 and 14 were added to the emulsion, and the resulting mixture was stirred homogeneously. Hereby, a W/O sunscreen milk was prepared.

| Composition | % |
|---|---|
| 1. Solution (30% solution) prepared in Example 9 | 3 |
| 2. Phenyl-modified crosslinked dimethylpolysiloxane composition (*1) | 3 |
| 3. Alkyl-silicone branched polyether-modified silicone (*2) | 2 |
| 4. Decamethylcyclopentasiloxane | 20 |
| 5. Diphenylsiloxy phenyl trimethicone (*3) | 5.5 |
| 6. Isononyl isononanoate | 5 |
| 7. Stearyl glycyrrhetinate | 0.2 |
| 8. BHT | 0.1 |
| 9. 2-Ethylhexyl p-methoxycinnamate | 7.5 |
| 10. Octocrylene | 2.5 |
| 11. 2-[4-(Diethylamino)-2-hydroxybenzoyl]benzoic acid hexyl ester | 1 |
| 12. Silicone composite powder (*4) | 0.5 |
| 13. Dispersion of microparticulate titanium oxide (*5) | 5 |
| 14. Dispersion of microparticulate zinc oxide (*6) | 10 |
| 15. 1,3-Butylene glycol | 3 |
| 16. Ethanol | 6 |
| 17. Sodium citrate | 0.2 |
| 18. Na hydroxide | QS |
| 19. Ascorbic acid 2-glucoside | 2 |
| 20. Ethylenediamine tetraacetate | 0.1 |
| 21. Purified water | B alance |
| Total | 100.0 |

| | |
|---|---|
| (*1) KSG-18A produced by Shin-Etsu Chemical Co., Ltd. (*2) KF-6038 produced by Shin-Etsu Chemical Co., Ltd. (*3) KF-56A produced by Shin-Etsu Chemical Co., Ltd. (*4) KSP-105 produced by Shin-Etsu Chemical Co., Ltd. (*5) SPD-T7 produced by Shin-Etsu Chemical Co., Ltd. (*6) SPD-Z5 produced by Shin-Etsu Chemical Co., Ltd. | |

It was confirmed that the W/O sunscreen milk had a suitable feel on use, suitable long-lasting performance, and excellent elongation.

### [Example 24]

W/O Sunscreen Milk

### <Preparation of Cosmetic>

A: The components 1 to 7 were mixed with one another homogeneously.
B: The components 10 to 13 were mixed with one another homogeneously.
C: The mixture prepared in B was added to the mixture prepared in A, and emulsification was then performed. The components 8 and 9 were added to the emulsion, and the resulting mixture was stirred homogeneously. Hereby, a W/O sunscreen milk was prepared.

| Composition | % |
|---|---|
| 1. Solution (30% solution) prepared in Example 5 | 2 |
| 2. Crosslinked polyether-modified silicone composition (*1) | 3 |
| 3. Crosslinked dimethylpolysiloxane composition (*2) | 2 |
| 4. Silicone-branched polyether-modified silicone (*3) | 1 |
| 5. Dimethylpolysiloxane (6cs) | 5 |
| 6. Decamethylcyclopentasiloxane | 3 |
| 7. Isotridecyl isononanoate | 4 |
| 8. Dispersion of microparticulate titanium oxide (*4) | 25 |
| 9. Dispersion of microparticulate zinc oxide (*5) | 35 |
| 10. Dipropylene glycol | 2 |
| 11. Sodium citrate | 0.2 |
| 12. Sodium chloride | 1 |
| 13. Purified water | Balance |
| Total | 100.0 |

| | |
|---|---|
| (*1) KSG-210 produced by Shin-Etsu Chemical Co., Ltd. (*2) KSG-19 produced by Shin-Etsu Chemical Co., Ltd. (*3) KF-6028 produced by Shin-Etsu Chemical Co., Ltd. (*4) SPD-T5 produced by Shin-Etsu Chemical Co., Ltd. (*5) SPD-Z5 produced by Shin-Etsu Chemical Co., Ltd. | |

It was confirmed that the W/O sunscreen milk had a suitable feel on use, suitable long-lasting performance, and excellent elongation.

### [Example 25]

W/O Cream Foundation

### <Preparation of Cosmetic>

A: The components 9 to 12 were dispersed using a three-roll mill.
B: The components 3 to 5 were mixed with one another homogeneously. The components 1, 2 and 6 to 8 were added to the resulting mixture. Subsequently, stirring was performed homogeneously.
C: The components 13 to 17 were mixed with one another homogeneously.
D: The mixture prepared in C was added to the mixture prepared in B, and emulsification was then performed. The mixture prepared in A was added to the emulsion. Hereby, a W/O cream foundation was prepared.

| Composition | % |
|---|---|
| 1. Alkyl-modified crosslinked polyether-modified silicone composition (*1) | 3.5 |
| 2. Alkyl-modified crosslinked dimethylpolysiloxane composition (*2) | 6 |
| 3. Alkyl-branched polyether-modified silicone (*3) | 3 |
| 4. Organically modified clay mineral | 1.2 |
| 5. Decamethylcyclopentasiloxane | 20 |
| 6. 2-Ethylhexyl p-methoxycinnamate | 7.5 |
| 7. Solution (30% solution) prepared in Example 6 | 2 |
| 8. Phenyl-modified silicone composite powder (*4) | 2 |
| 9. Ethylhexyl palmitate | 7 |
| 10. Acrylic-silicone-type graft copolymer (*5) | 0.2 |
| 11. Silicone-treated titanium oxide (*6) | 8.5 |
| 12. Silicone-treated iron oxide (*7) | QS |
| 13. 1,3-Butylene glycol | 5 |
| 14. Methyl p-hydroxybenzoate | 0.15 |
| 15. Sodium citrate | 0.2 |
| 16. Sodium chloride | 0.5 |
| 17. Purified water | Balance |
| Total | 100.0 |

| | |
|---|---|
| (*1) KSG-330 produced by Shin-Etsu Chemical Co., Ltd. (*2) KSG-41A produced by Shin-Etsu Chemical Co., Ltd. (*3) KF-6048 produced by Shin-Etsu Chemical Co., Ltd. (*4) KSP-300 produced by Shin-Etsu Chemical Co., Ltd. (*5) KP-578 produced by Shin-Etsu Chemical Co., Ltd. (*6) KTP-09W produced by Shin-Etsu Chemical Co., Ltd. (*7) KTP-09Y, R, B produced by Shin-Etsu Chemical Co., Ltd. | |

It was confirmed that the W/O cream foundation had a suitable feel on use, suitable long-lasting performance, and suitable elongation and finish and was excellent in terms of rubfastness.

### [Example 26]

W/O Liquid Foundation

### <Preparation of Cosmetic>

A: The components 7 to 13 were dispersed using a disper.
B: The components 4 to 6 were mixed with one another homogeneously while being heated. The components 1 to 3 were added to the resulting mixture. Subsequently, stirring was performed homogeneously.
C: The components 14 to 19 were mixed with one another homogeneously.
D: The mixture prepared in C was added to the mixture prepared in B, and emulsification was then performed. The mixture prepared in A was added to the emulsion. Hereby, a W/O liquid foundation was prepared.

| Composition | % |
|---|---|
| 1. Crosslinked polyether-modified silicone composition (*1) | 3.5 |
| 2. Phenyl-modified crosslinked dimethylpolysiloxane composition (*2) | 5 |
| 3. Diphenylsiloxy phenyl trimethicone (*4) | 9 |
| 4. Silicone-branched polyether-modified silicone (*3) | 3 |
| 5. Organically modified clay mineral | 0.8 |
| 6. Decamethylcyclopentasiloxane | 15 |
| 7. Isopropyl myristate | 6 |
| 8. Solution (30% solution) prepared in Example 1 | 1 |
| 9. Metal soap-treated microparticulate titanium oxide (average primary particle size: 20 nm) | 5 |
| 10. Alkylsilane-treated titanium oxide (*5) | 6.5 |
| 11. Alkylsilane-treated yellow iron oxide (*5) | QS |
| 12. Alkylsilane-treated red iron oxide (*5) | QS |
| 13. Alkylsilane-treated black iron oxide (*5) | QS |
| 14. Glycerin | 2 |
| 15. Dipropylene glycol | 3 |
| 16. Phenoxyethanol | 0.2 |
| 17. Sodium citrate | 0.2 |
| 18. Sodium chloride | 0.5 |
| 19. Purified water | 37 |
| Total | **100.0** |

| | |
|---|---|
| (*1) KSG-210 produced by Shin-Etsu Chemical Co., Ltd. (*2) KSG-18A produced by Shin-Etsu Chemical Co., Ltd. (*3) KF-6028 produced by Shin-Etsu Chemical Co., Ltd. (*4) KF-56A produced by Shin-Etsu Chemical Co., Ltd. (*5) AES-3083 treatment produced by Shin-Etsu Chemical Co., Ltd. | |

It was confirmed that the W/O liquid foundation had a suitable feel on use, suitable long-lasting performance, and suitable elongation and finish and was excellent in terms of rubfastness.

### [Example 27]

W/O Stick Foundation

### <Preparation of Cosmetic>

A: The components 10 to 14 were dispersed using a three-roll mill.
B: The components 1 to 9 were heated to 95°C and subsequently mixed with one another homogeneously.
C: A and the components 15 and 16 were mixed with one another homogeneously, and heating was subsequently performed to 85°C.
D: The mixture prepared in C was added to the mixture prepared in B. Then, emulsification was performed at 85°C. The resulting emulsion was charged into a stick container and subsequently cooled slowly. Hereby, a W/O stick foundation was prepared.

| Composition | % |
|---|---|
| 1. Crosslinked polyglycerol-modified silicone composition (*1) | 4.5 |
| 2. Silicone-alkyl branched polyether-modified silicone (*2) | 1.5 |
| 3. Stearoyl inulin (*3) | 1.8 |
| 4. Ceresin | 6 |
| 5. Neopentyl glycol diethylhexanoate | 6 |
| 6. Cetyl ethylhexanoate | 4 |
| 7. Dimethylpolysiloxane (6cs) | 11.5 |
| 8. Polymethyl silsesquioxane (*4) | 1.5 |
| 9. Solution (30% solution) prepared in Example 2 | 1 |
| 10. Silicone-treated titanium oxide (*5) | 6.5 |
| 11. Silicone-treated iron oxide (*6) | QS |
| 12. Polyether-modified silicone (*7) | 0.2 |
| 13. Polyether-modified silicone (*8) | 0.3 |
| 14. Dipropylene glycol | 5 |
| 15. Methyl p-hydroxybenzoate | 0.1 |
| 16. Purified water | Balance |
| Total | 100.0 |

| | |
|---|---|
| (*1) KSG-710 produced by Shin-Etsu Chemical Co., Ltd. (*2) KF-6038 produced by Shin-Etsu Chemical Co., Ltd. (*3) Leoparl ISK2 produced by Chiba Flour Milling Co., Ltd. (*4) KMP-590 produced by Shin-Etsu Chemical Co., Ltd. (*5) KTP-09W produced by Shin-Etsu Chemical Co., Ltd. (*6) KTP-09R, Y, B produced by Shin-Etsu Chemical Co., Ltd. (*7) KF-6011 produced by Shin-Etsu Chemical Co., Ltd. (*8) KF-6013 produced by Shin-Etsu Chemical Co., Ltd. | |

It was confirmed that the W/O stick foundation had a suitable feel on use, suitable long-lasting performance, and suitable elongation and finish and was excellent in terms of rubfastness.

### [Example 28]

Compact Poured Foundation

### <Preparation of Cosmetic>

A: The components 10 to 14 were dispersed using a roll mill.
B: The components 1 to 7 were dispersed using a disper. The components 8 and 9 were added to the resulting dispersion. The resulting mixture was heated to 95°C and then stirred homogeneously.
C: The mixture prepared in A was added to the mixture prepared in B. The resulting mixture was stirred homogeneously and then heated to 85°C.
D: The mixture prepared in C was charged into a container. Hereby, a compact poured foundation was prepared.

| | Composition | % |
|---|---|---|
| 1. | PG dicaprate | Balance |
| 2. | Dry-up product of Example 13 (*1) | 1 |
| 3. | Silicone composite powder (*2) | 10 |
| 4. | Silicone composite powder (*3) | 4 |
| 5. | Crosslinked dimethylpolysiloxane composition (*4) | 6 |
| 6. | Diphenylsiloxy phenyl trimethicone (*5) | 12 |
| 7. | Silicone-alkyl branched polyglycerol-modified silicone (*6) | 0.5 |
| 8. | Paraffin wax | 6 |
| 9. | Polyethylene wax | 2 |
| 10. | Dimethicone (6cs) | 11 |
| 11. | Silicone-branched polyglycerol-modified silicone (*7) | 1 |
| 12. | Metal soap-treated microparticulate zinc oxide (average primary particle size: 30 nm) | 8 |
| 13. | Silicone-treated titanium oxide (*8) | 8.5 |
| 14. | Silicone-treated iron oxide (*9) | 1.5 |
| | Total | 100.0 |

| | | |
|---|---|---|
| (* 1) Example 13 was dried up using a spray drier to form a solid substance. (*2) KSP-101 produced by Shin-Etsu Chemical Co., Ltd. (*3) KSP-105 produced by Shin-Etsu Chemical Co., Ltd. (*4) KSG-16 produced by Shin-Etsu Chemical Co., Ltd. (*5) KF-56A produced by Shin-Etsu Chemical Co., Ltd. (*6) KF-6105 produced by Shin-Etsu Chemical Co., Ltd. (*7) KF-6106 produced by Shin-Etsu Chemical Co., Ltd. (*8) KTP-09W produced by Shin-Etsu Chemical Co., Ltd. (*9) KTP-09R, Y, B produced by Shin-Etsu Chemical Co., Ltd. | | |

It was confirmed that the compact poured foundation had a suitable feel on use, suitable long-lasting performance, and suitable elongation and finish and was excellent in terms of rubfastness.

### [Example 29]

Eye Cream

### <Preparation of Cosmetic>

A: The components 1 to 4 were mixed with one another homogeneously.
B: The components 8 to 12 were mixed with one another homogeneously.
C: The mixture prepared in B was added to the mixture prepared in A, and emulsification was subsequently performed. The components 5 to 7 were added to the resulting emulsion, and the resulting mixture was stirred homogeneously. Hereby, an eye cream was prepared.

| | Composition | % |
|---|---|---|
| 1 . | Silicone-alkyl-modified-crosslinked polyether-modified silicone composition (*1) | 4 |
| 2. | Silicone-alkyl-modified-crosslinked dimethylpolysiloxane composition (*2) | 6 |
| 3. | Silicone-alkyl branched polyether-modified silicone (*3) | 0.5 |
| 4. | Dimethicone (6cs) | 12 |
| 5. | Vaseline | 4.5 |
| 6. | Solution (30% solution) prepared in Example 1 | 2.5 |
| 7. | Alkyl-modified silicone composite powder (*4) | 2 |
| 8. | 1,3-Butylene glycol | 7 |
| 9. | Phenoxyethanol | 0.25 |
| 10. | Sodium citrate | 0.2 |
| 11. | Sodium chloride | 0.5 |
| 12. | Purified water | Balance |
| | Total | 100.0 |

| | | |
|---|---|---|
| (*1) KSG-350Z produced by Shin-Etsu Chemical Co., Ltd. (*2) KSG-045Z produced by Shin-Etsu Chemical Co., Ltd. (*3) KF-6038 produced by Shin-Etsu Chemical Co., Ltd. (*4) KSP-441 produced by Shin-Etsu Chemical Co., Ltd. | | |

It was confirmed that the eye cream had a suitable feel on use, allowed a foundation deposited thereon to have suitable long-lasting performance and finish, and was excellent in terms of elongation.

### [Example 30]

Wrinkle Concealer

### <Preparation of Cosmetic>

A: The components 1 to 7 were mixed with one another homogeneously.
B: The component 8 was added to A, and the resulting mixture was stirred. Hereby, a wrinkle concealer was prepared.

| | Composition | % |
|---|---|---|
| 1. | Crosslinked polyether-modified silicone composition (*1) | 5 |
| 2. | Crosslinked dimethylpolysiloxane composition (*2) | 55 |
| 3. | Crosslinked dimethylpolysiloxane composition (*3) | 15 |
| 4. | Decamethylcyclopentasiloxane | Balance |
| 5. | Highly polymerized dimethylpolysiloxane/D5 mixed solution (*4) | 5 |
| 6. | Solution (30% solution) prepared in Example 10 | 1 |
| 7. | Silicone-modified polysaccharide compound solution (*6) | 1 |
| 8. | Silicone composite powder (*5) | 12 |
| | Total | 100 |

| | | |
|---|---|---|
| (*1) KSG-210 produced by Shin-Etsu Chemical Co., Ltd. (*2) KSG-15 produced by Shin-Etsu Chemical Co., Ltd. (*3) KSG-016F produced by Shin-Etsu Chemical Co., Ltd. (*4) KF-9028 produced by Shin-Etsu Chemical Co., Ltd. (*5) KSP-411 produced by Shin-Etsu Chemical Co., Ltd. (*6) TSPL-30-D5 produced by Shin-Etsu Chemical Co., Ltd. | | |

It was confirmed that the wrinkle concealer had a suitable feel on use, suitable long-lasting performance, and excellent elongation.

### [Example 31]

W/O Sunscreen Cream

### <Preparation of Cosmetic>

A: The components 1 to 8 were mixed with one another homogeneously.
B: The components 9 to 15 were mixed with one another homogeneously.
C: The mixture prepared in B was added to the mixture prepared in A, and emulsification was then performed. Hereby, a W/O sunscreen cream was prepared.

| | Composition | % |
|---|---|---|
| 1. | Alkyl-modified/crosslinked polyglycerol-modified silicone composition (*1) | 3 |
| 2. | Alkyl-modified/crosslinked dimethylpolysiloxane composition (*2) | 3 |
| 3. | Silicone-alkyl branched polyglycerol-modified silicone (*3) | 1.5 |
| 4. | Diphenylsiloxy phenyl trimethicone (*4) | 11 |
| 5. | 2-Ethylhexyl p-methoxycinnamate | 6 |
| 6. | Octyl salicylate | 1 |
| 7. | Silicone composite powder (*5) | 2 |
| 8. | Solution (30% solution) prepared in Example 13 | 3 |
| 9. | Xanthan gum | 0.3 |
| 10. | Dipropylene glycol | 5 |
| 11. | Glycerin | 3 |
| 12. | Methyl p-hydroxybenzoate | 0.1 |
| 13. | 2K glycyrrhizate | 0.2 |
| 14. | Sodium chloride | 0.5 |
| 15. | Purified water | Balance |
| | Total | 100.0 |

| | | |
|---|---|---|
| (*1) KSG-840 produced by Shin-Etsu Chemical Co., Ltd. (*2) KSG-43 produced by Shin-Etsu Chemical Co., Ltd. (*3) KF-6105 produced by Shin-Etsu Chemical Co., Ltd. (*4) KF-56A produced by Shin-Etsu Chemical Co., Ltd. (*5) KSP-100 produced by Shin-Etsu Chemical Co., Ltd. | | |

The W/O sunscreen cream had a suitable feel on use and excellent long-lasting performance.

### [Example 32]

O/W Sunscreen Cream

### <Preparation of Cosmetic>

A: The components 1 to 6 were heated to 85°C and subsequently mixed with one another homogeneously.
B: The components 7 to 15 were heated to 85°C and subsequently mixed with one another homogeneously.
C: The mixture prepared in B was added to the mixture prepared in A. Subsequently, emulsification was performed at 85°C. The resulting emulsion was slowly cooled while being stirred. Hereby, an O/W sunscreen cream was prepared.

| | Composition | % |
|---|---|---|
| 1. | Sodium hyaluronate | 0.1 |
| 2. | Ethanol | 10 |
| 3. | 1,3-Butylene glycol | 6 |
| 4. | Methyl p-hydroxybenzoate | 0.1 |
| 5. | Sodium acrylate-sodium acryloyldimethyltaurate copolymer composition (*1) | 2.5 |
| 6. | Purified water | Balance |
| 7. | Solution (30% solution) prepared in Example 2 | 1 |
| 8. | Diphenylsiloxy phenyl trimethicone (*2) | 3 |
| 9. | Crosslinked dimethylpolysiloxane composition (*3) | 1 |
| 10. | Cetanol | 2 |
| 11. | 2-Ethylhexyl p-methoxycinnamate | 5 |
| 12. | 2,4-Bis-[{4-(2-ethylhexyloxy)-2-hydroxy}-phenyl]-6-(4-methoxyphenyl)-1,3,5-triazine | 1 |
| 13. | Polyoxyethylene (60) hardened castor oil | 1 |
| 14. | Polyether-modified silicone (*4) | 0.5 |
| 15. | Tocopherol | 0.05 |
| | Total | 100.0 |

| | | |
|---|---|---|
| (*1) SIMULGEL EG produced by SEPPIC (*2) KF-56A produced by Shin-Etsu Chemical Co., Ltd. (*3) KSG-016F produced by Shin-Etsu Chemical Co., Ltd. (*4) KF-6011 produced by Shin-Etsu Chemical Co., Ltd. | | |

The O/W sunscreen cream had a suitable feel on use and excellent elongation.

### [Example 33]

Mousse Cheek

### <Preparation of Cosmetic>

A: The components 1 to 6 were heated to 80°C and subsequently mixed with one another homogeneously.
B: The components 7 to 12 were mixed with one another homogeneously using Henschel.
C: The mixture prepared in B was added to the mixture prepared in A. The resulting mixture was stirred homogeneously at 80°C and then slowly cooled. Hereby, a mousse cheek was prepared.

| | Composition | % |
|---|---|---|
| 1. | Crosslinked dimethylpolysiloxane composition (*1) | 32 |
| 2. | Decamethylcyclopentasiloxane | 30 |
| 3. | Neopentyl glycol diisostearate | 7 |
| 4. | Stearoyl inulin (*2) | 8 |
| 5. | Amorphous silicic anhydride (*3) | 0.5 |
| 6. | Solution (30% solution) prepared in Example 5 | 1.5 |
| 7. | Silicone-treated titanium oxide (*4) | 0.2 |
| 8. | Red No. 202 | QS |
| 9. | Silicone-treated yellow iron oxide (*4) | QS |
| 10. | Silicone-treated black iron oxide (*4) | QS |
| 11. | Silicone-treated mica (*4) | 5.4 |
| 12. | Silicone-treated sericite (*4) | 10 |
| | Total | 100.0 |

| | | |
|---|---|---|
| (*1) KSG-16 produced by Shin-Etsu Chemical Co., Ltd. (*2) Leoparl ISK2 produced by Chiba Flour Milling Co., Ltd. (*3) AEROSIL200 produced by Nippon Aerosil Co., Ltd. (*4) KP-574 treatment produced by Shin-Etsu Chemical Co., Ltd. | | |

The mousse cheek had a suitable feel on use, suitable long-lasting performance, and suitable elongation and finish and was excellent in terms of rubfastness.

### [Example 34]

Gel Eye Color

### <Preparation of Cosmetic>

A: The components 1 to 5 were heated to 80°C and then mixed with one another homogeneously.
B: The components 6 to 9 were added to the mixture prepared in A. The resulting mixture was heated to 90°C and then stirred homogeneously.
C: The mixture prepared in B was charged into a container. Hereby, a gel eye color was prepared.

| | Composition | % |
|---|---|---|
| 1. | Crosslinked dimethylpolysiloxane composition (*1) | 10.5 |
| 2. | Squalane | 17 |
| 3. | Dextrin palmitate (*2) | 8.5 |
| 4. | Isotridecyl isononanoate | Balance |
| 5. | Solution (30% solution) prepared in Example 6 | 3 |
| 6. | Amorphous silicic anhydride (*3) | 0.1 |
| 7. | Silicone composite powder (*4) | 5 |
| 8. | Ba sulfate | 9 |
| 9. | Silicone-treated titanium dioxide-coated mica (*5) | 32.5 |
| | Total | 100.0 |

| | | |
|---|---|---|
| (*1) KSG-16 produced by Shin-Etsu Chemical Co., Ltd. (*2) Leoparl KL2 produced by Chiba Flour Milling Co., Ltd. (*3) AEROSIL972 produced by Nippon Aerosil Co., Ltd. (*4) KSP-100 produced by Shin-Etsu Chemical Co., Ltd. (*5) KP-574 treatment produced by Shin-Etsu Chemical Co., Ltd. | | |

The gel eye color had a suitable feel on use, suitable long-lasting performance, and suitable elongation and finish and was excellent in terms of rubfastness.

### [Example 35]

Powder Foundation

### <Preparation of Cosmetic>

A: The components 1 to 4 were heated to 50°C and subsequently mixed with one another homogeneously. The resulting mixture was cooled to room temperature.
B: The components 5 to 14 were mixed with one another homogeneously.
C: The mixture prepared in A was added to the mixture prepared in B. The resulting mixture was stirred homogeneously with a Henschel mixer. The resulting powder was passed through a mesh and then pressed into a metal compact using a mold. Hereby, a powder foundation was prepared.

| | Composition | % |
|---|---|---|
| 1. | 2-Ethylhexyl p-methoxycinnamate | 4 |
| 2. | Diphenylsiloxy phenyl trimethicone (*1) | 4.5 |
| 3. | Triethylhexanoin | 1.5 |
| 4. | Silicone wax (*2) | 1 |
| 5. | Solution (30% solution) prepared in Example 14 | 1 |
| 6. | Silicone-treated mica (*3) | 30 |
| 7. | Ba sulfate | 10 |
| 8. | Phenyl-modified silicone composite powder (*4) | 5 |
| 9. | Silicone composite powder (*5) | 4 |
| 10. | Silicone-treated talc (*3) | Balance |
| 11. | Silicone-treated titanium oxide (*3) | 6 |
| 12. | Silicone-treated yellow iron oxide (*3) | QS |
| 13. | Silicone-treated red iron oxide (*3) | QS |
| 14. | Silicone-treated black iron oxide (*3) | QS |
| | Total | 100.0 |

| | | |
|---|---|---|
| (*1) KF-56A produced by Shin-Etsu Chemical Co., Ltd. (*2) KP-561P produced by Shin-Etsu Chemical Co., Ltd. (*3) KF-9909 treatment produced by Shin-Etsu Chemical Co., Ltd. (*4) KSP-300 produced by Shin-Etsu Chemical Co., Ltd. (*5) KSP-100 produced by Shin-Etsu Chemical Co., Ltd. | | |

The powder foundation had a suitable feel on use and suitable long-lasting performance and was excellent in terms of elongation and finish.

### [Example 36]

Leave-On Hair Treatment

### <Preparation of Cosmetic>

A: The components 1 to 4 were mixed with one another homogeneously.
B: The components 7 to 12 were mixed with one another homogeneously.
C: The mixture prepared in B was added to the mixture prepared in A, and emulsification was then performed. The components 5 and 6 were added to the resulting emulsion. Hereby, a leave-on treatment was prepared.

| | Composition | % |
|---|---|---|
| 1. | Crosslinked polyglycerol-modified silicone composition (*1) | 3 |
| 2. | Crosslinked dimethylpolysiloxane composition (*2) | 1 |
| 3. | Polyether-modified silicone (*3) | 0.2 |
| 4. | Dimethylpolysiloxane (6CS) | 8 |
| 5. | Fragrance | QS |
| 6. | Solution (30% solution) prepared in Example 15 | 1 |
| 7. | Dipropylene glycol | 8 |
| 8. | Ethanol | 5 |
| 9. | Methyl p-hydroxybenzoate | 0.1 |
| 10. | Sodium citrate | 0.2 |
| 11. | Sodium chloride | 0.5 |
| 12. | Purified water | Balance |
| | Total | 100.0 |

| | | |
|---|---|---|
| (*1) KSG-210 produced by Shin-Etsu Chemical Co., Ltd. (*2) KSG-19 produced by Shin-Etsu Chemical Co., Ltd. (*3) KF-6017 produced by Shin-Etsu Chemical Co., Ltd. | | |

The leave-on hair treatment could spread easily and impart luster to the hair and was excellent in terms of smoothness.

### [Example 37]

Hair Treatment

### <Preparation of Cosmetic>

A: The components 6 to 9 were heated to 70°C and then mixed with one another homogeneously.
B: The components 1 to 5 were heated to 70°C and then mixed with one another homogeneously.
C: The mixture prepared in B was added to the mixture prepared in A, and emulsification was then performed. After cooling had been performed slowly, the components 10 and 11 were added to the resulting emulsion. Hereby, a treatment was prepared.

| | Composition | % |
|---|---|---|
| 1. | Solution (30% solution) prepared in Example 7 | 0.5 |
| 2. | Cetanol | 2 |
| 3. | Cetyl octanoate | 3 |
| 4. | Butyl p-hydroxybenzoate | 0.1 |
| 5. | Diphenylsiloxy phenyl trimethicone (*1) | 1 |
| 6. | Behentrimonium chloride | 1 |
| 7. | Propylene glycol | 5 |
| 8. | Hydroxyethyl cellulose | 0.1 |
| 9. | Purified water | Balance |
| 10. | Amino-modified silicone emulsion (*2) | 4 |
| 11. | Fragrance | QS |
| | Total | 100.0 |

| | | |
|---|---|---|
| (*1) KF-56A produced by Shin-Etsu Chemical Co., Ltd. (*2) X-52-2328 produced by Shin-Etsu Chemical Co., Ltd. | | |

The hair treatment could spread easily and impart luster to the hair and was excellent in terms of smoothness.

### [Example 38]

Hair Oil

### <Preparation of Cosmetic>

A: The components 1 to 7 were mixed with one another homogeneously to form a hair oil.

| Composition | % |
|---|---|
| 1. Solution (30% solution) prepared in Example 7 | 3 |
| 2. Diphenylsiloxy phenyl trimethicone (*1) | 7 |
| 3. Diethylhexyl succinate | 10 |
| 4. Highly polymerized dimethiconol mixed solution (*2) | 1.5 |
| 5. Tocopherol | 0.1 |
| 6. Fragrance | 0.1 |
| 7. Light liquid isoparaffin | Balance |
| Total | 100.0 |

| | |
|---|---|
| (*1) KF-56A produced by Shin-Etsu Chemical Co., Ltd. (*2) X-21-5613 produced by Shin-Etsu Chemical Co., Ltd. | |

The hair oil could spread easily and impart luster to the hair and was excellent in terms of smoothness.

### [Example 39]

Hair Wax

### <Preparation of Cosmetic>

A: The components 1 to 9 were heated to 80°C and then mixed with one another homogeneously.
B: The components 10 to 16 were heated to 90°C and then mixed with one another homogeneously.
C: The mixture prepared in B was added to the mixture prepared in A, and emulsification was then performed at 80°C. The resulting emulsion was cooled to room temperature.
D: The components 17 to 19 were added to the mixture prepared in C. The resulting mixture was stirred homogeneously to form a hair wax.

| Composition | % |
|---|---|
| 1. Solution (30% solution) prepared in Example 15 | 1 |
| 2. Methyl trimethicone (*1) | 10 |
| 3. Candelilla wax | 14 |
| 4. Beeswax | 6 |
| 5. POE glyceryl isostearate | 2 |
| 6. Glycerin monostearate | 3 |
| 7. Polyether-modified silicone (*2) | 2 |
| 8. Stearic acid | 2 |
| 9. 2-Ethylhexyl p-methoxycinnamate | 0.1 |
| 10. Propylene glycol | 6 |
| 11. 1,3-Butylene glycol | 6 |
| 12. Methyl p-hydroxybenzoate | 0.2 |
| 13. Phenoxyethanol | 0.3 |
| 14. Trisodium edetate | QS |
| 15. Purified water | 31 |
| 16. Potassium hydroxide (10% solution) | QS |
| 17. Carboxy vinyl polymer (2% solution) | 15 |
| 18. Potassium hydroxide (10% solution) | QS |
| 19. Fragrance | QS |
| Total | 100.0 |

| | |
|---|---|
| (*1) TMF-1.5 produced by Shin-Etsu Chemical Co., Ltd. (*2) KF-6011 produced by Shin-Etsu Chemical Co., Ltd. | |

The stickiness of the hair wax was low. The hair was excellent in terms of holding power and antiperspirant effect.

### [Example 40]

Shampoo

### <Preparation of Cosmetic>

A: The components 1 to 10 were mixed with one another homogeneously at 70°C. The resulting mixture was slowly cooled to form a shampoo.

| Composition | % |
|---|---|
| 1. Solution (30% solution) prepared in Example 15 | 0.5 |
| 2. Polyoxyethylene lauryl ether | 0.5 |
| 3. Glycol distearate | 2 |
| 4. Na methyl cocoyl taurate | 8 |
| 5. Cocamidopropyl betaine | 8 |
| 6. Na lauryl sulfate | 10 |
| 7. Cationic cellulose | 0.5 |
| 8. Fragrance | 0.1 |
| 9. Methyl paraben | 0.1 |
| 10. Purified water | Balance |
| Total | 100.0 |

The shampoo allowed the fingers to easily run through the hair, could impart luster to the hair, and was excellent in terms of smoothness.

### [Example 41]

Roll-On Antiperspirant

### <Preparation of Cosmetic>

A: The components 1 to 4 were mixed with one another homogeneously.
B: The components 5 to 11 were mixed with one another homogeneously.
C: The mixture prepared in B was added to the mixture prepared in A, and emulsification was then performed. Hereby, a roll-on antiperspirant was prepared.

| Composition | % |
|---|---|
| 1. Crosslinked polyether-modified silicone composition (*1) | 5 |
| 2. Silicone branched polyether-modified silicone (*2) | 0.8 |
| 3. Solution (30% solution) prepared in Example 15 | 5 |
| 4. Decamethylcyclopentasiloxane | 9 |
| 5. 1,3-Butylene glycol | 5 |
| 6. Chlorohydroxyaluminum | 10 |
| 7. Benzalkonium chloride | 0.2 |
| 8. Menthol | 0.05 |
| 9. Ethanol | 15 |
| 10. Fragrance | QS |
| 11. Purified water | Balance |
| Total | 100.0 |

| | |
|---|---|
| (*1) KSG-210 produced by Shin-Etsu Chemical Co., Ltd. (*2) KF-6028 produced by Shin-Etsu Chemical Co., Ltd. | |

The roll-on antiperspirant could spread easily, did not whiten the skin, and was excellent in terms of the durability of antiperspirant effect.

### [Example 42]

Nail Enamel Overcoat

### <Preparation of Cosmetic>

A: The components 5 to 8 were mixed with one another. The component 4 was added to the resulting mixture. Subsequently, stirring was performed homogeneously.
B: The components 1 to 3 were added to the mixture prepared in A. The resulting mixture was stirred to form a nail enamel overcoat.

| Composition | % |
|---|---|
| 1. Solution (30% solution) prepared in Example 11 | 5 |
| 2. Nitrocellulose | 17 |
| 3. Alkyd resin | 4 |
| 4. Acetyl triethyl citrate | 5 |
| 5. Butyl acetate | Balance |
| 6. Ethyl acetate | 25 |
| 7. Isopropyl alcohol | 3 |
| 8. n-Butvl alcohol | 1 |
| Total | 100 |

The enamel overcoat could spread easily, increased the gloss of the enamel, and was excellent in terms of durability.

## Claims

1. A cosmetic preparation comprising a crosslinked organosilicon resin that is a product of an addition reaction between a component (A) and a component (B), an amount of a hydrogen gas generated by the crosslinked organosilicon resin per weight under a normal condition being up to 1.5 mL/g:
(A) an alkenyl group-containing organosilicon resin represented by formula (1), the alkenyl group-containing organosilicon resin having one or more alkenyl groups per molecule,
[Chem. 1]
(R¹R²₂SiO_{1/2})ₐ₁(R²₃SiO_{1/2})ₐ₂(R³₃SiO_{1/2})ₐ₃(R²₂SiO_{2/2})_{b}(R²SiO_{3/2})_{c}(SiO_{4/2})_{d} (1)
wherein R¹'s each independently are an alkenyl group having 2 to 8 carbon atoms, R²'s each independently represent a group selected from an alkyl group having 1 to 30 carbon atoms, an aryl group having 6 to 30 carbon atoms and an aralkyl group having 7 to 30 carbon atoms, R³'s each independently are a group selected from an organopolysiloxane-containing group and R², one or more R³'s included in each of the R³₃SiO_{1/2} units are organopolysiloxane-containing groups, and a1, a2, a3, b, c and d are numbers that satisfy 0 < a1 ≤ 5, 0 < a2 ≤ 400, 0 ≤ a3 ≤ 400, 0 ≤ b ≤ 320, 0 ≤ c ≤ 320, 0 < d ≤ 1,000 and 0.5 ≤ (a1 + a2 + a3)/d ≤ 1.5; and
(B) an organohydrogenpolysiloxane represented by formula (2), the organohydrogenpolysiloxane having two or more hydrosilyl groups per molecule: in an amount such that an amount of the hydrosilyl groups is 0.5 to 1.2 moles per mole of the alkenyl groups included in the component (A),
[Chem. 2]
(R⁴₃SioO_{1/2})ₑ(R⁴₂SiO_{2/2})_{f}(R²SiO_{3/2})_{g}(SiO_{4/2})ₕ (2)
wherein R²'s are the same things as above, R⁴'s each independently are a hydrogen atom or a group represented by R²'s, two or more of R⁴'s are hydrogen atoms, and e, f, g and h are zero or a positive number, where 2 ≤ e + f + g + h < 32.

2. The cosmetic preparation according to claim 1, wherein the organopolysiloxane-containing group represented by R³ is one or more groups represented by formulae (3) to (6),
[Chem. 3]
-CₘH₂ₘ-(CH₂)₂-(SiOR²₂)ᵢ-SiR²₃ (3)
-CₘH₂ₘ-(CH₂)₂-SiR²ⱼ₁-(OSiR²₃)₃₋ⱼ₁ (4)
-CₘH₂ₘ-(CH₂)₂-SiR²ⱼ₁-(OSiR²ⱼ₂(OSiR²₃)₃₋ⱼ₂)₃₋ⱼ₁ (5)
-CₘH₂ₘ-(CH₂)₂-SiR²ⱼ₁-(OSiR²ⱼ₂(OSiR²ⱼ₃(OSiR²₃)₃₋ⱼ₃)₃₋ⱼ₂)₃₋ⱼ₁ (6)
wherein R² is the same thing as above, m is an integer of 0 ≤ m ≤ 5, i is an integer of 0 ≤ i ≤ 500, and j1 to j3 each are an integer of 0 to 2.

3. The cosmetic preparation according to claim 1, wherein, in formula (1), b = 0 and c = 0.

4. The cosmetic preparation according to claim 1, wherein, in formula (2), g = 0 and h = 0.

5. The cosmetic preparation according to claim 1, wherein the crosslinked organosilicon resin has a weight-average molecular weight of 5,000 to 1,000,000.

6. The cosmetic preparation according to claim 1, wherein, in formula (2), 0 ≤ f < 30, two of R⁴'s are hydrogen atoms, and the crosslinked organosilicon resin is solid at 25°C.

7. The cosmetic preparation according to claim 1, wherein the amount of the hydrogen gas generated by the crosslinked organosilicon resin per weight under the normal condition is 0.01 to 1.2 mL/g.

8. The cosmetic preparation according to claim 1, wherein the crosslinked organosilicon resin is soluble in a volatile oil at 25°C, the volatile oil having a boiling point of up to 250°C at 1,013 hPa.

9. The cosmetic preparation according to claim 8, wherein the volatile oil is one or more selected from an silicone oil, isododecane and ethanol.

10. The cosmetic preparation according to claim 1, wherein a content of the crosslinked organosilicon resin is 0.1% to 40% by weight.

11. The cosmetic preparation according to claim 1, the cosmetic preparation being a sunscreen cosmetic preparation or a makeup cosmetic preparation.

12. The cosmetic preparation according to claim 1, the cosmetic preparation being a non-aqueous composition.

13. A method for producing the cosmetic preparation according to any one of claims 1 to 12, the method comprising a step of reacting:
(A) an alkenyl group-containing organosilicon resin represented by formula (1), the alkenyl group-containing organosilicon resin having one or more alkenyl groups per molecule,
[Chem. 4]
(R¹R²₂SiO_{1/2})ₐ₁(R²₃SiO_{1/2})ₐ₂(R³₃SiO_{1/2})ₐ₃(R²₂SiO_{2/2})_{b}(R²SiO_{3/2})_{c}(SiO_{4/2})_{d} (1)
wherein R¹'s each independently are an alkenyl group having 2 to 8 carbon atoms, R²'s each independently are a group selected from an alkyl group having 1 to 30 carbon atoms, an aryl group having 6 to 30 carbon atoms and an aralkyl group having 7 to 30 carbon atoms, R³'s each independently are a group selected from an organopolysiloxane-containing group and R², one or more R³'s included in each of the R³₃SiO_{1/2} units are organopolysiloxane-containing groups, and a1, a2, a3, b, c and d are numbers that satisfy 0 < a1 ≤ 5, 0 < a2 ≤ 400, 0 ≤ a3 ≤ 400, 0 ≤ b ≤ 320, 0 ≤ c ≤ 320, 0 < d ≤ 1,000, and 0.5 ≤ (a1 + a2 + a3)/d ≤ 1.5; with
(B) an organohydrogenpolysiloxane represented by formula (2), the organohydrogenpolysiloxane having two or more hydrosilyl groups per molecule,
[Chem. 5]
(R⁴₃SiO_{1/2})ₑ(R⁴₂SiO_{2/2})_{f}(R²SiO_{3/2})_{g}(SiO_{4/2})ₕ (2)
wherein R²'s are the same things as above, R⁴'s each independently are a hydrogen atom or a group represented by R², two or more of R⁴'s are hydrogen atoms, and e, f, g and h are zero or a positive number, where 2 ≤ e + f + g + h < 32,
such that an amount of the hydrosilyl groups is 0.5 to 1.2 moles per mole of the alkenyl groups included in the component (A), to produce a crosslinked organosilicon resin.
